(19) Europäisches Patentamt
European Patent Office
Office européen des brevets

(11) EP 4 474 446 B1

(12) **EUROPEAN PATENT SPECIFICATION**

(45) Date of publication and mention of the grant of the patent:
**17.12.2025 Bulletin 2025/51**

(21) Application number: **23305920.3**

(22) Date of filing: **09.06.2023**

(51) International Patent Classification (IPC):
***C09K 9/02*** (2006.01) ***C07D 407/14*** (2006.01)
***C07D 409/04*** (2006.01) ***C07D 409/14*** (2006.01)
***C07D 495/04*** (2006.01) ***C07D 519/00*** (2006.01)

(52) Cooperative Patent Classification (CPC):
**C09K 9/02; C07D 407/14; C07D 409/04; C07D 409/14; C07D 495/04; C07D 519/00;**
G02F 2202/14

(54) **ELECTROCHROMIC COMPOUNDS, COMPOSITIONS AND DEVICES COMPRISING THE SAME**

ELEKTROCHROME VERBINDUNGEN, ZUSAMMENSETZUNGEN UND VORRICHTUNGEN DAMIT

COMPOSÉS ÉLECTROCHROMIQUES, COMPOSITIONS ET DISPOSITIFS LES COMPRENANT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**11.12.2024 Bulletin 2024/50**

(73) Proprietor: **Essilor International**
**94220 Charenton-Le-Pont (FR)**

(72) Inventors:
- **GABBUTT, Christopher David**
  **Huddersfield (GB)**
- **ARMITAGE, Georgina Kate**
  **Huddersfield (GB)**
- **HERON, Bernard Mark**
  **Huddersfield (GB)**
- **BIVER, Claudine**
  **94220 Charenton-le-Pont (FR)**
- **BERIT-DEBAT, Fabien**
  **94220 Charenton-le-Pont (FR)**
- **ARCHAMBEAU, Samuel**
  **94220 Charenton-le-Pont (FR)**
- **CROSSLEY, Daniel**
  **Huddersfield (GB)**
- **AIKEN, Stuart**
  **Huddersfield (GB)**

(74) Representative: **Plasseraud IP**
**104 Rue de Richelieu**
**CS92104**
**75080 Paris Cedex 02 (FR)**

(56) References cited:
**US-A1- 2021 284 902**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 474 446 B1

## Description

This disclosure relates to a family of electrochromic compounds. More specifically, it relates to a group of electrochromic compounds comprising a 5-membered heterocycle containing sulfur, selenium or oxygen atoms. The present disclosure also refers to compositions and optical articles comprising said electrochromic compounds.

Electrochromism is a well-known physical phenomenon which is observed with certain classes of chemical compounds that reversibly change color when a voltage is applied to them. The material undergoes reversible changes in optical properties by oxidation and reduction. Advantageously, the electrochromic material is colorless when an electric field is not applied and becomes colored when an electric field is applied.

An electrochromic device, i.e. a device containing electrochromic compounds (for instance electrochromic dyes), the absorbance of which depends only on the presence of an electric field, can thus have two states, i.e. a colored state (when electrically activated) and a bleached state (in the inactive state). The optical transmission properties of the device depend on the nature of the electrochromic compounds.

Electrochromic compositions having a desired color can be obtained by mixing different electrochromic compounds. These different electrochromic compounds can be oxidizing electrochromic compounds (compounds undergoing a reduction when activated) as well as reducing electrochromic compounds (compounds undergoing an oxidation when activated). However, obtaining the desired color for an electrochromic composition is an exercise much more complex than simply mixing colors. Indeed, in addition to the numerous requirements that the electrochromic compounds should meet, the challenge of using a combination of different electrochromic compounds lays in the compatibility of said electrochromic compounds with each other.

For example, when preparing an electrochromic composition to be used as transparent media for forming high quality optical articles, in particular high quality ophthalmic lenses and electrochromic eyewear devices, the choice of electrochromic compounds is an important factor. Indeed, electrochromic compounds need not only to show good electrochromic properties such as high absorption of the visible light in the colored state, low absorption of visible radiations in the bleached state, fast coloring and fading rates, but should also have long-term stability, in particular in the presence of oxygen, and good solubility in conventional solvents. In addition, compounds with a narrow absorption band advantageously allow absorbing specific wavelengths in order to provide specific eye protection, visual aid and contrast enhancement.

Gathering all the required properties in one single compound is a real challenge. Several studies have already been conducted for providing electrochromic compounds having the best compromise. For example, viologen compounds have been identified as oxidizing electrochromic compounds of particular interest due to their high molar absorption coefficient.

On the other hand, the use of a combination of several electrochromic compounds in a single composition further requires that the electrochromic compounds have oxido-reducing potentials close enough so that they can change color simultaneously when a potential is applied to the composition and avoid producing the so-called chameleon effect during the activation of the formulation.

Another difficulty to face when using electrochromic composition in ophthalmic applications is to meet the consumer demand, which requires a wide range of colors available, and in particular neutral colors (i.e. brown, grey, grey-green...). In this context, blue viologens are well-known. Green, orange, yellow or red viologens have also been described. These molecules are oxidizing electrochromic compounds which are colored under their reduced state and are usually associated with reducing electrochromic molecules that are colored under oxidation; however some of those are weakly colored.

There remains therefore a need for new electrochromic compounds which would allow the expansion of the range of colors, avoiding the so-called chameleon effect, while at the same time providing increased molar absorptivity. This would allow minimizing the concentration of those dyes in the formulation while providing the desired tint with the desired intensity when those are activated and the manufacturing of optical devices providing superior eye protection, visual aid and contrast enhancement.

An electrochromic device comprising an electrochromic composite is disclosed in US2021284902.

After conducting extensive research, the present inventors provide a family of electrochromic compounds of formula (I), comprising an aromatic ring thiophene, selenophene or furan, which solve some of the issues encountered in the prior art.

The present disclosure refers, therefore, to an electrochromic compound represented by formula (I):

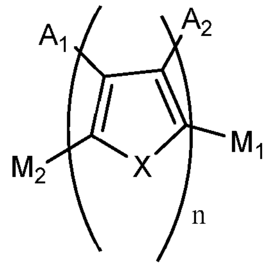

(I)

wherein

(i) **$A_1$** and **$A_2$** are both H; **n** is 1, 2 or 3; and **$M_1$** and **$M_2$** are each independently selected from the group consisting of H, 2,6-di-$C_{1-12}$ alkoxyphenyl, Ma, Mb and Mc:

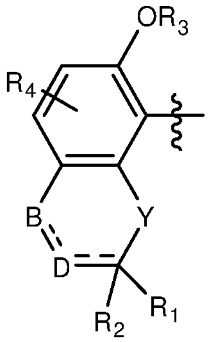

(Ma),

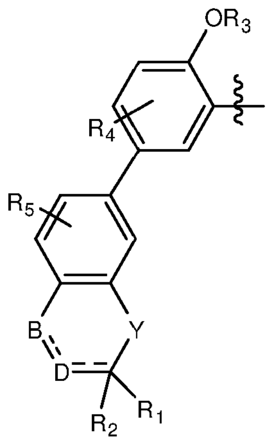

(Mb) and

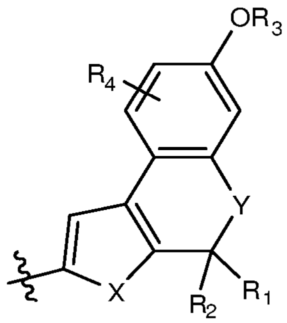

(Mc),

and wherein **at least one** of **$M_1$** and **$M_2$** is selected from the group consisting of Ma, Mb and Mc;
or

(ii) **n** is 1; **$A_2$** is H; **$A_1$** and **$M_2$** form together a heterocyclic group fused to the central 5-membered heterocyclic ring they are attached to; wherein said heterocyclic group formed is a group Ia:

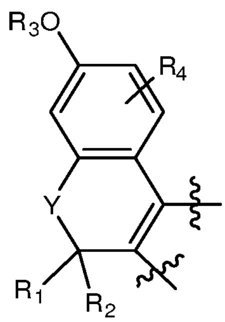

(Ia);

and **$M_1$** is selected from the group consisting of H, 2,6-di-$C_{1-12}$ alkoxyphenyl, Ma, Mb and Mc;
or

(iii) **n** is 1; **$A_1$** is H; **$M_2$** is selected from the group consisting of H, 2,6-di-$C_{1-12}$ alkoxyphenyl, Ma, Mb and Mc; and **$A_2$** and **$M_1$** form together a heterocyclic group fused to the central 5-membered heteroaromatic ring they are attached to; wherein said heterocyclic group is selected from the group consisting of Ib, Ic and Id:

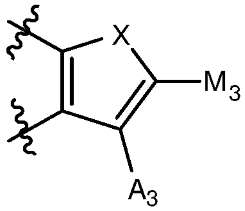 (Ib), 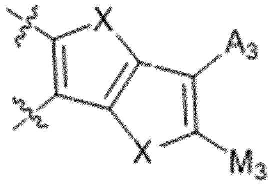 (Ic) and 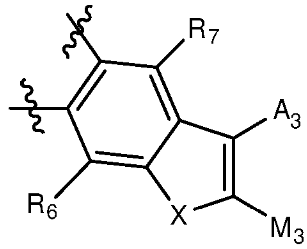 (Id);

and wherein $\mathbf{A_3}$ is H and $\mathbf{M_3}$ is selected from the group consisting of H, 2,6-di-$C_{1-12}$ alkoxyphenyl, Ma, Mb and Mc, wherein **at least one** of $\mathbf{M_2}$ and $\mathbf{M_3}$ is selected from the group consisting of Ma, Mb and Mc;
and wherein

- - - - - - is a single bond or a double bond;

each **X** is an atom independently selected from the group consisting of S, Se and O;

each **Y** is an atom independently selected from the group consisting of O, S, $-NR_8$, $N-C(O)R_8$ and $N-S(O)_2R_8$ ;

**B** is present or not, and, if present is CH or $CH_2$;

**D** is C-$\mathbf{R_{10}}$, CH or $(CH_2)_m$, wherein m is 1, 2 or 3;

$\mathbf{R_1}$ is present or not;

when $\mathbf{R_1}$ is present, each $\mathbf{R_1}$ is independently selected from the group consisting of H, $C_{3-12}$ cycloalkyl, linear $C_{1-12}$ alkyl and branched $C_{3-12}$ alkyl; and each $\mathbf{R_2}$ is independently selected from the group consisting of H, $C_{3-12}$ cycloalkyl, linear $C_{1-12}$ alkyl and branched $C_{3-12}$ alkyl; or $\mathbf{R_1}$ and $\mathbf{R_2}$ form together with the carbon atom to which they are attached, a $C_4$-$_{12}$ spiro-cycloalkyl group;

when $\mathbf{R_1}$ is not present, **D** is C-$R_{10}$, wherein $\mathbf{R_2}$ and $\mathbf{R_{10}}$ form together with the carbon atom to which they are attached a $C_{6-10}$ aryl group fused to the ring to which $\mathbf{R_{10}}$ and $\mathbf{R_2}$ are attached to;

each $\mathbf{R_3}$ is independently selected from the group consisting of linear $C_{1-12}$ alkyl, branched $C_{3-12}$ alkyl, $C_{1-12}$ haloalkyl, $C_{3-12}$ cycloalkyl, $-C(O)NR_8R_9$, and a 6-membered heteroaromatic ring including at least 1 oxygen atom; and each $\mathbf{R_4}$ is independently selected from the group consisting of H, $C_{1-12}$ alkoxy, $C_{1-12}$ alkyl and $-NR_8R_9$; or when $\mathbf{R_4}$ and $\mathbf{OR_3}$ are substituents of two adjacent carbon atoms of the benzene ring they are attached to, said $\mathbf{R_4}$ and $\mathbf{OR_3}$ form together with the carbon atom to which they are attached, a 5 to 7-membered oxygen-containing ring fused to the benzene ring to which $\mathbf{R_4}$ and $\mathbf{OR_3}$ are attached; wherein said 5 to 7-membered oxygen-containing ring is optionally substituted with one or more $C_{1-6}$ alkyl groups;

each $\mathbf{R_5}$ is independently selected from the group consisting of H, $C_{1-12}$ alkoxy, $C_{1-12}$ alkyl and - $NR_8R_9$

$\mathbf{R_6}$ and $\mathbf{R_7}$ are each independently selected from the group consisting of H, linear $C_{1-12}$ alkyl, branched $C_{3-12}$ alkyl, $C_{6-10}$ aryl, $C_{1-12}$ alkoxy; and

$\mathbf{R_8}$ and $\mathbf{R_9}$ are each independently selected from the group consisting of H, linear $C_{1-12}$ alkyl, branched $C_{3-12}$ alkyl and $C_{6-10}$ aryl.

In particular, the compounds of formula (I) herein disclosed provide an intense, , absorption in the visible range and are strongly colored when oxidized, allowing reducing the concentration of said dyes in the compositions and devices comprising those. Advantageously, some of the compounds of formula (I) herein disclosed also present a narrow absorption band which makes them particularly useful in the manufacturing of optical devices providing superior eye protection, visual aid and contrast enhancement.

Advantageously, some of the electrochromic compounds of formula (I) herein disclosed also feature increased molar absorptivity on the derived radical cation species compared to other compounds of the prior art.

Indeed, the side groups $\mathbf{M_1}$ and $\mathbf{M_2}$**,** may efficiently conjugate with and act as an electron donor to the central

aromatic ring thiophene, selenophene or furan, due to their highly restricted conformational mobility. Without wishing to be bound by theory, it appears that the structural characteristics of the groups $\mathbf{M_1}$ and $\mathbf{M_2}$ exert a very significant increase in the molar absorptivity of the compounds compared to other structurally similar compounds.

For all of the above, the present invention also relates to an electrochromic composition comprising at least one compound of formula (I).

Finally, the present invention relates to an electrochromic device, such as an ophthalmic lens, comprising an electrochromic compound of formula (I) or an electrochromic composition according to the invention.

The expression "alkyl" or "$C_1$-$C_{18}$ alkyl" represents any monovalent radical of a linear or branched hydrocarbon chain comprising 1 to 18 carbon atoms. The expression "$C_{1}$-$_{6}$ alkyl" represents an alkyl group having 1 to 6 carbon atoms. Examples of $C_1$-$_{18}$ alkyl groups include, among others, $C_1$-$_4$ alkyl groups such as methyl, ethyl, *n*-propyl, *i*-propyl, *n*-butyl, *i*-butyl, *s*-butyl or *t*-butyl, $C_6$-$_8$ alkyl groups such as *n*-hexyl, *n*-heptyl or *n*-octyl, as well as *n*-pentyl, 2-ethylhexyl, 3,5,5-trimethylhexyl, *n*-nonyl, *n*-decyl, *n*-undecyl, *n*-dodecyl or *n*-octadecyl.

The expression "alkoxy" means an alkyl-O- group, wherein said alkyl is as defined herein. Examples of alkoxy groups notably include, among others, methoxy, ethoxy and isopropoxy. The expression "haloalkyl" refers to an alkyl group as defined herein comprising one or more halogen atom (F, Cl, Br or I) substitution, among which (per)fluoroalkyl groups refer to alkyl groups as defined herein, having one to three fluorine atom substitutions.

As used herein, the expression "aromatic group" or "aromatic ring" or "heteroaromatic ring" refer to unsaturated chemical groups characterized by one or more planar rings of atoms joined by covalent bonds. The expressions "heteroaromatic group" or "heteroaromatic ring" refers to aromatic groups or rings comprising at least one heteroatom, i.e., contain at least one sulfur, selenium, nitrogen, oxygen, or other non-carbon atoms.

The expression "aryl" represents any monovalent radical of an aromatic group comprising 6 to 18 carbon atoms, notably 6 to 10 carbon atoms, either monocyclic or polycyclic. Examples of $C_6$-$_{18}$ aryl groups include, among others, phenyl, naphthyl, anthracenyl and phenanthrenyl. The expressions "aryl" and "aromatic group" refer to the same chemical group, but while "aryl" refers to a monovalent radical, "aromatic group" may refer indistinctively to a monovalent radical or to a bivalent or a trivalent radical of the corresponding aromatic group.

The expression "heteroaryl" represents any monovalent radical of a monocyclic or polycyclic 5 to 10 membered aromatic group (such as a bicyclic group) comprising from 1 to 3 heteroatoms independently selected from oxygen, nitrogen and sulfur. Thus, the expression "heteroaryl" includes heteroatom-containing aromatic rings or heteroatom-containing polycyclic aromatic groups. The expressions "heteroaryl" and "hetero aromatic group" refer to the same chemical group, but while "heteroaryl" refers to a monovalent radical, "hetero aromatic group" may refer indistinctively to a monovalent radical or to a bivalent or a trivalent radical of the corresponding chemical group. Examples of heteroaryl or hetero aromatic groups include, among others, furyl, thienyl (or thiophen-2-yl or thiophen-3-yl), selenophenyl, pyrrolyl, pyrazoyl, imidazolyl, isoxazolyl, isothiazoyl, thiazolyl, oxazolyl, 1,2,3-triazolyl, 1,2,4-triazolyl, pyridinyl, pyridazinyl, pyrimidinyl and pyrazinyl, as well as their respective bivalent and trivalent radicals.

The expression "heterocycle" refers to a chemical group featuring a closed ring of atoms, aromatic or non-aromatic comprises one or more heteroatoms. Accordingly, a heterocyclic cycle may refer to a heteroaryl, but also to non-aromatic rings containing heteroatoms. Examples of heterocycles include, among others, furan, dihydro and tetrahydrofuran, thiophene, dihydro and tetrahydrothiophene, selenophene, dihydro and tetrahydroselenophene, pyrrole, pyrrolidine, pyrazole, pyrazolidine, imidazole, imidazoline, imidazolidine, oxazole, isoxazole, thiazole, isothiazole, pyridine, pyridazine, pyrimidine, piperidine, piperazine, pyran, tetrahydropyran, thiane, dithiane, thiopyran, as well as their respective monovalent, bivalent and trivalent radicals. In an analogous manner, the expression "heterocyclic group" refers to chemical groups featuring one or more closed rings of atoms, aromatic or non-aromatic, wherein at least one ring comprises one or more heteroatoms. Accordingly, a heterocyclic group may contain an aromatic group, a hetero aromatic group, but also non-aromatic heterocycles. Examples of heterocyclic groups include, among others, furyl, thienyl (or thiophen-2-yl or thiophen-3-yl), selenophenyl, pyrrolyl, pyrazoyl, imidazolyl, isoxazolyl, isothiazoyl, thiazolyl, oxazolyl, 1,2,3-triazolyl, 1,2,4-triazolyl, 1-benzofuryl, 1-benzothienyl, indolyl, benzimidazolyl, benzimidazolium, benzimidazoliumyl, indazolyl, 1,2-benzisoxazolyl, 2,1-benzisoxazolyl, 1,2-benzisothiazolyl, 2,1-benzisothiazolyl, benzothiazolyl, benzoxazolyl, benzoxazoliumyl, benzoselenazoliumyl, benzotriazolyl, 2H-chromenyl, 4H-chromenyl, 1H-isochromenyl, 3H-isochromenyl, chromanyl (dihydrobenzopyranyl), benzothianyl, dihydrobenzothianyl, benzofuranyl, 2,3-dihydrobenzofuranyl, dibenzofuranyl, pyridinyl, pyridiniumyl, N-alkylpyridiniumyl, N-arylpyridiniumyl, quinolinyl, quinolinium, quinoliniumyl, isoquinolinyl, isoquinolinium, isoquinoliniumyl, pyridazinyl, cinnolinyl, phthalazinyl, pyrimidinyl, quinazolinyl, pyrazinyl and quinoxalinyl, as well as their respective bivalent and trivalent radicals.

In some embodiments the compound of formula (I) comprises more than one atom **X** and:

- each **X** is the same type of atom and is selected from the group consisting of S, Se and O; or

- each **X** is a different type of atom, and each **X** is independently selected from the group consisting of S, Se and O.

In some embodiments the compound of formula (I) comprises more than one atom **X** and at least one **X** is S, or all **X** are S. In other embodiments the compound of formula (I) comprises more than one atom **X** and at least one **X** is Se or all **X** are Se. In yet other embodiments the compound of formula (I) comprises more than one atom **X** and at least one at least one **X** is O or all **X** are O. In some embodiments, the compound of formula (I) comprises more than one atom **X** and each **X** is **S** or **Se,** or each **X** is **S** or **O,** or each **X** is **Se** or **O.**

In some embodiments one or more of **X** is S. In other embodiments one or more of **X** is Se. In yet other embodiments one or more of **X** is O.

In some embodiments $\mathbf{A_1}$ and $\mathbf{A_2}$ are both H; **n is** 1, 2 or 3, preferably **n** is 1 or 2, also preferably **n** is 1.

In some embodiments each **Y** is independently O or S. In some embodiments **Y** is O. In some other embodiments **Y** is S. In some embodiments one or more of **Y** is S. In other embodiments one or more of **Y** is O.

In some preferred embodiments **B** is present, both **B** and **D** are $CH_2$ and ------ is a single bond. In other embodiments **B** is present, both **B** and **D** are CH and ------ is a double bond. In some embodiments **B** is not present.

In some embodiments $\mathbf{R_1}$ is present and:

each $\mathbf{R_1}$ is independently selected from the group consisting of H, $C_{3-12}$ cycloalkyl, linear $C_{1-12}$ alkyl and branched $C_{3-12}$ alkyl; and each $\mathbf{R_2}$ is independently selected from the group consisting of H, $C_{3-12}$ cycloalkyl, linear $C_{1-12}$ alkyl and branched $C_{3-12}$ alkyl; preferably each $\mathbf{R_1}$ is independently a linear $C_{1-6}$ alkyl or branched $C_{3-6}$ alkyl; and each $\mathbf{R_2}$ is independently a linear $C_{1-6}$ alkyl or a branched $C_{3-6}$ alkyl; more preferably each $\mathbf{R_1}$ and $\mathbf{R_2}$ are independently methyl, ethyl, propyl or isopropyl; or

$\mathbf{R_1}$ and $\mathbf{R_2}$ form together with the carbon atom to which they are attached, a $C_{4-12}$ spiro-cycloalkyl, preferably a $C_{5-6}$ spiro-cycloalkyl; more preferably a $C_6$ spiro-cycloalkyl group.

In some embodiments $\mathbf{R_1}$ is present and both $\mathbf{R_1}$ and $\mathbf{R_2}$ are a same moiety, being said moiety selected from the group consisting of H, $C_{3-12}$ cycloalkyl, linear $C_{1-12}$ alkyl and branched $C_{3-12}$ alkyl, preferably a linear $C_{1-6}$ alkyl or branched $C_{3-6}$ alkyl; more preferably methyl, ethyl, propyl or isopropyl.

In other embodiments $\mathbf{R_1}$ is present and both $\mathbf{R_1}$ and $\mathbf{R_2}$ are a different moiety, being each $\mathbf{R_1}$ and $\mathbf{R_2}$ independently selected from the group consisting of H, $C_{3-12}$ cycloalkyl, linear $C_{1-12}$ alkyl and branched $C_{3-12}$ alkyl, preferably a linear $C_{1-6}$ alkyl or branched $C_{3-6}$ alkyl; more preferably methyl, ethyl, propyl or isopropyl.

In some embodiments $\mathbf{R_1}$ is present and both $\mathbf{R_1}$ and $\mathbf{R_2}$ are methyl, ethyl, propyl or isopropyl; or $\mathbf{R_1}$ and $\mathbf{R_2}$ form together with the carbon atom to which they are attached a $C_{5-6}$ spiro-cycloalkyl; more preferably a $C_6$ spiro-cycloalkyl group.

In some embodiments $\mathbf{R_1}$ is not present and **D** is C-$\mathbf{R}_{10}$, wherein $\mathbf{R_2}$ and $\mathbf{R_{10}}$ form together with the carbon atom to which they are attached a $C_{6-10}$ aryl group, preferably a benzene ring, fused to the ring to which $\mathbf{R_{10}}$ and $\mathbf{R_2}$ are attached to.

In some embodiments, **B** and $\mathbf{R_1}$ are not present and **D** is C-$\mathbf{R_{10}}$, wherein $\mathbf{R_2}$ and $\mathbf{R_{10}}$ form together with the carbon atom to which they are attached a $C_{6-10}$ aryl group, preferably a benzene ring, fused to the ring to which $\mathbf{R_{10}}$ and $\mathbf{R_2}$ are attached to.

In some embodiments each $\mathbf{R_3}$ is independently selected from the group consisting of linear $C_{1-12}$ alkyl, branched $C_{3-12}$ alkyl, $C_{1-12}$ haloalkyl, $C_{3-12}$ cycloalkyl, -C(O)$NR_8R_9$, and a 6-membered heteroaromatic ring including at least 1 oxygen atom, such as pyran or dioxine; preferably each $\mathbf{R_3}$ is independently a linear $C_{1-12}$ alkyl, branched $C_{3-12}$ alkyl, or $C_{1-12}$ (per)fluoroalkyl, more preferably a linear $C_{1-6}$ alkyl, a branched $C_{3-6}$ alkyl or $C_{1-6}$ (per)fluoroalkyl, even more preferably methyl, ethyl, propyl or isopropyl.

In some embodiments $\mathbf{R_1}$ and $\mathbf{R_2}$ are each independently a linear $C_{1-6}$ alkyl group or a branched $C_{3-6}$ alkyl group; $\mathbf{R_3}$ is independently a linear $C_{1-6}$ alkyl group, a branched $C_{3-6}$ alkyl group or $C_{1-6}$ (per)fluoroalkyl group; and $\mathbf{R_4}$**,** $\mathbf{R_5}$**,** $\mathbf{R_6}$ and $\mathbf{R_7}$ are all H.

In some embodiments $\mathbf{R_1}$**,** $\mathbf{R_2}$ and $\mathbf{R_3}$ are all a same moiety, being said moiety selected from the group consisting of H, $C_{3-12}$ cycloalkyl, linear $C_{1-12}$ alkyl and branched $C_{3-12}$ alkyl, preferably a linear $C_{1-6}$ alkyl or branched $C_{3-6}$ alkyl; more preferably methyl, ethyl, propyl or isopropyl.

In other embodiments $\mathbf{R_1}$**,** $\mathbf{R_2}$ and $\mathbf{R_3}$ may be each a different moiety, being each $\mathbf{R_1}$**,** $\mathbf{R_2}$ and $\mathbf{R_3}$ independently selected from the group consisting of H, $C_{3-12}$ cycloalkyl, linear $C_{1-12}$ alkyl and branched $C_{3-12}$ alkyl, preferably a linear $C_{1-6}$ alkyl or branched $C_{3-6}$ alkyl; more preferably methyl, ethyl, propyl or isopropyl.

In some embodiments $\mathbf{R_1}$**,** $\mathbf{R_2}$ and $\mathbf{R_3}$ are each independently selected from the group consisting of methyl, ethyl, propyl and isopropyl.

In some embodiments, each $\mathbf{R_4}$ is independently selected from the group consisting of H, $C_{1-12}$ alkoxy, $C_{1-12}$ alkyl and -$NR_8R_9$; wherein $\mathbf{R_8}$ and $\mathbf{R_9}$ are each independently selected from the group consisting of H, linear $C_{1-12}$ alkyl, branched $C_{3-12}$ alkyl and $C_{6-10}$ aryl, preferably each independently selected from the group consisting of H, linear $C_{1-6}$ alkyl, branched $C_{3-6}$ alkyl and $C_6$ aryl. In some preferred embodiments $\mathbf{R_4}$ is H.

In some other embodiments, when $\mathbf{R_4}$ and $\mathbf{OR_3}$ are substituents of two adjacent carbon atoms of the benzene ring

they are attached to, said $\mathbf{R_4}$ and $\mathbf{OR_3}$ form together with the carbon atom to which they are attached, a 5 to 7-membered oxygen-containing ring, preferably dihydropyran, fused to the benzene ring to which $\mathbf{R_4}$ and $\mathbf{OR_3}$ are attached; wherein said 5 to 7-membered oxygen-containing ring is optionally substituted with one or more $C_{1-6}$ alkyl groups. In some embodiments, when $\mathbf{R_4}$ and $\mathbf{OR_3}$ are substituents of two adjacent carbon atoms of the benzene ring they are attached to, said $\mathbf{R_4}$ and $\mathbf{OR_3}$ form together with the carbon atom to which they are attached, a dihydropyran substituted with one or more $C_{1-6}$ alkyl groups.

In some embodiments each $\mathbf{R_5}$ is independently selected from the group consisting of H, $C_{1-12}$ alkoxy, $C_{1-12}$ alkyl and $-NR_8R_9$, preferably selected from the group consisting of H, linear $C_{1-6}$ alkyl, branched $C_{3-6}$ alkyl and $C_6$ aryl. In some preferred embodiments $\mathbf{R_5}$ is H.

In some embodiments $\mathbf{R_4}$ and $\mathbf{R_5}$ are both H.

In some embodiments $\mathbf{R_6}$ and $\mathbf{R_7}$ are each independently selected from the group consisting of H, linear $C_{1-12}$ alkyl, branched $C_{3-12}$ alkyl, $C_{6-10}$ aryl, $C_{1-12}$ alkoxy; preferably each independently selected from the group consisting of H, linear $C_{1-6}$ alkyl, branched $C_{3-6}$ alkyl, $C_6$ aryl and $C_{1-6}$ alkoxy.

In some embodiments $\mathbf{R_6}$ and $\mathbf{R_7}$ are both H.

In some embodiments, $\mathbf{R_1}$, $\mathbf{R_2}$ and $\mathbf{R_3}$ are each independently a $C_{1-6}$ alkyl group and $\mathbf{R_4}$, $\mathbf{R_5}$, $\mathbf{R_6}$ and $\mathbf{R_7}$ are all H.

In some embodiments, $\mathbf{A_1}$ and $\mathbf{A_2}$ are both H; **n** is 1, 2 or 3; and $\mathbf{M_1}$ and $\mathbf{M_2}$ are each independently selected from the group consisting of H, 2,6-di-$C_{1-12}$ alkoxyphenyl, Ma, Mb and Mc:

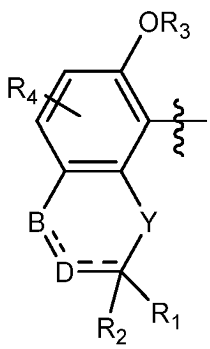
(Ma),
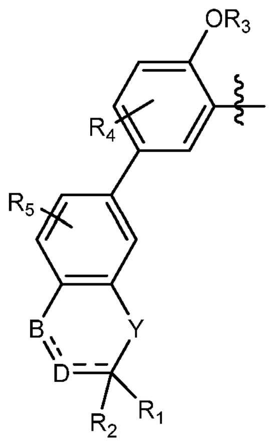
(Mb) and
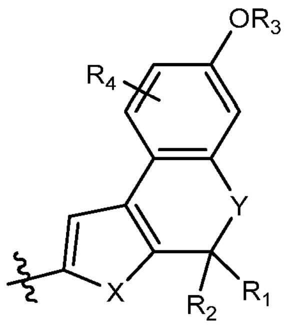
(Mc),

wherein **at least one** of $\mathbf{M_1}$ and $\mathbf{M_2}$ is selected from the group consisting of Ma, Mb and Mc; and wherein $\mathbf{R_1}$, $\mathbf{R_2}$, $\mathbf{R_3}$, $\mathbf{R_4}$, $\mathbf{R_5}$, ------, **X**, **Y**, **B** and **D** are each independently as described in the claims and the present description.

In some embodiments, $\mathbf{A_1}$ and $\mathbf{A_2}$ are both H; **n** is 1, and $\mathbf{M_1}$ and $\mathbf{M_2}$ are each independently selected from the group consisting of H, 2,6-di-$C_{1-12}$ alkoxyphenyl, Ma, Mb and Mc as described in the claims and the present description; wherein **at least one** of $\mathbf{M_1}$ and $\mathbf{M_2}$ is selected from the group consisting of Ma, Mb and Mc.

In some embodiments, $\mathbf{M_1}$ is Ma and $\mathbf{M_2}$ is Mb. In other embodiments $\mathbf{M_1}$ is Ma and $\mathbf{M_2}$ is Mc. In yet other embodiments $\mathbf{M_1}$ is Mb and $\mathbf{M_2}$ is Mc.

In some embodiments, $\mathbf{M_1}$ and $\mathbf{M_2}$ are the same moiety, wherein said moiety is selected from the group consisting of Ma, Mb and Mc as described in the claims and the present description, preferably $\mathbf{M_1}$ and $\mathbf{M_2}$ are the same moiety, wherein said moiety is Ma or Mb.

In some embodiments $\mathbf{M_1}$ and $\mathbf{M_2}$ are different and are each independently selected from the group consisting of H, 2,6-di-$C_{1-12}$ alkoxyphenyl, Ma, Mb and Mc as described in the claims and the present description, wherein at least one of $\mathbf{M_1}$ and $\mathbf{M_2}$ is selected from the group consisting of Ma, Mb and Mc.

In some embodiments $\mathbf{M_1}$ and $\mathbf{M_2}$ are different, wherein one of $\mathbf{M_1}$ and $\mathbf{M_2}$ is H or 2,6-di-$C_{1-12}$ alkoxyphenyl and another one of $\mathbf{M_1}$ and $\mathbf{M_2}$ is selected from the group consisting of Ma, Mb and Mc as described in the claims and the present description.

In some embodiments, at least one of $\mathbf{M_1}$ and $\mathbf{M_2}$ is Ma as described in the claims and the present description. In some embodiments both $\mathbf{M_1}$ and $\mathbf{M_2}$ are a group Ma as described in the claims and the present description. In some embodiments one or both of $\mathbf{M_1}$ and $\mathbf{M_2}$ is Ma as described in the claims and the present description.

In some embodiments one of $\mathbf{M_1}$ is Ma and $\mathbf{M_2}$ is H. In some embodiments, X is S; $\mathbf{A_1}$ and $\mathbf{A_2}$ are both H; **n** is 1; $\mathbf{M_1}$ is Ma:

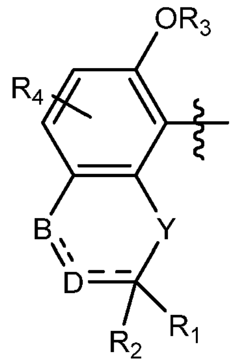

(Ma);

and $M_2$ is H;

wherein $R_1$, $R_2$, $R_3$, $R_4$, ------, **Y**, **B** and **D** are each independently as described in the claims and the present description; preferably $R_1$ is a linear $C_{1-6}$ alkyl or branched $C_{3-6}$ alkyl; $R_2$ is a linear $C_{1-6}$ alkyl or a branched $C_{3-6}$ alkyl; $R_3$ is a linear $C_{1-6}$ alkyl ,a branched $C_{3-6}$ alkyl, or $C_{1-6}$ (per)fluoroalkyl; $R_4$ is H; **Y** is S; **B** is present, and both **B** and **D** are $CH_2$ and ------ is a single bond; more preferably $R_1$, $R_2$ and $R_3$ are each independently selected from the group consisting of methyl, ethyl, propyl and isopropyl; $R_4$ is H; **Y** is S; **B** is present, and both **B** and **D** are $CH_2$ and ------ is a single bond.

In some embodiments, $A_1$ and $A_2$ are both H; **n** is 2, and $M_1$ and $M_2$ are each independently selected from the group consisting of H, 2,6-di-$C_{1-12}$ alkoxyphenyl, Ma, Mb and Mc, as described in the claims and the present description; wherein **at least one** of $M_1$ and $M_2$ is selected from the group consisting of Ma, Mb and Mc.

In some embodiments, **X** is S, $A_1$ and $A_2$ are both H; **n** is 2, and $M_1$ and $M_2$ are both Ma:

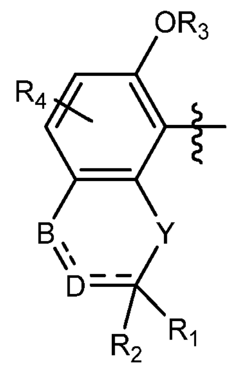

(Ma);

wherein $R_1$, $R_2$, $R_3$, $R_4$, ------, **Y**, **B** and **D** are each independently as described in the claims and the present description, preferably, each $R_1$ is independently a linear $C_{1-6}$ alkyl or branched $C_{3-6}$ alkyl; each $R_2$ is independently a linear $C_{1-6}$ alkyl or a branched $C_{3-6}$ alkyl; each $R_3$ is independently a linear $C_{1-6}$ alkyl, a branched $C_{3-6}$ alkyl, or $C_{1-6}$ (per)fluoroalkyl; $R_4$ is H; **Y** is O; B is present, and both **B** and **D** are $CH_2$ and ------ is a single bond; more preferably $R_1$, $R_2$ and $R_3$ are each independently selected from the group consisting of methyl, ethyl, propyl and isopropyl; $R_4$ is H; **Y** is O; **B** is present, and both **B** and **D** are $CH_2$ and ------ is a single bond.

In some embodiments $A_1$ and $A_2$ are both H; **n** is 1, both $M_1$ and $M_2$ are Ma, and the electrochromic compound disclosed is represented by Formula (II):

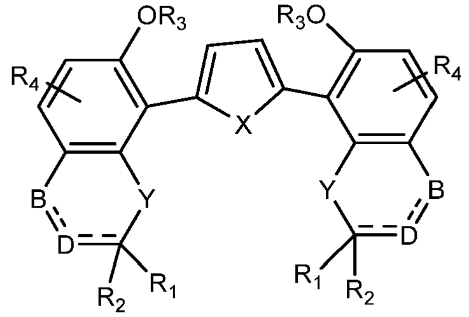

(II)

wherein **$R_1$**, **$R_2$**, **$R_3$**, **$R_4$**, ------, **Y**, **B** and **D** are each independently as described in the claims and the present description.

In some embodiments, when one or both of **$M_1$** and **$M_2$** is Ma:

each **$R_1$** is independently selected from the group consisting of H, $C_3$-$_{12}$ cycloalkyl, linear $C_{1-12}$ alkyl and branched $C_3$-$C_{12}$ alkyl; and each **$R_2$** is independently selected from the group consisting of H, $C_3$-$_{12}$ cycloalkyl, linear $C_{1-12}$ alkyl and branched $C_3$-$_{12}$ alkyl; preferably each **$R_1$** is independently a linear $C_{1-6}$ alkyl or branched $C_{3-6}$ alkyl; and each **$R_2$** is independently a linear $C_{1-6}$ alkyl or a branched $C_{3-6}$ alkyl; more preferably **$R_1$** and **$R_2$** are each independently selected from the group consisting of methyl, ethyl, propyl and isopropyl; or **$R_1$** and **$R_2$** form together with the carbon atom to which they are attached, a $C_4$-$_{12}$ spiro-cycloalkyl, preferably a $C_5$-$_6$ spiro-cycloalkyl, more preferably a $C_6$ spiro-cycloalkyl;

each **$R_3$** is independently selected from the group consisting of linear $C_{1-12}$ alkyl, branched $C_{3-12}$ alkyl, $C_3$-$_{12}$ cycloalkyl, $C_{1-12}$ haloalkyl -C(O)$NR_8R_9$, and a 6-membered heteroaromatic ring including at least 1 oxygen atom; such as pyran or dioxine; preferably each **$R_3$** is independently a linear $C_{1-12}$ alkyl, branched $C_{3-12}$ alkyl or $C_{1-12}$ (per) fluoroalkyl, more preferably linear $C_{1-6}$ alkyl, a branched $C_{3-6}$ alkyl or $C_{1-6}$ (per)fluoroalkyl; even more preferably methyl, ethyl, propyl or isopropyl; and

each **$R_4$** is independently selected from the group consisting of H, $C_{1-12}$ alkoxy, $C_{1-12}$ alkyl and -$NR_8R_9$; **$R_4$** preferably is H; or when **$R_4$** and **$OR_3$** are substituents of two adjacent carbon atoms of the benzene ring they are attached to, said **$R_4$** and **$OR_3$** form together with the carbon atom to which they are attached, a 5 to 7-membered oxygen-containing ring fused to the benzene ring to which **$R_4$** and **$OR_3$** are attached; wherein said 5 to 7-membered oxygen-containing ring is optionally substituted with one or more $C_{1-6}$ alkyl groups; preferably a pyran or a dihydropyran ring, more preferably a dihydropyran ring, fused to the benzene ring to which **$R_4$** and **$OR_3$** are attached; wherein said pyran or dihydropyran ring, more preferably said dihydropyran ring, is substituted with one or more $C_{1-6}$ alkyl groups.

In some embodiments, **X** is S, O or Se, more preferably S; **$A_1$** and **$A_2$** are both H; **n** is 1, both **$M_1$** and **$M_2$** are Ma, and the electrochromic compound disclosed is represented by Formula (II):

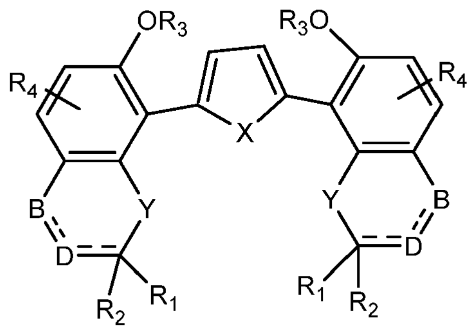

(II)

wherein

each **Y** is independently O or S;

each **$R_1$** is independently a linear $C_{1-6}$ alkyl or branched $C_{3-6}$ alkyl, preferably methyl, ethyl, propyl or isopropyl; and each **$R_2$** is independently a linear $C_{1-6}$ alkyl or a branched $C_{3-6}$ alkyl, preferably methyl, ethyl, propyl or isopropyl; or **$R_1$** and **$R_2$** form together with the carbon atom to which they are attached, a $C_6$ cycloalkyl;

each **$R_3$** is independently a linear $C_{1-6}$ alkyl, a branched $C_{3-6}$ alkyl or $C_{1-6}$ (per)fluoroalkyl, preferably methyl, ethyl, propyl or isopropyl; and **$R_4$** is H; or when **$R_4$** and **$OR_3$** are substituents of two adjacent carbon atoms of the benzene ring they are attached to, said **$R_4$** and **$OR_3$** form together dihydropyran ring fused to the benzene ring to which **$R_4$** and **$OR_3$** are attached, wherein said dihydropyran ring, is substituted with one or more $C_{1-6}$ alkyl groups; and

**B** is present, and both **B** and **D** are $CH_2$ and ------ is a single bond or both **B** and **D** are CH and ------ is a double bond;

or wherein

each **Y** is independently O or S, preferably O;

**$R_1$** is not present and **D** is C-**$R_{10}$**, wherein **$R_2$** and **$R_{10}$** form together with the carbon atom to which they are attached a $C_{6-10}$ aryl group, preferably a benzene ring, fused to the ring to which **$R_{10}$** and **$R_2$** are attached to;

each **$R_3$** is independently a linear $C_{1-6}$ alkyl, a branched $C_{3-6}$ alkyl or $C_{1-6}$ (per)fluoroalkyl; and **$R_4$** is H.

In some embodiments one of **$M_1$** or **$M_2$** is a group 2,6-di-$C_{1-12}$ alkoxyphenyl, preferably a group 2,6-di-$C_{1-6}$ alkoxyphenyl.

In some embodiments **$M_1$** is Ma:

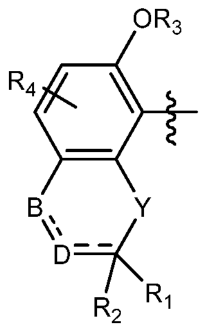

(Ma)

and **$M_2$** is a group 2,6-di-$C_{1-12}$ alkoxyphenyl;
wherein **$R_1$, $R_2$**, **$R_3$**, **$R_4$**, ------, **Y**, **B** and **D** are each independently as described in the claims and the present description.

In some embodiments **X** is S; **$A_1$** and **$A_2$** are both H; **n** is 1; **$M_1$** is Ma:

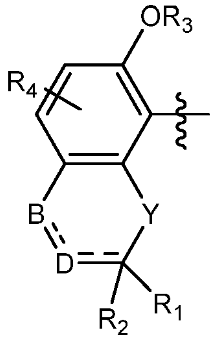

(Ma)

and **$M_2$** is a group 2,6-di-$C_{1-12}$ alkoxyphenyl, preferably a group 2,6-di-$C_{1-6}$ alkoxyphenyl;
wherein **$R_1$, $R_2$**, **$R_3$**, **$R_4$**, ------, **Y**, **B** and **D** are each independently as described in the claims and the present description, preferably **$R_1$** is a linear $C_{1-6}$ alkyl or branched $C_{3-6}$ alkyl; **$R_2$** is a linear $C_{1-6}$ alkyl or a branched $C_{3-6}$ alkyl; **$R_3$** is a linear $C_{1-6}$ alkyl or a branched $C_{3-6}$ alkyl; **$R_4$** is H; **Y** is O; **B** is present, and both **B** and **D** are $CH_2$ and ------ is a single bond or both **B** and **D** are CH and ------ is a double bond; more preferably **$R_1$, $R_2$** and **$R_3$** are each independently selected from the group consisting of methyl, ethyl, propyl and isopropyl; **$R_4$** is H; **Y** is O; **B** is present, and both **B** and **D** are $CH_2$ and ------ is a single bond or both **B** and **D** are CH and ------ is a double bond.

In some embodiments **$M_1$** is Ma, wherein Ma is as described in the claims and the present description, and **$M_2$** is H.

In some embodiments, **X** is S; **$A_1$** and **$A_2$** are both H; **n** is 1; **$M_1$** is Ma:

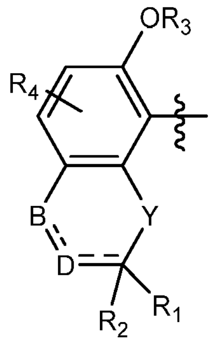

(Ma)

and $\mathbf{M_2}$ is H;

wherein $\mathbf{R_1}$, $\mathbf{R_2}$, $\mathbf{R_3}$, $\mathbf{R_4}$, - - - - - -, **Y**, **B** and **D** are each independently as described in the claims and the present description, preferably $\mathbf{R_1}$ is a linear $C_{1-6}$ alkyl or branched $C_{3-6}$ alkyl; $\mathbf{R_2}$ is a linear $C_{1-6}$ alkyl or a branched $C_{3-6}$ alkyl; $\mathbf{R_3}$ is a linear $C_{1-6}$ alkyl, a branched $C_{3-6}$ alkyl or $C_{1-6}$ (per)fluoroalkyl; $\mathbf{R_4}$ is H, **Y** is O; **B** is present, and both **B** and **D** are $CH_2$ and - - - - - - is a single bond; more preferably $\mathbf{R_1}$, $\mathbf{R_2}$ and $\mathbf{R_3}$ are each independently selected from the group consisting of methyl, ethyl, propyl and isopropyl; $\mathbf{R_4}$ is H, **Y** is O; **B** is present, and both **B** and **D** are $CH_2$ and - - - - - - is a single bond.

In some embodiments one or both $\mathbf{M_1}$ and $\mathbf{M_2}$ is Mb:

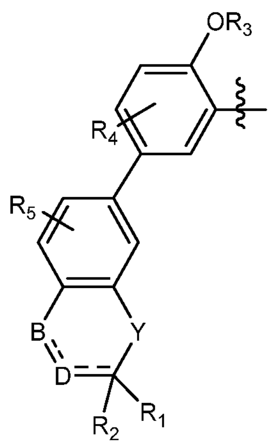

(Mb);

wherein $\mathbf{R_1}$, $\mathbf{R_2}$, $\mathbf{R_3}$, $\mathbf{R_4}$, $\mathbf{R_5}$, - - - - - -, **Y**, **B** and **D** are each independently as described in the claims and the present description.

In some embodiments both $\mathbf{M_1}$ and $\mathbf{M_2}$ are Mb, wherein Mb is as described in the claims and the present description.

In some embodiments **X** is S; $\mathbf{A_1}$ and $\mathbf{A_2}$ are both H; **n** is 1; $\mathbf{M_1}$ and $\mathbf{M_2}$ are both Mb:

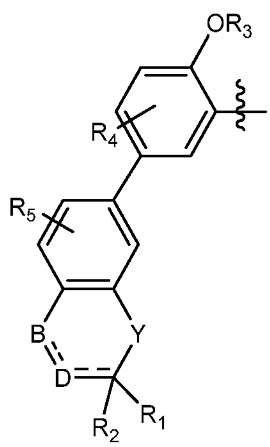

(Mb);

wherein $\mathbf{R_1}$, $\mathbf{R_2}$, $\mathbf{R_3}$, $\mathbf{R_4}$, $\mathbf{R_5}$, - - - - - -, **Y**, **B** and **D** are each independently as described in the claims and the present

description, preferably each **$R_1$** is independently a linear $C_{1-6}$ alkyl or branched $C_{3-6}$ alkyl; each **$R_2$** is independently a linear $C_{1-6}$ alkyl or a branched $C_{3-6}$ alkyl; each **$R_3$** is independently a linear $C_{1-6}$ alkyl, a branched $C_{3-6}$ alkyl or $C_{1-6}$ (per)fluoroalkyl; **$R_4$** and **$R_5$** are H; **Y** is O; **B** is present, and both **B** and **D** are $CH_2$ and ------ is a single bond; more preferably **$R_1$, $R_2$** and **$R_3$** are each independently selected from the group consisting of methyl, ethyl, propyl and isopropyl; **$R_4$** is H, **Y** is O; **B** is present, and both **B** and **D** are $CH_2$ and ------ is a single bond.

In some embodiments **n** is 1; **$A_2$** is H; **$A_1$** and **$M_2$** form together a heterocyclic group fused to the central 5-membered heterocyclic ring they are attached to; wherein said heterocyclic group formed is a group **Ia:**

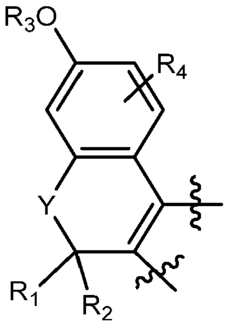

(Ia);

and **$M_1$** is selected from the group consisting of H, 2,6-di-$C_{1-12}$ alkoxyphenyl, Ma, Mb and Mc;
wherein **$R_1$, $R_2$**, **$R_3$**, **$R_4$, Y**, Ma, Mb and Mc are each independently as described in the claims and the present description.

In some embodiments **X** is S, **$A_2$** is H; **$A_1$** and **$M_2$** form together a heterocyclic group fused to the central 5-membered heterocyclic ring they are attached to; wherein said heterocyclic group formed is a group **Ia:**

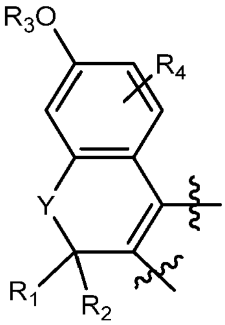

(Ia);

and **$M_1$** is 2,6-di-$C_{1-12}$ alkoxyphenyl or Mc, wherein **Mc, $R_1$, $R_2$**, **$R_3$**, **$R_4$,** and **Y** are each independently as described in the claims and the present description; preferably each **$R_1$** is independently a linear $C_{1-6}$ alkyl or branched $C_{3-6}$ alkyl; each **$R_2$** is independently a linear $C_{1-6}$ alkyl or a branched $C_{3-6}$ alkyl; each **$R_3$** is independently a linear $C_{1-6}$ alkyl, a branched $C_{3-6}$ alkyl or $C_{1-6}$ (per)fluoroalkyl; **$R_4$** is H and **Y** is O; more preferably **$R_1$, $R_2$** and **$R_3$** are each independently selected from the group consisting of methyl, ethyl, propyl and isopropyl; **$R_4$** is H and **Y** is O.

In one embodiment **X** is S, **$A_2$** is H; **$A_1$** and **$M_2$** form together a heterocyclic group fused to the central 5-membered heterocyclic ring they are attached to; wherein said heterocyclic group formed is a group **Ia**

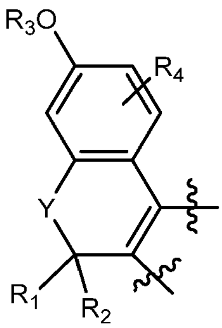

(Ia);

and **$M_1$** is 2,6-di-$C_{1-12}$ alkoxyphenyl, preferably 2,6-di-$C_{1-6}$ alkoxyphenyl;

wherein $R_1$, $R_2$, $R_3$, $R_4$ and **Y** are each independently as described in the claims and the present description; preferably $R_1$ is a linear $C_{1-6}$ alkyl or branched $C_{3-6}$ alkyl; $R_2$ is a linear $C_{1-6}$ alkyl or a branched $C_{3-6}$ alkyl; $R_3$ is a linear $C_{1-6}$ alkyl, a branched $C_{3-6}$ alkyl or $C_{1-6}$ (per)fluoroalkyl; $R_4$ is H and **Y** is O; more preferably $R_1$, $R_2$ and $R_3$ are each independently selected from the group consisting of methyl, ethyl, propyl and isopropyl; $R_4$ is H and **Y** is O.

In another embodiment X is S, $A_2$ is H; $A_1$ and $M_2$ form together a heterocyclic group fused to the central 5-membered heterocyclic ring they are attached to; wherein said heterocyclic group formed is a group **Ia:**

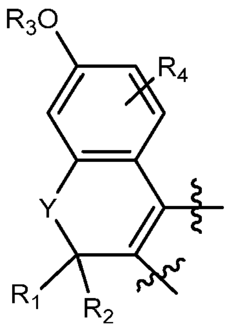

(Ia);

and $M_1$ is Mc, wherein **Mc,** $R_1$, $R_2$, $R_3$, $R_4$, **Y** and **Mc** are each independently as described in the claims and the present description; preferably each $R_1$ is independently a linear $C_{1-6}$ alkyl or branched $C_{3-6}$ alkyl; each $R_2$ is independently a linear $C_{1-6}$ alkyl or a branched $C_{3-6}$ alkyl; each $R_3$ is independently a linear $C_{1-6}$ alkyl, a branched $C_{3-6}$ alkyl or $C_{1-6}$ (per) fluoroalkyl; $R_4$ is H and **Y** is O; more preferably $R_1$, $R_2$ and $R_3$ are each independently selected from the group consisting of methyl, ethyl, propyl and isopropyl; $R_4$ is H and **Y** is O.

In some embodiments, **n** is 1; $A_1$ is H; $M_2$ is selected from the group consisting of H, 2,6-di-$C_{1-12}$ alkoxyphenyl, Ma, Mb and Mc; and $A_2$ and $M_1$ form together a heterocyclic group fused to the central 5-membered heteroaromatic ring they are attached to; wherein said heterocyclic group is selected from the group consisting of Ib, Ic and Id:

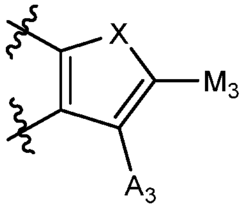

(Ib),

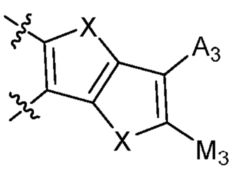

(Ic) and

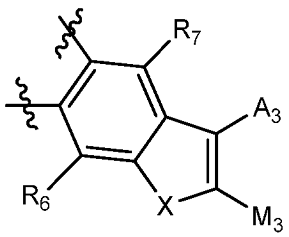

(Id);

wherein $A_3$ is H and $M_3$ is selected from the group consisting of H, 2,6-di-$C_{1-12}$ alkoxyphenyl, Ma, Mb and Mc, and wherein **at least one** of $M_2$ and $M_3$ is selected from the group consisting of Ma, Mb and Mc; and wherein $R_6$, $R_7$, **X,** Ma, Mb and Mc are each independently as described in the claims and the present description.

In some embodiments n is 1; $A_1$ is H; $M_2$ is selected from the group consisting of H, 2,6-di-$C_{1-12}$ alkoxyphenyl, Ma, Mb and Mc; and $A_2$ and $M_1$ form together a heterocyclic group fused to the central 5-membered heteroaromatic ring they are attached to; wherein said heterocyclic group is Ib:

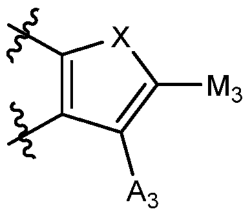

(Ib);

wherein $A_3$ is H and $M_3$ is selected from the group consisting of H, 2,6-di-$C_{1-12}$ alkoxyphenyl, Ma, Mb and Mc, and wherein **at least one** of $M_2$ and $M_3$ is selected from the group consisting of Ma, Mb and Mc; and wherein **X,** Ma, Mb and Mc are each independently as described in the claims and the present description.

In some embodiments **X** is S, **n** is 1; $A_1$ is H; $M_2$ is Ma and $A_2$ and $M_1$ form together a heterocyclic group fused to the central 5-membered heteroaromatic ring they are attached to; wherein said heterocyclic group is **Ib;**

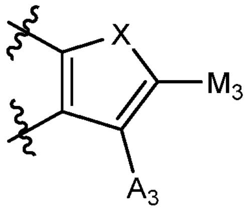 (Ib);

wherein $\mathbf{A_3}$ is H and $\mathbf{M_3}$ is Ma, and wherein Ma, $\mathbf{R_1}$, $\mathbf{R_2}$, $\mathbf{R_3}$, $\mathbf{R_4}$, ------, **Y**, **B** and **D** are each independently as described in the claims and the present description, preferably wherein each $\mathbf{R_1}$ is independently a linear $C_{1-6}$ alkyl or branched $C_{3-6}$ alkyl; each $\mathbf{R_2}$ is independently a linear $C_{1-6}$ alkyl or a branched $C_{3-6}$ alkyl; each $\mathbf{R_3}$ is independently a linear $C_{1-6}$ alkyl, a branched $C_{3-6}$ alkyl or $C_{1-6}$ (per)fluoroalkyl; $\mathbf{R_4}$ is H, **Y** is O; **B** is present, and both **B** and **D** are $CH_2$ and ------ is a single bond; more preferably wherein $\mathbf{R_1}$, $\mathbf{R_2}$ and $\mathbf{R_3}$ are each independently selected from the group consisting of methyl, ethyl, propyl and isopropyl; $\mathbf{R_4}$ is H, **Y** is O; **B** is present, and both **B** and **D** are $CH_2$ and ------ is a single bond.

In some embodiments **n** is 1; $\mathbf{A_1}$ is H; $\mathbf{M_2}$ is selected from the group consisting of H, 2,6-di-$C_{1-12}$ alkoxyphenyl, Ma, Mb and Mc; and $\mathbf{A_2}$ and $\mathbf{M_1}$ form together a heterocyclic group fused to the central 5-membered heteroaromatic ring they are attached to; wherein said heterocyclic group is **Ic**:

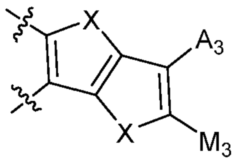 (Ic);

and wherein $\mathbf{A_3}$ is H and $\mathbf{M_3}$ is selected from the group consisting of H, 2,6-di-$C_{1-12}$ alkoxyphenyl, Ma, Mb and Mc, wherein **at least one** of $\mathbf{M_2}$ and $\mathbf{M_3}$ is selected from the group consisting of Ma, Mb and Mc; and wherein **X,** Ma, Mb and Mc are each independently as described in the claims and the present description.

In some embodiments **n** is 1; $\mathbf{A_1}$ is H; $\mathbf{M_2}$ is selected from the group consisting of H, 2,6-di-$C_{1-12}$ alkoxyphenyl, Ma, Mb and Mc; and $\mathbf{A_2}$ and $\mathbf{M_1}$ form together a heterocyclic group fused to the central 5-membered heteroaromatic ring they are attached to; wherein said heterocyclic group is **Id:**

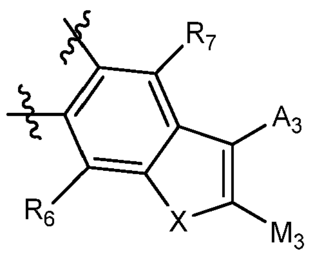 (Id);

and wherein $\mathbf{A_3}$ is H and $\mathbf{M_3}$ is selected from the group consisting of H, 2,6-di-$C_{1-12}$ alkoxyphenyl, Ma, Mb and Mc, wherein **at least one** of $\mathbf{M_2}$ and $\mathbf{M_3}$ is selected from the group consisting of Ma, Mb and Mc; and wherein $\mathbf{R_6}$, $\mathbf{R_7}$, **X,** Ma, Mb and Mc are each independently as described in the claims and the present description.

Some preferred embodiments refer to a compound of formula (I) as defined in the claims and the present description, selected from the group consisting of compound 1, compound 2, compound 3, compound 4, compound 5, compound 6, compound 7, compound 8, compound 9, compound 10, compound 11, compound 12, compound 13, compound 14, compound 15, compound 16 and compound 17, herein disclosed:

| | |
|---|---|
| Compound 1 | 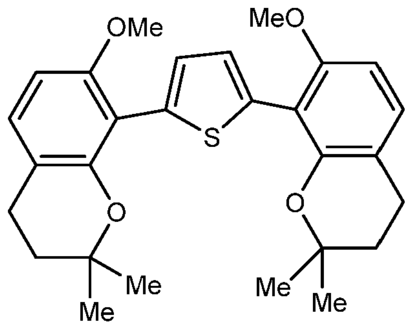 |
| Compound 2 | 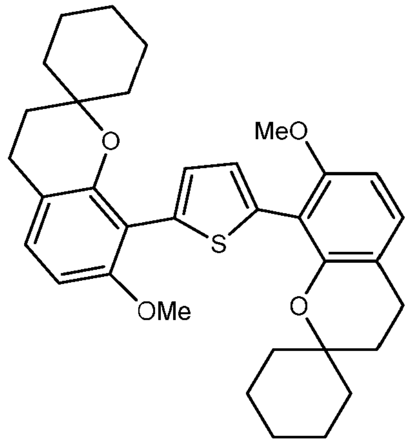 |
| Compound 3 | 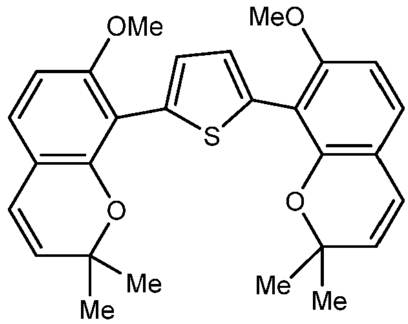 |
| Compound 4 | 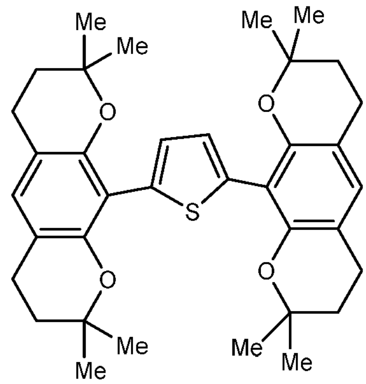 |

(continued)

| | |
|---|---|
| Compound 5 | 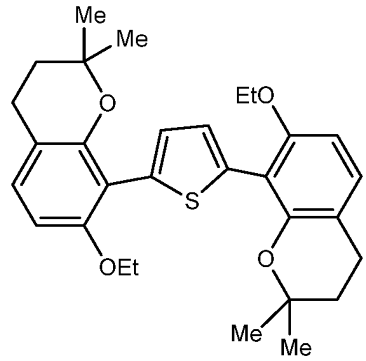 |
| Compound 6 | 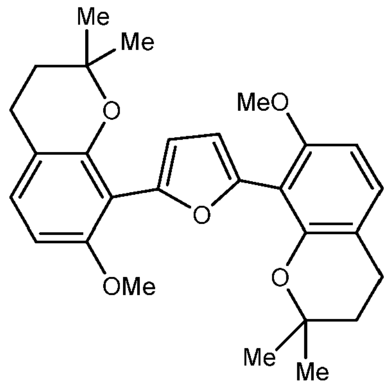 |
| Compound 7 | 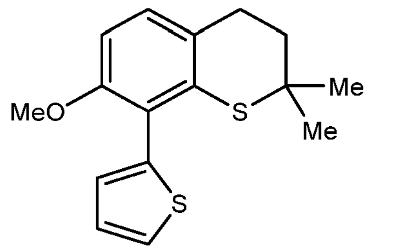 |
| Compound 8 | 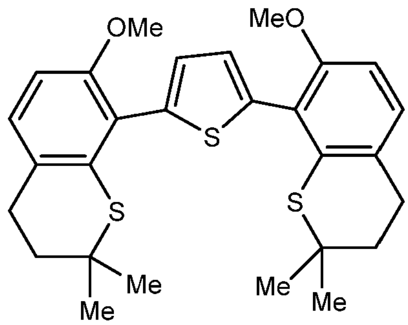 |
| Compound 9 | 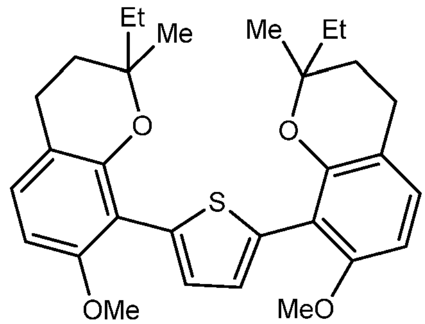 |

(continued)

| | |
|---|---|
| Compound 10 | 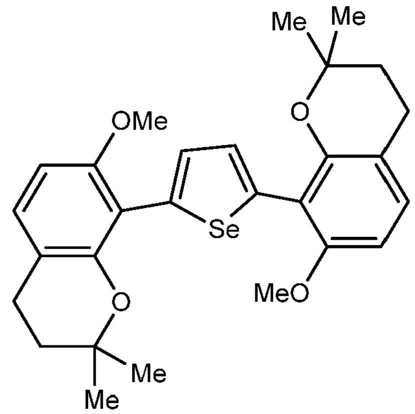 |
| Compound 11 | 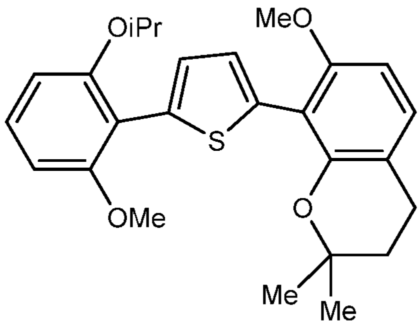 |
| Compound 12 | 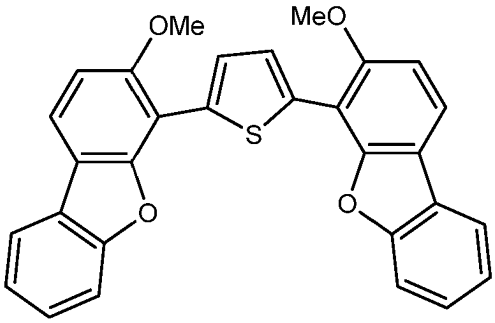 |
| Compound 13 | 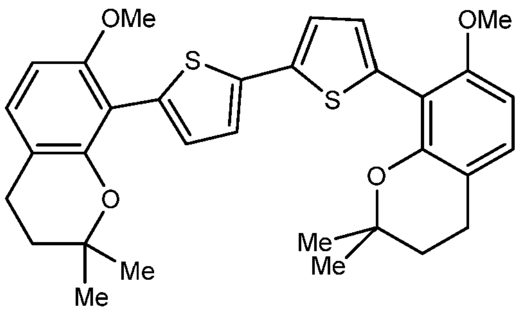 |
| Compound 14 | 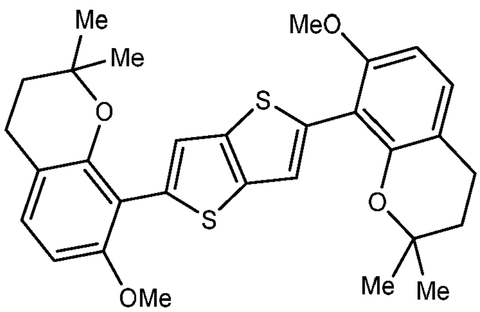 |

(continued)

| | |
|---|---|
| Compound 15 | 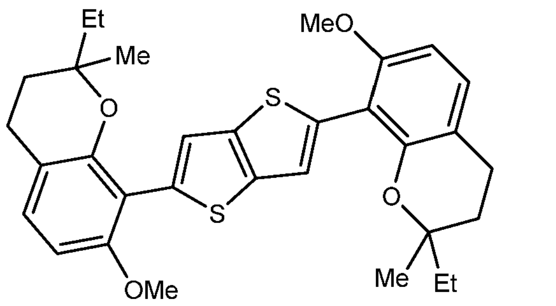 |
| Compound 16 | 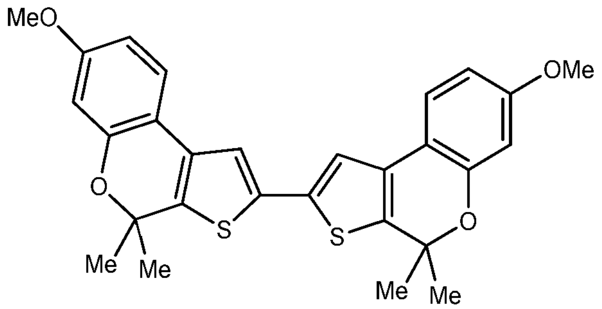 |
| Compound 17 | 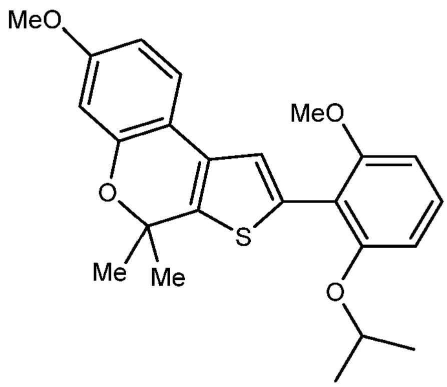 |

**Electrochromic composition**

The present invention also relates to electrochromic compositions comprising at least one compound of formula (I) as described in the claims and the present description and a host medium.

The composition may also comprise at least one or more additional reducing electrochromic compound. The reducing compound may also be an electrochromic compound. Example of reducing compounds include 5,10-dihydrophenazine, phenothiazine, phenoxazine, N,N,N',N'-tetramethyl-p-phenylenediamine, thioanthrene, tetrathiafulvalene, ferrocene and their derivatives.

Preferably, the additional compound has a redox potential close to the compound of formula (I).

One or more oxidizing electrochromic compounds can be added to the composition of the invention. For example, the additional electrochromic compound can be selected from anthraquinones, alkylviologens, arylviologens, alkylarylviologens and cationic derivatives of the nitrogen containing heterocyclic systems including benzazoles, condensed azoles, benzoxazoles, benzoisoxazoles, imidazo[1,2-o]pyridines, benzothiazoles, 2,1,3-benzothiadiazoles, benzoisothiazoles, imidazoles, benzimidazoles, imidazole pyridines, phenantroimidazoles, benzoselenadiazoles, benzoselenazoles pyridopyrazines, dihydropyridopyrazines, dipyridopyrazines, pyridopyrazinoquinoline, dihydropyridopyrazinoquinoline, benzimidazolepyridopyrazine, dipyridinylpyrazines, triazines, dipyridinyltriazines, thienopyrazines, and derivatives. In particular derivatives thereof comprising pyridine, N-alkyl or N-aryl pyridinium, quinoline, or N-alkyl or N-aryl quinolinium, isoquinoline, N-alkyl or N-aryl isoquinolinium substitutions.

The composition of the invention may comprise a host medium that may be a fluid, a mesomorphous medium or a gel. The host medium is introduced in the composition of the invention to dissolve the electrochromic compounds. The host medium is preferably selected from the group consisting of organic solvents, liquid crystals, polymers, liquid crystal polymers and mixtures thereof.

Examples of suitable organic solvents that can be used as host medium are redox-compatible solvents which cannot react with the electrochromic compounds of the composition, such as ethylene carbonate, propylene carbonate, γ-butyrolactone, γ-valerolactone, acetronitrile, propionitrile, benzonitrile, glutaronitrile, methylglutaronitrile, dimethylformamide, N-methylpyrrolidone, sulfolane, 3-methyl sulfolane, benzene, toluene, methyl ethyl ketone, acetone, ethanol, tetrahydrofurfuryl alcohol, 2-methoxyethyl ether, xylene, cyclohexane, 3-methylcyclohexanone, ethyl acetate, ethyl phenylacetate, tetrahydrofuran, methanol, methyl propionate, ethylene glycol, ethylene carbonate, ionic liquids, and mixtures thereof. Preference is given to carbonates and particularly propylene carbonate.

Examples of suitable liquid crystals that can be used as host medium are nematic or chiral nematic media.

Examples of suitable polymers that can be used as host medium are polymers which are soluble with the solvent, in particular PMMA or other acrylate polymers, polyurethane, polyethylene oxide, polypropylene oxide, polyvinyl acetate, poly(N-vinyl pyrrolidone), and polyvinylidene fluoride.

Examples of suitable liquid crystal polymers that may be used as host medium are Merck RM257 (Merck), LC242 (BASF) or SLM 90519 (Wacker). These liquid crystal polymers are generally used in combination with an organic solvent, for example one of the organic solvents mentioned above.

## Electrochromic device

The present invention also relates to an electrochromic device comprising a compound of formula (I) or a composition according to the invention. Said device may be selected from an optical article, preferably an optical lens, or an optical filter, a window, preferably an aircraft window, a visor, a mirror, a variable transmission device and a display element or device, in particular a segmented or matrix display. Preferably, the device of the invention is an optical article, more preferably an optical lens, and even more preferably an ophthalmic lens.

Non-limiting examples of ophthalmic lens include corrective and non-corrective lenses, including single vision or multi-vision lenses, which may be either segmented or non-segmented, as well as other elements used to correct, protect, or enhance vision, including without limitation contact lenses, intra-ocular lenses, sunglasses, ski goggles, sport lenses, augmented reality and virtual reality lenses, magnifying lenses, protective lenses and visors, for example motorcycle visors and helmets. Non-limiting examples of display elements and devices include screens and monitors. Non-limiting examples of windows include automotive, marine, aircraft or building windows. Non limiting examples of variable transmission devices include filters, shutters, and optical switches. Variable transmission devices (lenses, goggles, visors, etc.) refer in general to a device which can quickly change between a high-transmission (or "clear") state and a low-transmission (or "dark") state.

Preferably, the device of the invention comprises a mechanism for holding the compound or composition of the invention in a mechanically stable environment. More preferably, said device may comprise a pair of opposed substrates having a gap there between for receiving the mixture of the host medium and said compound or said composition of the present invention, and a frame for holding said pair of substrates adjacent one another.

The device of the present invention may thus comprise an optical component provided with at least one transparent cell arrangement juxtaposed in a parallel direction to the surface thereof, as disclosed in WO 2006/013250, each cell being tightly closed and containing at least one compound or composition of the present invention.

Other devices according to the invention can be devices as described in FR 2937154 or FR2950710 comprising at least one compound or composition of the invention.

This invention will be further illustrated by the following non-limiting examples which are given for illustrative purposes only and should not restrict the scope of the appended claims.

## Examples

## Example 1: Synthesis of exemplary compounds of formula (I)

The synthesis of chroman-4-ones by either the Kabbe base-catalysed condensation protocol between 2'-hydroxyacetophenones and ketones (H. J. Kabbe, Synthesis, 1978, 886-887) or by the acid-catalysed condensation of phenols with substituted acrylic acids (K. Meraz et al., Tetrahedron Letters, 2016, 57, 5057-5061 ; R. Chaturvedi et al., Indian Journal of Chemistry, Section B: 1992, 31B, 338-339) are well established protocols. The latter reaction, when applied to thiophenols, readily affords thiochroman-4-ones (S. E. Clayton et al., Tetrahedron, 1993, 49, 939-946). The subsequent transformation of the carbonyl function of the chroman-4-ones and thiochroman-4-ones by reduction to the (thio)chroman-4-ol and subsequent acid-catalysed dehydration enables the synthesis of chromenes (P. J. Brogden et al., Journal of the Chemical Society, Perkin Transactions 1, 1983, 827-830) and thiochromenes (J. Tercio et al., Synthesis, 1987, 2, 149-153). Alternatively, complete reduction of the carbonyl function can be accomplished leading to the chroman system (B. M. Trost et al., Journal of the American Chemical Society, 1998, 120, 9074-9075; A. Bermejo et al., Bioorganic & Medicinal Chemistry, 2019, 27, 115162) and thiochroman system (B. Robillard et al., Journal of Organic Chemistry, 1986,

51, 1700-1704). Catalytic hydrogenation of chromenes and thiochromenes can be accomplished and provides an alternative route to chromans and thiochromans (S. R. Fletcher et al., Journal of Medicinal Chemistry, 2002, 45, 492-503).

The concept of ortho-metalation or heteroatom directed metalation is a well-established synthetic chemistry protocol for the introduction of substituents adjacent to heteroatoms such as O or S, or functional groups, for example, ethers, amides, thioethers, alcohols, on aromatic rings wherein such functional groups have the ability to coordinate to an organolithium reagent and enable reaction with an electrophile such that the electrophile is introduced into a site adjacent to the coordinating group (V. Snieckus, Chemical Reviews, 1990, 90, 879-933; V. Snieckus et al., in 'Modern Arene Chemistry', Ed. D. Astruc, 2002, 330-367; V. Snieckus, Pure and Applied Chemistry, 1990, 62, 2047-2056; V. Snieckus, Bulletin de la Societe Chimique de France,1988, 67-78; V. Snieckus et al., in 'Handbook of C-H Transformations', Ed. G. Dyker, 2005, 1, 106-118, 262-264).

The ability of the oxygen heteroatom of a benzopyran ring to introduce a substituent into the peri position (C-8) has been described (M. Hallet et al., Bulletin des Societes Chimiques Belges, 1952, 61, 33-43; L. A. Paquette et al., Tetrahedron Letters, 1993, 34, 3235-3238; R. J. Bethune et al., Journal of the Chemical Society, Perkin Transactions 1, 1994, 1925-1933).

Employing the foregoing published strategies readily provided access to a range of substituted chromans, thiochromans and chromenes. Their subsequent heteroatom directed metalation was accomplished and quenching the generated organolithium with, for example, trimethyl borate afforded the substituted chroman, thiochroman and chromene substituted 8-boronic acids. The coupling of the foregoing substituted chroman, thiochroman and chromene substituted 8-boronic acids with a range of dibromo substituted thiophenes, selenophenes and dibromo substituted bithiophenes proceeded smoothly using the widely established Suzuki cross-coupling methodology (N. Miyaura et al., Chemical Reviews, 1995, 95, 2457-2483)) to afford the new electrochromic compounds as depicted by the following reaction schemes (reaction schemes A and A.1 to A.4, and reaction schemes B and B.1 to B.15). The compounds of formula (I) and their intermediates had spectroscopic data in full accord with their proposed structures.

**A. Synthesis of (7-Methoxy-2,2-dimethylchroman-8-yl)boronic acid**

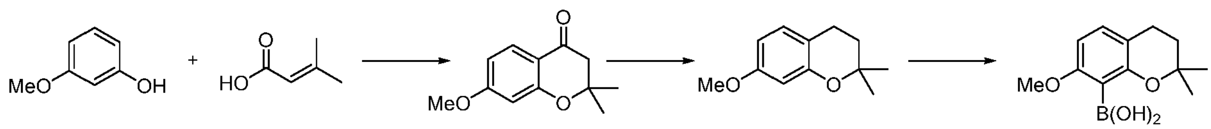

*Reaction scheme A*

3-Methoxyphenol (5.5 mL, 50.1 mmol) and 3,3-dimethylacrylic acid (5.51 g, 55.1 mmol, 1.1 equiv.) were dissolved in $MeSO_3H$ (50 mL) and stirred at 70 °C for 4 hours. The reaction was allowed to cool, poured onto 200 mL ice-water and stirred for 30 min. The product was extracted with $Et_2O$ (3 × 100 mL) and the combined organic portions were washed with 5 % NaOH solution (2 × 50 mL), water (100 mL) and brine (100 mL). The organic portion was dried ($Na_2SO_4$) and the solvent removed *in vacuo* to afford an orange oil which solidified. The solid was recrystallised from EtOH to afford 7-methoxy-2,2-dimethylchroman-4-one as colourless crystals (8.47g, 82 %). m.p. 76-77 °C (lit. m.p. 79-81 °C [K. Meraz et al., Tetrahedron Letters, 2016, 57, 5057 - 5061]). $\nu_{max}$ 3004, 2985, 2962, 2907, 1665, 1600, 1574, 1261, 1022, 839 $cm^{-1}$. $\delta_H$ (400 MHz, $CDCl_3$) 1.45 [6H, s, C(C*H*$_3$)$_2$], 2.66 (2H, s, C*H*$_2$), 3.82 (3H, s, OC*H*$_3$), 6.37 (1H, d, *J* = 2.4 Hz, 8-*H*), 6.53 (1H, dd, *J* = 2.4, 8.8 Hz, 6-*H*), 7.89 (1H, d, *J* = 8.8 Hz, 5-*H*). $\delta_C$ (100 MHz, $CDCl_3$) 26.7 (2 × $CH_3$), 48.6 ($CH_2$), 55.6 ($CH_3$), 79.6 (C), 101.1 (CH), 109.3 (CH), 114.1 (C), 128.3 (CH), 162.0 (C), 166.2 (C), 191.1 (C). HRMS (ESI-TOF) found $[M+H]^+$ = 207.1019 $C_{12}H_{15}O_3$ requires $[M+H]^+$ = 207.1016.

7-Methoxy-2,2-dimethylchroman-4-one (1.00 g, 4.8 mmol) was added to Zn dust (7.93 g, 121 mmol, 25 equiv.) in AcOH (30 mL) and stirred at reflux overnight. The reaction was allowed to cool to r.t. and filtered through Celite. The reaction mixture was diluted with EtOAc (200 mL) and PhMe (200 mL). The solvent was removed under reduced pressure. The residue was dissolved in DCM and the solvent removed, the insoluble material was discarded. The product was purified by flash column chromatography [10 % EtOAc in hexane] to afford 7-methoxy-2,2-dimethylchroman as a colourless oil (0.81 g, 86.9 %). $\nu_{max}$ 2973, 2932, 1619, 1580, 1504, 1147, 1119 $cm^{-1}$. $\delta_H$ (400 MHz, $CDCl_3$) 1.33 [6H, s, C(C*H*$_3$)$_2$], 1.79 [2H, t, *J* = 6.7 Hz, $CH_2$C*H*$_2$C($CH_3$)$_2$], 2.71 [2H, t, *J* = 6.7 Hz, C*H*$_2$$CH_2$C($CH_3$)$_2$], 3.75 (3H, s, OC*H*$_3$), 6.36 (1H, d, *J* = 2.6 Hz, 8-*H*), 6.43 (1H, dd, *J* = 2.6, 8.3 Hz, 6-*H*), 6.95 (1H, d, *J* = 8.3 Hz, 5-*H*). $\delta_C$ (100 MHz, $CDCl_3$) 21.7 ($CH_2$), 26.9 (2 × $CH_3$), 33.0 ($CH_2$), 55.2 ($CH_3$), 74.3 (C), 101.7 (CH), 106.9 (CH), 113.0 (C), 129.9 (CH), 154.7 (C), 159.1 (C). HRMS (ESI-TOF) found $[M+H]^+$ = 193.1230 $C_{12}H_{17}O_2$ requires $[M+H]^+$ = 193.1223.

To a solution of 7-methoxy-2,2-dimethylchroman (1.94 g, 10.1 mmol) in anhydrous THF (35 mL) at -20 °C under $N_2$ was added *n*-BuLi [2.5 M in hexanes, 4.9 mL, 12.2 mmol, 1.2 equiv.] dropwise over 5 min followed by the addition of

TMEDA (1.84 mL, 12.2 mmol, 1.2 equiv.) in one portion. The reaction mixture was stirred at -20 °C for 30 min and allowed to warm to r.t., the reaction was stirred for a further 3.5 h. The reaction mixture was cooled to -78 °C and $B(OMe)_3$ (2.2 mL, 20.0 mmol, 2 equiv.) was added. The mixture was allowed to warm to r.t. and stirred for 16 h. The reaction was quenched with 2 M HCl at 0 °C and stirred for 30 min. The product was extracted with EtOAc (3 × 50 mL) and washed with water and brine. The organic layer was dried ($Na_2SO_4$) and the solvent was removed under reduced pressure. The residue was triturated with hexane and the solid isolated by vacuum filtration to afford (7-methoxy-2,2-dimethylchroman-8-yl)boronic acid as a white crystalline solid (1.24 g, 52 %). m.p. 100-101 °C. $v_{max}$ 3513, 3473, 2971, 2943, 2924, 1599, 1582, 1313, 1085, 798, 690 $cm^{-1}$. $\delta_H$ (400 MHz, $CDCl_3$) 1.40 [6H, s, C(C$H_3$)$_2$], 1.83 [2H, t, *J* = 6.8 Hz, $CH_2CH_2C(CH_3)_2$], 2.76 [2H, t, *J* = 6.8 Hz, $CH_2CH_2C(CH_3)_2$], 3.88 (3H, s, OC$H_3$), 6.52 (1H, d, *J* = *8.4* Hz, 6-*H*), 7.12 (1H, d, *J* = 8.4 Hz, 5-*H*), 7.39 [2H, s, B(O*H*)$_2$]. $\delta_C$ (100 MHz, $CDCl_3$) 22.0 ($CH_2$), 27.0 (2 × $CH_3$), 32.3 ($CH_3$), 55.9 ($CH_2$), 76.4 (C), 103.4 (CH), 106.0 (C)*, 114.4 (C), 133.0 (CH), 159.5 (C), 163.9 (C). HRMS (ESI-TOF) found $[M+NH_4]^+$ = 253.1596 $C_{12}H_{21}BNO_4$ requires $[M+NH_4]^+$ = 253.1594. (*by HMBC)

**A.1. Synthesis of 2,5-bis(7-methoxy-2,2-dimethylchroman-8-yl)thiophene - Compound 1**

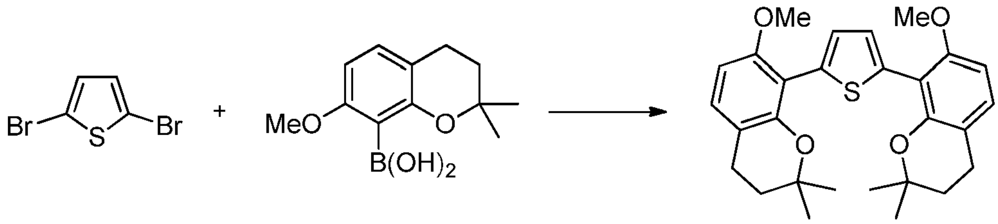

*Reaction scheme A.1*

2,5-Dibromothiophene (0.28 mL, 2.5 mmol), (7-methoxy-2,2-dimethylchroman-8-yl)boronic acid (1.48 g, 6.3 mmol, 2.5 equiv.), potassium carbonate (2.07 g, 15 mmol, 6 equiv.) and tetrakis(triphenylphosphine)palladium(0) (0.29 g, 0.3 mmol, 10 mol%) were added to degassed PhMe (20 mL) and EtOH (20 mL), the reaction mixture was stirred at reflux under $N_2$ for 3 h. The reaction was allowed to cool to r.t., poured onto water (80 mL) and extracted with DCM (3 × 20 mL). The combined organic portions were dried ($Na_2SO_4$) and the solvent removed. The product was purified by flash column chromatography [10 % EtOAc in hexane] to afford 2,5-bis(7-methoxy-2,2-dimethylchroman-8-yl)thiophene as a yellow oil which was crystallised from pentane (1.16 g, 100%). m.p. 118 - 120 °C. $v_{max}$ 2973, 2930, 2831, 1481, 1417, 1154, 1122, 1104, 1082, 804 $cm^{-1}$. $\delta_H$ (400 MHz, $CDCl_3$) 1.40 [12H, s, 2 × C(C$H_3$)$_2$], 1.82 [4H, t, *J* = 6.8 Hz, 2 × C$H_2$C(CH$_3$)$_2$], 2.80 [4H, t, *J* = 6.8 Hz, 2 × C$H_2$CH$_2$C(CH$_3$)$_2$], 3.84 (6H, s, 2 × OC$H_3$), 6.54 (2H, d, *J* = 8.4 Hz, 2 × 6-*H*), 6.94 (2H, d, *J* = 8.4 Hz, 2 × 5-*H*), 7.62 (2H, s, 3-*H*, 4-*H*). $\delta_C$ (100 MHz, $CDCl_3$) 22.5 (2 × $CH_2$), 27.0 (4 × $CH_3$), 32.8 (2 × $CH_2$), 56.0 (2 × $CH_3$), 74.9 (2 × C), 103.5 (2 × CH), 112.9 (2 × C), 113.9 (2 × C), 127.7 (2 × CH), 127.8 (2 × CH), 133.9 (2 × C), 151.1 (2 × C), 156.0 (2 × C). HRMS (ESI-TOF) found $[M+K]^+$ = 503.1650 $C_{28}H_{32}KO_4S$ requires $[M+K]^+$ = 503.1653.

**A.2. Synthesis of 8-[5-(2-isopropoxy-6-methoxyphenyl)thiophenyl]-7-methoxy-2,2-dimethylchromane - Compound 11**

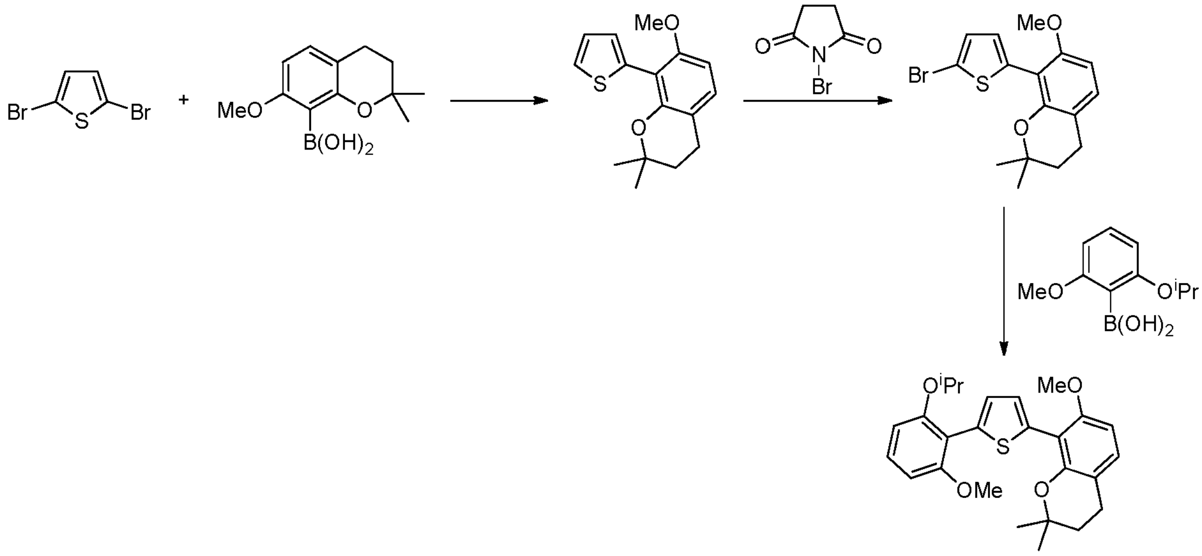

*Reaction scheme A.2*

2,5-Dibromothiophene (0.43 mL, 3.9 mmol), (7-methoxy-2,2-dimethylchroman-8-yl)boronic acid (1.00 g, 4.2 mmol, 1.1 equiv.), potassium carbonate (3.19 g, 23.1 mmol, 6 equiv.) and tetrakis(triphenylphosphine)palladium(0) (0.44 g, 0.4 mmol, 10 mol%) were added to degassed PhMe (30 mL) and EtOH (30 mL), the reaction mixture was stirred at reflux under $N_2$ for 3 h. The reaction was allowed to cool to r.t., poured onto water (80 mL) and extracted with DCM (3 × 20 mL). The combined organic portions were dried ($Na_2SO_4$) and the solvent removed. The product was purified by flash column chromatography [5 % EtOAc in hexane] to afford 7-methoxy-2,2-dimethyl-8-(thiophen-2-yl)chromane as a fluorescent yellow oil (0.76 g, 72%). $\nu_{max}$ 2972, 2930, 2835, 1601, 1480, 1102, 689 $cm^{-1}$. $\delta_H$ (400 MHz, $CDCl_3$) 1.37 [6H, s, $C(CH_3)_2$], 1.81 [2H, t, *J* = 6.8 Hz, $CH_2CH_2C(CH_3)_2$], 2.79 [2H, t, *J* = 6.8 Hz, $CH_2CH_2C(CH_3)_2$], 3.82 (3H, s, $OCH_3$), 6.53 (1H, d, *J* = 8.4 Hz, 6-Ch-*H*), 6.97 (1H, d, *J* = 8.4 Hz, 5-Ch-*H*), 7.08 (1H, dd, *J* = 3.7, 5.2 Hz, 4-Th-*H*), 7.34 (1H, dd, *J* = 1.1, 5.2 Hz, 5-Th-*H*), 7.58 (1H, dd, *J* = 1.1, 3.7 Hz, 3-Th-*H*). $\delta_C$ (100 MHz, $CDCl_3$) 22.3 ($CH_2$), 26.9 (2 × $CH_3$), 32.7 ($CH_2$), 55.9 ($CH_3$), 75.1 (C), 103.2 (CH), 112.0 (C), 114.0 (C), 124.8 (CH), 125.6 (CH), 128.3 (CH), 128.6 (CH), 134.5 (C), 151.6 (C), 155.8 (C).

7-Methoxy-2,2-dimethyl-8-(thiophen-2-yl)chromane (0.70 g, 2.5 mmol) was dissolved in anhydrous THF (10 mL) followed by the addition of a solution of NBS (0.45 g, 2.5 mmol, 1 equiv.) in anhyd. THF (15 mL). The reaction was stirred at 35 °C under $N_2$ for 6 h and allowed to cool to r.t. The reaction was poured onto water (100 mL) and the product extracted with DCM (3 × 50 mL), the combined organic layers were dried ($Na_2SO_4$) and the solvent removed *in vacuo* to afford 8-(5-bromothiophen-2-yl)-7-methoxy-2,2-dimethylchromane as a yellow oil which slowly solidified (0.86 g, 95%). m.p. 90-92 °C. $\nu_{max}$ 3077, 2972, 2910, 1688, 1429, 1105, 811, 796 $cm^{-1}$. $\delta_H$ (400 MHz, $CDCl_3$) 1.41 [6H, s, $C(CH_3)_2$], 1.82 [2H, t, *J* = 6.8 Hz, $CH_2CH_2C(CH_3)_2$], 2.79 [2H, t, *J* = 6.8 Hz, $CH_2CH_2C(CH_3)_2$], 3.85 (3H, s, $OCH_3$), 6.53 (1H, d, *J* = 8.3 Hz, 6-Ch-*H*), 6.97 (1H, d, *J* = 8.3 Hz, 5-Ch-*H*), 7.03 (1H, d, *J* = 4.0 Hz, 4-Th-*H*), 7.49 (1H, d, *J* = 4.0 Hz, 3-Th-*H*). $\delta_C$ (100 MHz, $CDCl_3$) 22.3 ($CH_2$), 26.9 (2 × $CH_3$), 32.6 ($CH_2$), 55.9 ($CH_3$), 75.6 (C), 103.3 (CH), 111.3 (C), 112.0 (C), 114.0 (C), 128.4 (CH), 128.6 (CH), 128.9 (CH), 136.5 (C), 151.4 (C), 155.7 (C).

8-(5-Bromothiophen-2-yl)-7-methoxy-2,2-dimethylchromane (0.25 g, 0.7 mmol), (2-isopropoxy-6-methoxyphenyl)boronic acid (0.27 g, 1.3 mmol, 1.8 equiv.), potassium carbonate (0.59 g, 4.2 mmol, 6 equiv.) and tetrakis(triphenylphosphine)palladium(0) (0.09 g, 0.1 mmol, 10 mol%) were added to degassed PhMe (20 mL) and EtOH (20 mL), the reaction mixture was stirred at reflux under $N_2$ for 16 h. The reaction was allowed to cool to r.t., poured onto water (50 mL) and extracted with DCM (3 × 20 mL). The combined organic portions were dried ($Na_2SO_4$) and the solvent removed. The product was purified by flash column chromatography [5 % EtOAc in hexane] to afford 8-[5-(2-isopropoxy-6-methoxyphenyl)thiophenyl]-7-methoxy-2,2-dimethylchromane as a yellow glass (0.22 g, 71%). $\nu_{max}$ 2973, 2931, 2834, 1582, 1463, 1248, 1102, 1073, 805 $cm^{-1}$. $\delta_H$ (400 MHz, $CDCl_3$) 1.37 [6H, d, *J* = 6.0 Hz, $CH(CH_3)_2$], 1.42 [6H, s, $C(CH_3)_2$], 1.85 [2H, t, *J* = 6.8 Hz, $CH_2CH_2C(CH_3)_2$], 2.82 [2H, t, *J* = 6.8 Hz, $CH_2CH_2C(CH_3)_2$], 3.87 (6H, m, 2 × $OCH_3$), 4.58 [1H, sep, *J* = 6.0 Hz, $CH(CH_3)_2$], 6.57 (1H, d, *J* = 8.4 Hz, 6-Ch-*H*), 6.66 (1H, d, *J* = 8.3 Hz, 5-Ar-*H*), 6.69 (1H, d, J= 8.3 Hz, 3-Ar-*H*), 6.97 (1H, d, J= 8.4 Hz, 5-Ch-*H*), 7.20 (1H, t, *J* = 8.3 Hz, 4-Ar-*H*), 7.55 (1H, d, *J* = 4.0 Hz, 4-Th-*H*), 7.67 (1H, d, *J* = 4.0 Hz, 3-Th-*H*). $\delta_C$ (100 MHz, $CDCl_3$) 22.2 (2 × $CH_3$), 22.4 ($CH_2$), 27.0 (2 × $CH_3$), 32.7 ($CH_2$), 55.9 ($CH_3$), 56.0 ($CH_3$), 71.4 (CH), 75.0 (C), 103.5 (CH), 104.4 (CH), 108.0 (CH), 112.8 (C), 113.9 (C), 115.0 (C), 127.6 (CH), 127.8 (3 × CH), 133.5 (C), 134.3 (C), 151.6 (C), 155.9 (C), 156.0 (C), 158.0 (C). HRMS (Jetstream) found $[M+H]^+$ = 439.1938 $C_{26}H_{31}O_4S$ requires $[M+H]^+$ = 439.1938.

**A.3. Synthesis of 2,5-bis(7-methoxy-2,2-dimethylchroman-8-yl)selenophene - Compound 10**

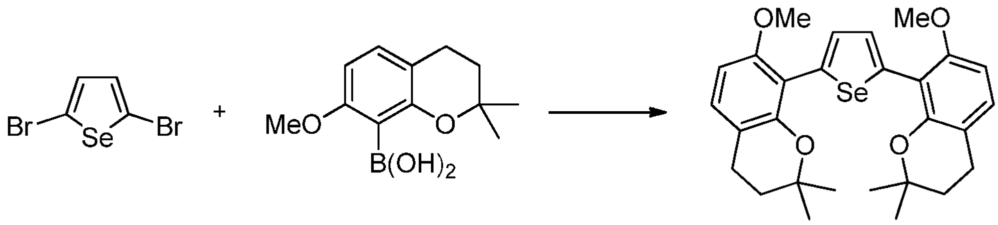

*Reaction scheme A.3*

2,5-Dibromoselenophene (0.4 mL, 3.5 mmol), (7-methoxy-2,2-dimethylchroman-8-yl)boronic acid (1.72 g, 7.3 mmol, 2.1 equiv.), potassium carbonate (2.88 g, 21 mmol, 6 equiv.) and tetrakis(triphenylphosphine)palladium(0) (0.40 g, 0.3 mmol, 10 mol%) were added to degassed PhMe (25 mL) and EtOH (25 mL), the reaction mixture was stirred at reflux under $N_2$ for 3 h. The reaction was allowed to cool to r.t., poured onto water (80 mL) and extracted with DCM (3 × 20 mL). The combined organic portions were dried ($Na_2SO_4$) and the solvent removed. The product was purified by flash column chromatography [5 % EtOAc in hexane] to afford 2,5-bis(7-methoxy-2,2-dimethylchroman-8-yl)selenophene as an orange glass (0.20 g, 11%). $v_{max}$ 2970, 2930, 1477, 1415, 1152, 1101, 810, 787 $cm^{-1}$. $\delta_H$ (400 MHz, $CDCl_3$) 1.43 [12H, s, 2 × C(C*H*$_3$)$_2$], 1.83 [4H, t, *J* = 6.8 Hz,2 × C*H*$_2$C(CH$_3$)$_2$], 2.80 [4H, t, *J* = 6.8 Hz, 2 × C*H*$_2$CH$_2$C(CH$_3$)$_2$], 3.86 (6H, s, 2 × OC*H*$_3$), 6.54 (2H, d, *J* = 8.4 Hz, 2 × 6-*H*), 6.91 (2H, d, *J* = 8.4 Hz, 2 × *5-H),* 8.07 (2H, s, 3-*H,* 4-*H*). $\delta_C$ (100 MHz, $CDCl_3$) 22.5 (2 × $CH_2$), 27.1 (4 × $CH_3$), 32.8 (2 × $CH_2$), 55.9 (2 × $CH_3$), 75.2 (2 × C), 103.7 (2 × CH), 113.9 (2 × C), 115.0 (2 × C), 127.2 (2 × CH), 130.2 (2 × CH), 138.6 (2 × C), 150.9 (2 × C), 155.5 (2 × C). HRMS (Jetstream) found $[M+H]^+$ = 513.1539 $C_{28}H_{33}O_4Se$ requires $[M+H]^+$ = 513.1539.

**A.4. Synthesis of 5,5'-bis(7-methoxy-2,2-dimethylchroman-8-yl)-2,2'-bithiophene - Compound 13**

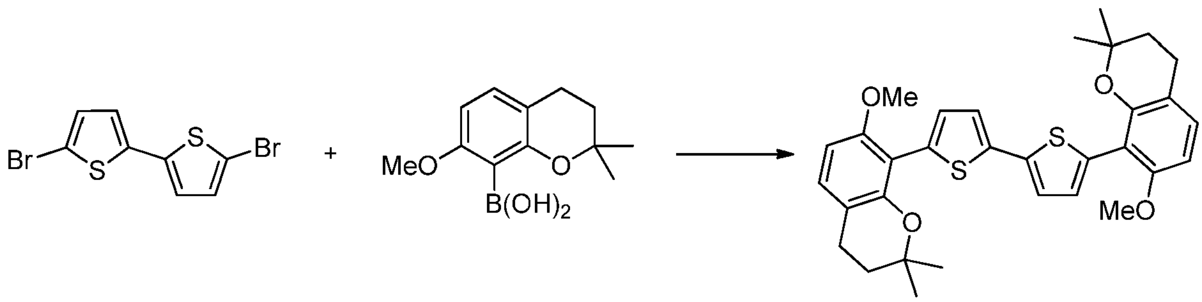

*Reaction scheme A.4*

5,5'-Dibromo-2,2'-bithiophene (1.12 g, 3.5 mmol), (7-methoxy-2,2-dimethylchroman-8-yl)boronic acid (1.72 g, 7.3 mmol, 2.1 equiv.), potassium carbonate (2.88 g, 20.8 mmol, 6 equiv.) and tetrakis(triphenylphosphine)palladium(0) (0.4 g, 0.3 mmol, 10 mol%) were added to degassed PhMe (25 mL) and EtOH (25 mL), the reaction mixture was stirred at reflux under $N_2$ for 28 h. The reaction was allowed to cool to r.t., poured onto water (50 mL) and extracted with DCM (3 × 20 mL). The combined organic portions were dried ($Na_2SO_4$) and the solvent removed. The product was purified by flash column chromatography [5 % EtOAc in hexane] to afford 5,5'-bis(7-methoxy-2,2-dimethylchroman-8-yl)-2,2'-bithiophene as a pale yellow powder which was crystallised from pentane (0.26 g, 14%). m.p. 206 - 208 °C. $v_{max}$ 2973, 2923, 2829, 1485, 1464, 1453, 1420, 1158, 1122, 1105, 794 $cm^{-1}$. $\delta_H$ (400 MHz, $CDCl_3$) 1.40 [12H, s, 2 × C(C*H*$_3$)$_2$], 1.82 [4H, t, *J* = 6.8 Hz,2 × C*H*$_2$C(CH$_3$)$_2$], 2.79 [4H, t, *J* = 6.8 Hz, 2 × C*H*$_2$CH$_2$C(CH$_3$)$_2$], 3.85 (6H, s, 2 × OC*H*$_3$), 6.53 (2H, d, *J* = 8.4 Hz, 2 × 6-*H*), 6.95 (2H, d, *J* = 8.4 Hz, 2 × *5-H),* 7.16 (2H, d, *J* = 3.7 Hz, 4-*H*, 4'-*H*) 7.57 (2H, d, *J* = 3.7 Hz, 3-*H,* 3'-*H*). $\delta_C$ (100 MHz, $CDCl_3$) 22.4 (2 × $CH_2$), 26.9 (4 × $CH_3$), 32.7 (2 × $CH_2$), 56.0 (2 × $CH_3$), 75.3 (2 × C), 103.4 (2 × CH), 114.0 (2 × C), 122.1 (2 × C, 2 × CH), 128.1 (2 × CH), 129.5 (2 × C, 2 × CH), 137.2 (2 × C), 151.5 (2 × C), 155.8 (2 × C). HRMS (Jetstream) found $[M+H]^+$ = 547.1972 $C_{32}H_{35}O_4S_2$ requires $[M+H]^+$ = 547.1973.

**B. Synthesis of (7-methoxy-2,2-dimethyl-2*H*-chromen-8-yl)boronic acid**

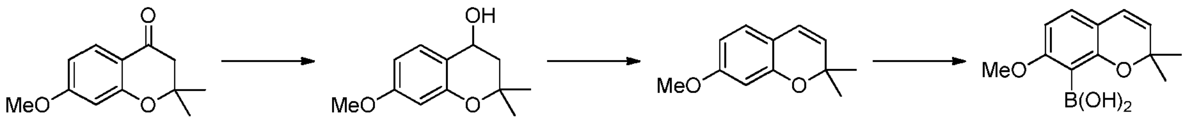

*Reaction scheme B*

7-Methoxy-2,2-dimethylchroman-4-one (1.99 g, 9.6 mmol) and $NaBH_4$ (0.44 g, 11.6 mmol, 1.2 equiv.) were stirred at reflux in EtOH (35 mL, anhyd.) for 2 hours under $N_2$. The reaction was allowed to cool to r.t. and poured onto 150 mL ice-water containing salt and stirred. The product was extracted with EtOAc (3 × 75 mL). The combined organic portions were dried ($Na_2SO_4$) and the solvent removed under reduced pressure to afford 7-methoxy-2,2-dimethylchroman-4-ol as a colourless oil (1.97 g, 98 %). $\delta_H$ (400 MHz, $CDCl_3$) 1.32 [3H, s, $C(CH_3)_a(CH_3)_b$], 1.44 [3H, s, $C(CH_3)_a(CH_3)_b$], 1.65 (1H, d, *J* = 7.5 Hz, O*H*), 1.85 (1H, dd, *J* = 8.2, 13.5 Hz, $CH_aH_b$), 2.15 (1H, dd, *J* = 6.0, 13.5 Hz, $CH_aH_b$), 3.76 (3H, s, $OCH_3$), 4.78-4.83 (1H, m, CHOH), 6.34 (1H, d, *J* = 2.5 Hz, 8-*H*), 6.52 (1H, dd, *J* = 2.5, 8.5 Hz, 6-*H*), 7.33 (1H, d, *J* = 8.5 Hz, 5-*H*). $\delta_C$ (100 MHz, $CDCl_3$) 26.1 ($CH_3$), 28.7 ($CH_3$), 42.8 ($CH_2$), 55.3 ($CH_3$), 63.4 (CH), 75.6 (C), 101.4 (CH), 107.7 (CH), 116.7 (C), 128.6 (CH), 154.3 (C), 160.6 (C). HRMS (ESI-TOF) found $[M+Na]^+$ = 231.0983 $C_{12}H_{16}NaO_3$ requires $[M+Na]^+$ = 231.0992.

To 7-methoxy-2,2-dimethylchroman-4-ol (2.24 g, 10.8 mmol) in dry THF (50 mL) was added *p*-TsOH (0.03 g, 0.2 mmol, 2 mol%) and the reaction was stirred at reflux for 2 hours. The reaction was allowed to cool and 10 % NaOH solution was added (25 mL). The product was extracted with DCM (2 × 50 mL). The combined organic portions were washed with 10 % NaOH (3 × 25 mL), brine and water and dried ($Na_2SO_4$). The solvent was removed under reduced pressure to afford 7-methoxy-2,2-dimethyl-2H-chromene as an orange oil (1.84 g, 90 %). $\nu_{max}$ 2973, 2835, 1613, 1501, 1156, 1128 $cm^{-1}$. $\delta_H$ (400 MHz, $CDCl_3$) 1.42 [6H, s, $C(CH_3)_2$], 3.77 (3H, s, $OCH_3$), 5.47 [1H, d, *J* = 9.8 Hz, $CHCHC(CH_3)_2$], 6.28 [1H, d, *J* = 9.8 Hz, $CHCHC(CH_3)_2$], 6.37-6.42 (2H, m, 6-*H*, *8-H)*, 6.88 (1H, d, *J* = 8.2 Hz, 5-*H*). $\delta_C$ (100 MHz, $CDCl_3$) 28.0 (2 × $CH_3$), 55.3 ($CH_3$), 76.4 (C), 102.0 (CH), 106.6 (CH), 114.6 (C), 121.9 (CH), 126.9 (CH), 127.9 (CH), 154.2 (C), 160.6 (C). HRMS (ESI-TOF) found $[M+H]^+$ = 191.1067 $C_{12}H_{15}O_2$ requires $[M+H]^+$ = 191.1067.

To a solution of 7-methoxy-2,2-dimethyl-2H-chromene (1.91 g, 10.0 mmol) in anhyd. THF (35 mL) at -20 °C under $N_2$ was added n-BuLi [2.5 M in hexanes, 4.9 mL, 12.2 mmol, 1.2 equiv.] dropwise over 5 min followed by the addition of TMEDA (1.84 mL, 12.2 mmol, 1.2 equiv.) in one portion. The reaction mixture was stirred at -20 °C for 30 min and allowed to warm to r.t., the reaction was stirred for a further 3.5 h. The reaction mixture was cooled to -78 °C and $B(OMe)_3$ (2.2 mL, 20.0 mmol, 2 equiv.) was added. The mixture was allowed to warm to r.t. and stirred for 16 h. The reaction was quenched with 2M HCl (30 mL) at 0 °C and stirred for 30 min. The product was extracted with EtOAc and washed with water and brine. The organic layer was dried ($Na_2SO_4$) and the solvent was removed under reduced pressure. The residue was triturated with hexane and the solid isolated by vacuum filtration to afford (7-methoxy-2,2-dimethyl-2*H*-chromen-8-yl)boronic acid as an orange solid (1.20 g, 51 %). m.p. 91-94 °C. $\nu_{max}$ 3508, 2976, 1595, 1575, 1318, 1081, 735 $cm^{-1}$. $\delta_H$ (400 MHz, $CDCl_3$) 1.49 [6H, s, $C(CH_3)_2$], 3.90 (3H, s, $OCH_3$), 5.57 [1H, d, *J* = 9.8 Hz, $CHCHC(CH_3)_2$], 6.30 [1H, d, *J* = 9.8 Hz, $CHCHC(CH_3)_2$], 6.50 (1H, d, *J* = 8.4 Hz, *6-H)*, 7.05 (1H, d, *J* = 8.4 Hz, *5-H)*, 7.26** [2H, s, $B(OH)_2$]. $\delta_C$ (100 MHz, $CDCl_3$) 28.0 (2 × $CH_3$), 55.9 ($CH_3$), 78.2 (C), 103.6 (CH), 106.8 (C)*, 115.6 (C), 121.9 (CH), 128.3 (CH), 130.0 (CH), 159.0 (C), 164.9 (C). HRMS (ESI-TOF) found $[M+NH_4]^+$ = 251.1434 $C_{12}H_{19}BNO_4$ requires $[M+NH_4]^+$ = 251.1438. (**overlaps with residual $CHCl_3$, *by HMBC)

**B.1. Synthesis of 2,5-Bis(7-methoxy-2,2-dimethyl-2*H*-chromen-8-yl)thiophene - Compound 3**

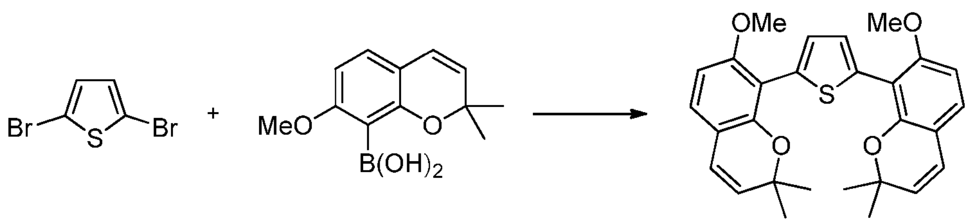

*Reaction scheme B.1*

2,5-Dibromothiophene (0.22 mL, 1.9 mmol), (7-methoxy-2,2-dimethyl-2H-chromen-8-yl)boronic acid (1.14 g, 4.9 mmol, 2.5 equiv.), potassium carbonate (1.61 g, 11.7 mmol, 6 equiv.) and tetrakis(triphenylphosphine)palladium(0) (0.22 g, 0.2 mmol, 10 mol%) were added to degassed PhMe (20 mL) and EtOH (20 mL), the reaction mixture was stirred at reflux under $N_2$ for 4 h. The reaction was allowed to cool to r.t., poured onto water (80 mL) and extracted with DCM (3 × 20 mL). The combined organic portions were dried ($Na_2SO_4$) and the solvent removed. The product was purified by flash column chromatography [10 % EtOAc in hexane] to afford 2,5-bis(7-methoxy-2,2-dimethyl-2*H*-chromen-8-yl)thiophene as a yellow oil (0.91 g, 100 %). $\nu_{max}$ 2971, 2935, 2835, 1594, 1121, 1099, 799 $cm^{-1}$. $\delta_H$ (400 MHz, $CDCl_3$) 1.50 [12H, s, 2 × $C(CH_3)_2$], 3.87 (6H, s, 2 × $OCH_3$), 5.55 [2H, d, *J* = 9.7 Hz, 2 × $CHCHC(CH_3)_2$], 6.33 [2H, d, *J* = 9.7 Hz, 2 × $CHCHC(CH_3)_2$], 6.52 (2H, d, *J* = 8.3 Hz, 2 × 6-*H*), 6.88 (2H, d, *J* = 8.3 Hz, 2 × 5-*H*), 7.64 (2H, s, 3-*H,* 4-*H*). $\delta_C$ (100 MHz, $CDCl_3$) 27.9 (4 × $CH_3$), 55.8 (2 × $CH_3$), 76.9 (2 × C), 103.5 (2 × CH), 112.9 (2 × C), 115.5 (2 × C), 122.4 (2 × CH), 125.0 (2 × CH), 128.0 (2 × CH), 128.4 (2 × CH), 133.5 (2 × C), 150.8 (2 × C), 157.5 (2 × C). HRMS (JS) found $[M+H]^+$ = 461.1777 $C_{28}H_{28}O_4S$ requires $[M+H]^+$ = 461.1777.

**B.2. Synthesis of 2,5-Bis(7-methoxyspiro[chromane-2,1'-cyclohexan]-8-yl)thiophene - Compound 2**

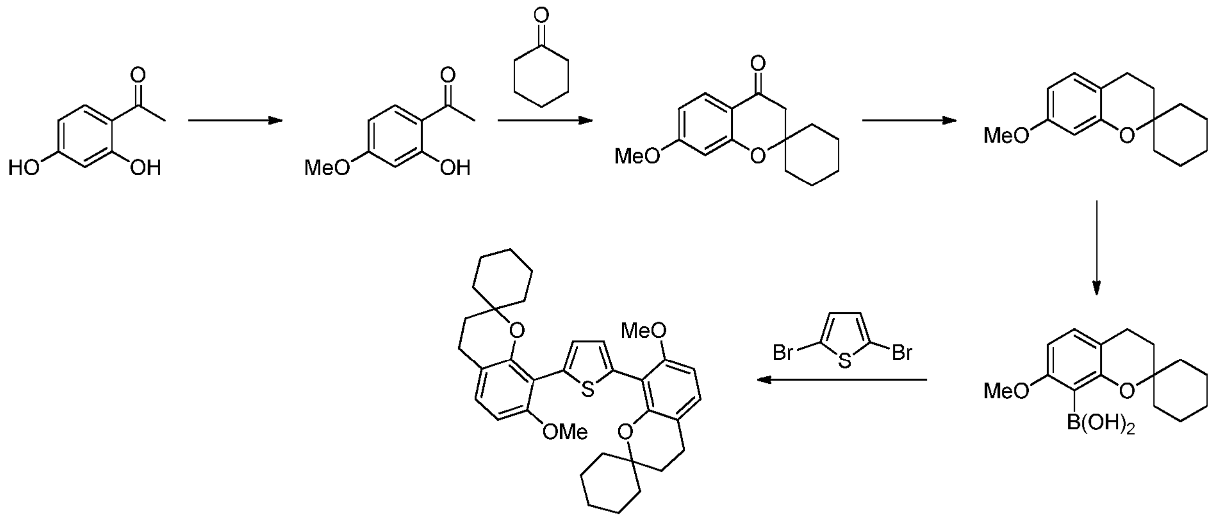

*Reaction scheme B.2*

To a solution of 2,4-dihydroxyacetophenone (5.00 g, 32.9 mmol) in acetone (30 mL) was added $K_2CO_3$ (2.27 g, 16.4 mmol, 0.5 equiv.) followed by iodomethane (2.25 mL, 36.1 mmol, 1.1 equiv.) at r.t. The reaction was then stirred at reflux for 8 h. The reaction mixture was allowed to cool and filtered. The filtrate was concentrated *in vacuo* and the product purified by flash column chromatography [10 % EtOAc in hexane] to afford 2-hydroxy-4-methoxyacetophenone as a colourless solid (3.98 g, 73 %). m.p. 48 - 49 °C (lit. m.p. 52-53 °C [M. V. B. Reddy et al., Bioorganic and Medicinal Chemistry, 2008, 16, 7358 - 7370]). $\nu_{max}$ 2974, 2846, 1616, 1574, 1365, 1247, 1206, 808, 570 $cm^{-1}$. $\delta_H$ (400 MHz, $CDCl_3$) 2.56 (3H, s, $COCH_3$), 3.84 (3H, s, $OCH_3$), 6.42-6.46 (2H, m, 3-*H,* 5-*H*), 7.64 (1H, d, *J* = 8.7 Hz, 6-*H*), 12.77 (1H, s, O*H*). $\delta_C$ (100 MHz, $CDCl_3$) 26.3 ($CH_3$), 55.6 ($CH_3$), 100.8 (CH), 107.7 (CH), 113.9 (C), 132.3 (CH), 165.3 (C), 166.1 (C), 202.6 (C). HRMS (ESI-TOF) found $[M+H]^+$ = 167.0702 $C_9H_{11}O_3$ requires $[M+H]^+$ = 167.0703.

To a solution of 2-hydroxy-4-methoxyacetophenone (3.74 g, 22.5 mmol) in EtOH (40 mL) was added cyclohexanone (23.3 mL, 225 mmol, 10 equiv.) and pyrrolidine (5.6 mL, 67.5 mmol, 3 equiv.). The reaction was stirred at reflux for 6 hours and the solvent was removed under reduced pressure. The residue was diluted with EtOAc (50 mL), washed with saturated $NH_4Cl$ (100 mL) and brine (100 mL). The product was extracted from the aqueous layer with EtOAc (2 × 75 mL) and the combined organic layers dried ($Na_2SO_4$) and the solvent removed under reduced pressure. The product was purified by flash column chromatography [10% EtOAc in hexane] to afford 7-methoxyspiro[chromane-2,1'-cyclohexan]-4-one as a yellow oil which slowly crystallised (4.84 g, 87 %). m.p. 69 - 70 °C (lit. m.p. 68 °C [Y. Jacquot et al., Medicinal Chemistry Research, 2013, 22, 681 - 691]). $\nu_{max}$ 3656, 2980, 2928, 2889, 1670, 1601, 1123 $cm^{-1}$. $\delta_H$ (400 MHz, $CDCl_3$) 1.28-1.99 (10H, m, Cy-H), 2.64 (2H, s, $COCH_2$), 3.83 (3H, s, $OCH_3$), 6.41 (1H, d, J = 2.3 Hz, 8-H), 6.52 (1H, dd, J = 2.3, 8.8 Hz, 6-H), 7.78 (1H, d, J = 8.8 Hz, 5-H). $\delta_C$ (100 MHz, $CDCl_3$) 21.5 (2 × $CH_2$), 25.2 ($CH_2$), 34.9 (2 × $CH_2$), 47.9 ($COCH_2$), 55.6 ($CH_3$), 80.4 (C), 101.2 (CH), 109.2 (CH), 114.7 (C), 128.2 (CH), 161.6 (C), 166.2 (C), 191.2 (C). HRMS (ESI) found $[M+H]^+$ = 247.1326 $C_{15}H_{18}O_3$ requires $[M+H]^+$ = 247.1329.

7-Methoxyspiro[chromane-2,1'-cyclohexan]-4-one (4.00 g, 16.2 mmol) was added to Zn dust (26.55 g, 406 mmol, 25 equiv.) in AcOH (100 mL) and stirred at reflux overnight. The reaction was allowed to cool to r.t. and filtered through Celite. The reaction mixture was diluted with EtOAc (100 mL) and PhMe (100 mL). The solvent was removed under reduced pressure. The residue was dissolved in DCM and the solvent removed to afford 7-methoxyspiro[chromane-2,1'-cyclohexane as an orange oil (3.79 g, 100 %). $v_{max}$ 2928, 2855, 1618, 1582, 1503, 1441, 1156, 1145, 985 $cm^{-1}$. $\delta_H$ (400 MHz, $CDCl_3$) 1.31-1.79 (12H, m, Cy-H, C*H*$_2$CCy), 2.68 (2H, t, *J* = 6.8 Hz, C*H*$_2$CH$_2$CCy), 3.76 (3H, s, OC*H*$_3$), 6.40 (1H, d, *J* = 2.6 Hz, 8-*H*), 6.42 (2H, dd, *J* = 2.6, 8.3 Hz, *6-H),* 6.93 (1H, d, *J* = 8.3 Hz, 5-*H*). $\delta_C$ (100 MHz, $CDCl_3$) 20.9 ($CH_2$), 21.8 (2 × $CH_2$), 26.0 ($CH_2$), 31.6 ($CH_2$), 35.1 (2 × $CH_2$), 55.3 ($CH_3$), 74.9 (C), 101.8 (CH), 106.7 (CH), 113.7 (C), 129.9 (CH), 154.6 (C), 159.0 (C). HRMS (ESI-TOF) found $[M+H]^+$ = 233.1538 $C_{15}H_{21}O_2$ requires $[M+H]^+$ = 233.1536.

To a solution of 7-methoxyspiro[chromane-2,1'-cyclohexane (3.50 g, 15.1 mmol) in anhydrous THF (50 mL) at -20 °C under $N_2$ was added *n*-BuLi [2.5 M in hexanes, 7.4 mL, 18.4 mmol, 1.2 equiv.] dropwise over 5 min followed by the addition of TMEDA (2.8 mL, 18.4 mmol, 1.2 equiv.) in one portion. The reaction mixture was stirred at -20 °C for 30 min and allowed to warm to r.t., the reaction was stirred for a further 3.5 h. The reaction mixture was cooled to -78 °C and $B(OMe)_3$ (3.4 mL, 30.1 mmol, 2 equiv.) was added. The mixture was allowed to warm to r.t. and stirred for 16 h. The reaction was quenched with 2M HCl (30 mL) at 0 °C and stirred for 30 min. The product was extracted with EtOAc and washed with water and brine. The organic layer was dried ($Na_2SO_4$) and the solvent was removed under reduced pressure. The residue was triturated with hexane and the solid isolated by vacuum filtration to afford 7-(methoxyspiro[chromane-2,1'-cyclohexan]-8-yl)boronic acid as a pale orange solid (2.10 g, 51 %). m.p. 86 - 87 °C. $v_{max}$ 3459, 3217, 2935, 2857, 1309, 1295, 1082, 797 $cm^{-1}$. $\delta_H$ (400 MHz, $CDCl_3$) 1.29-1.73 (8H, m, Cy-H), 1.80-1.86 (4H, m, Cy-H, C*H*$_2$Cy), 2.73 (2H, t, *J* = 6.7 Hz, C*H*$_2$CH$_2$Cy), 3.87 (3H, s, $CH_3$), 6.51 (1H, d, *J* = 8.4 Hz, 6-*H*), 7.11 (1H, d, *J* = 8.4 Hz, *5-H),* 7.48 [2H, s, B(O*H*)$_2$]. $\delta_C$ (100 MHz, $CDCl_3$) 21.2 ($CH_2$), 21.9 (2 × $CH_2$), 25.5 ($CH_2$), 31.3 ($CH_2$), 34.9 (2 × $CH_2$), 55.9 ($CH_3$), 77.4** (C), 103.4 (CH), 106.1 (C), 115.0 (C), 133.1 (CH), 159.4 (C), 163.9 (C). HRMS (ESI-TOF) found $[M+Na]^+$ = 298.1463 $C_{15}H_{21}BNaO_4$ requires $[M+Na]^+$ = 298.1461. (**Overlaps with residual $CHCl_3$)

2,5-Dibromothiophene (0.33 mL, 2.9 mmol), 7-(methoxyspiro[chromane-2,1'-cyclohexan]-8-yl)boronic acid (2.00 g, 7.2 mmol, 2.5 equiv.), potassium carbonate (2.40 g, 17.4 mmol, 6 equiv.) and tetrakis(triphenylphosphine) palladium(0) (0.33 g, 0.3 mmol, 10 mol%) were added to degassed PhMe (20 mL) and EtOH (20 mL), the reaction mixture was stirred at reflux under $N_2$ for 3 h. The reaction was allowed to cool to r.t., poured onto water (80 mL) and extracted with DCM (3 × 30 mL). The combined organic portions were dried ($Na_2SO_4$) and the solvent removed. The product was purified by flash column chromatography [10 % EtOAc in hexane → 50 % DCM in hexane] to afford 2,5-bis(7-methoxyspiro [chromane-2,1'-cyclohexan]-8-yl)thiophene as a yellow solid (1.36 g, 86 %). m.p. 167-169 °C. $v_{max}$ 2920, 2881, 2836, 1457, 1418, 1115, 1097, 787 $cm^{-1}$. $\delta_H$ (400 MHz, $CDCl_3$) 1.21-1.43 (10H, m, Cy-*H*), 1.52-1.59 92H, m, Cy-*H*), 1.66-1.70 (4H, m, Cy-*H*), 1.78 (4H, t, *J* = 6.8 Hz, 2 × CH$_2$C*H*$_2$Cy), 1.82-1.87 (4H, m, Cy-H), 2.76 (4H, t, *J* = 6.8 Hz, 2 × C*H*$_2$CH$_2$Cy), 3.80 (6H, s, 2 × OC*H*$_3$), 6.52 (2H, d, *J* = 8.4 Hz, 2 × 6-*H*), 6.94 (2H, d, *J* = 8.4 Hz, 2 × 5-*H*), 7.46 (2H, s, 3-*H,* 4-*H*). $\delta_C$ (100 MHz, $CDCl_3$) 21.6 (2 × $CH_2$), 21.8 (4 × $CH_2$), 26.1 (2 × $CH_2$), 31.8 (2 × $CH_2$), 35.3 (4 × $CH_2$), 56.0 (2 × $CH_3$), 75.7 (2 × C), 103.4 (2 × CH), 113.1 (2 × C), 114.6 (2 × C), 127.8 (2 × CH), 128.1 (2 × CH), 133.7 (2 × C), 151.8 (2 × C), 156.2 (2 × C). HRMS (JS) found $[M+H]^+$ = 545.2720 $C_{34}H_{40}O_4S$ requires $[M+H]^+$ = 545.2720.

**B.3. Synthesis of 2,5-Bis(3-methoxydibenzo[*b,d*]furan-4-yl)thiophene** - **Compound 12**

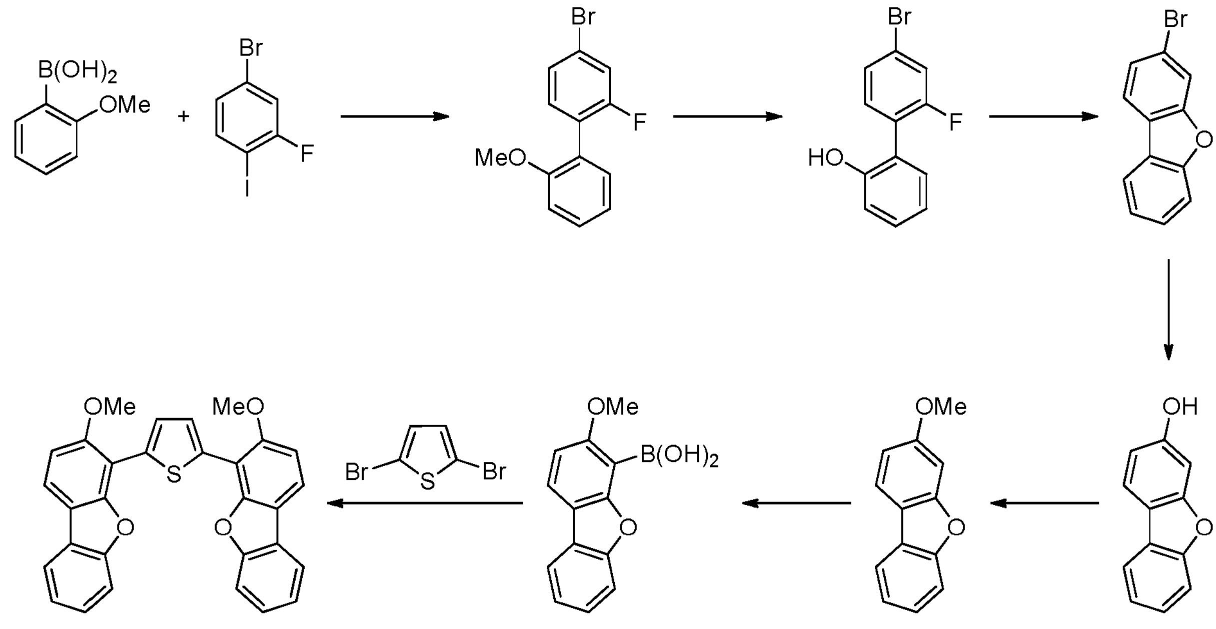

*Reaction scheme B.3*

3-Fluoro-4-iodobromobenzene (12.04 g, 40 mmol), 2-methoxybenzeneboronic acid (7.29 g, 48 mmol, 1.2 equiv.), potassium carbonate (33.17 g, 240 mmol, 6 equiv.) and tetrakis(triphenylphosphine)palladium(0) (4.62 g, 4 mmol, 10 mol%) were added to degassed PhMe (150 mL) and EtOH (150 mL), the reaction mixture was stirred at reflux under $N_2$ for 4 h. The reaction was allowed to cool to r.t., poured onto water (400 mL) and extracted with DCM (3 × 100 mL). The combined organic portions were dried ($Na_2SO_4$) and the solvent removed. The product was purified by flash column chromatography [10% EtOAc in hexane] to afford 4-bromo-2-fluoro-2'-methoxybiphenyl as a colourless oil (9.59 g, 85 %). $\nu_{max}$ 3069, 2954, 2835, 1477, 1244, 746 $cm^{-1}$. $\delta_H$ (400 MHz, $CDCl_3$) 3.84 (3H, s, OC*H*$_3$), 7.00 (1H, d, *J* = 8.3 Hz, 3-*H*), 7.03 (1H, dt, *J* = 0.9, 7.5 Hz, 5-*H*), 7.22 - 7.26 (1H, m, 6-*H*), 7.30 - 7.41 (4H, m, 4-*H*, 6'-*H*, 3'-*H*, 5'-*H*). $\delta_C$ (100 MHz, $CDCl_3$) 55.7 ($CH_3$), 111.1 (CH), 119.1 (d, $J_{C-F}$ = 27.0 Hz, CH), 120.6 (CH), 121.5 (d, $J_{C-F}$ = 9.4 Hz, C), 123.9 (C), 125.4 (d, $J_{C-F}$ = 16.2 Hz, C) 127.1 (d, $J_{C-F}$ = 3.7 Hz, CH), 129.8 (CH), 131.1 (d, $J_{C-F}$ = 3.0 Hz, CH), 132.9 (d, $J_{C-F}$ = 5.6 Hz, CH), 156.8 (C), 160.0 (d, $J_{C-F}$ = 251 Hz, C).

To a solution of 4-bromo-2-fluoro-2'-methoxybiphenyl (8.36 g, 29.7 mmol) in anhyd. DCM (50 mL) was added a solution of $BBr_3$ (5.7 mL, 59.5 mmol, 2 equiv.) in anhyd. DCM (35 mL) dropwise at 0 °C over 20 min. The reaction was stirred for 3 h at r.t. The reaction was cooled and quenched with ice-water (100 mL) and the product extracted with DCM (2 × 50 mL). The combined organic portions were dried ($Na_2SO_4$) and the solvent removed under reduced pressure. The residue was purified by flash column chromatography [10 % EtOAc in hexane] to afford 4'-bromo-2'-fluoro-[1,1'-biphenyl]-2-ol as an oil which crystallised (6.96 g, 88 %). m.p. 80 - 81 °C. $\nu_{max}$ 3243, 1470, 1213, 1195, 817, 754 $cm^{-1}$. $\delta_H$ (400 MHz, $CDCl_3$) 4.87 (1H, s, O*H*), 6.97 (1H, d, *J* = 8.1 Hz, 3-*H*), 7.00-7.04 (1H, m, 5-*H*), 7.22 (1H, d, *J* = 7.4 Hz, 6-*H*), 7.28-7.33 (2H, m, 4-*H*, 6'-*H*), 7.38-7.42 (2H, m, 3'-*H*, 5'-*H*). $\delta_C$ (100 MHz, $CDCl_3$) 116.2 (CH), 119.8 (d, $J_{C-F}$ = 25.5 Hz, CH), 121.0 (CH), 121.5 (C), 122.3 (d, $J_{C-F}$ = 9.3 Hz, C), 123.9 (d, $J_{C-F}$ = 15.9 Hz, C), 127.9 (d, $J_{C-F}$ = 3.6 Hz, CH), 130.1 (CH), 131.1 (d, $J_{C-F}$ = 1.2 Hz, CH), 132.9 (d, $J_{C-F}$ = 4.0 Hz, CH), 152.7 (C), 159.7 (d, $J_{C-F}$ = 250 Hz, C). HRMS (ESI) found $[M+H]^+$ = 266.9810 $C_{12}H_8BrFO$ requires $[M+H]^+$ = 266.9815.

To 4'-bromo-2'-fluoro-[1,1'-biphenyl]-2-ol (6.21 g, 23.2 mmol) in NMP (60 mL) was added $K_2CO_3$ (2.76 g, 20 mmol, 2 equiv.) and the reaction was stirred under $N_2$ at 180 °C for 3 h. The reaction was allowed to cool to r.t., poured onto water (500 mL) and stirred for 1 h. The colourless precipitate was isolated by vacuum filtration to afford 3-bromodibenzo [b,d]furan (5.20 g, 91 %). m.p. 119 - 121 °C (lit. m.p. 118 - 119 °C [Y Wei et al., Organic Letters, 2011, 13, 5504 - 5507]). $\nu_{max}$ 3062, 1588, 1455, 1412, 812, 742, 718, 687 $cm^{-1}$. $\delta_H$ (400 MHz, $CDCl_3$) 7.34-7.38 (1H, m, 8-*H*), 7.45-7.51 (2H, m, 2-*H*, 7-*H*), 7.57 (1H, d, *J* = 8.2 Hz, 6-*H*), 7.75 (1H, d, *J* = 1.6 Hz, 4-*H*), 7.81 (1H, d, *J* = 8.2 Hz, 1-*H*), 7.92-7.94 (1H, m, 9-*H*). $\delta_C$ (100 MHz, $CDCl_3$) 111.8 (CH), 115.2 (CH), 120.2 (C), 120.7 (CH), 121.6 (CH), 123.1 (CH), 123.4 (C), 123.5 (C), 126.1 (CH), 127.6 (CH), 156.3 (C), 156.5 (C). HRMS (APCI) found $[M]^+$ = 245.9677 $C_{12}H_7BrO$ requires $[M]^+$ = 245.9675.

To 3-bromodibenzo[*b*,*d*]furan (1.48 g, 6 mmol) in anhyd. THF (50 mL) was added *n*-BuLi (2.5 M in hexanes, 2.6 mL, 6.6 mmol, 1.1 equiv.) followed by TMEDA (1 mL, 6.6 mmol, 1.1 equiv.) at -78 °C under $N_2$, the reaction was stirred at -78 °C for 1.5 h. $B(OMe)_3$ (3.3 mL, 29.9 mmol, 5 equiv.) was added dropwise and the reaction stirred at -78 °C for a further 3 h. The reaction was allowed to warm to r.t. and stirred for a further 30 min. The reaction was cooled to 0 °C and $H_2O_2$ (30 %, 6.8 mL) and sat. aqueous $Na_2CO_3$ (6.8 mL) were added slowly and the reaction was stirred for 1 h at r.t. The reaction was

cooled to 0 °C before the addition of sat. aqueous $Na_2S_2O_3$ (50 mL) and sat. aqueous $NH_4Cl$ (80 mL) and the product extracted with EtOAc (3 × 75 mL) and the combined organic portions were washed with water (2 × 100 mL) and brine (100 mL), dried ($Na_2SO_4$) and the solvent removed under reduced pressure. The residue was purified by flash column chromatography [10 % EtOAc in hexane] to afford dibenzo[*b,d*]furan-3-ol as a colourless solid (0.70 g, 63 %). m.p. 145 - 147 °C (lit. m.p. 141 - 141.5 °C [H. Erdtman et al., Acta Chemica Scandinavica, 1961, 15, 1761-1764]). $v_{max}$ 3253, 3044, 1596, 1437, 1270, 846, 761, 740 $cm^{-1}$. $\delta_H$ (400 MHz, $CDCl_3$) 6.86 (1H, dd, *J* = 2.2, 8.3 Hz, 2-*H*), 7.05 (1H, d, *J* = 2.2 Hz, 4-*H*), 7.31 (1H, m, 7-*H*), 7.38 (1H, m, 8-*H*), 7.52 (1H, d, *J* = 8.1 Hz, 6-*H*), 7.78 (1H, d, 1-*H*), 7.85 (1H, m, 9-*H*). $\delta_C$ (100 MHz, $CDCl_3$) 98.8 (CH), 111.3 (CH), 111.4 (CH), 117.7 (C), 119.8 (CH), 121.2 (CH), 122.8 (CH), 124.3 (C), 125.9 (CH), 155.6 (C), 156.3 (C), 157.4 (C). HRMS (APCI) found $[M]^+$ = 184.0520 $C_{12}H_8O_2$ requires $[M]^+$ = 184.0519.

To dibenzo[*b,d*]furan-3-ol (1.66 g, 9 mmol) in 2-butanone (50 mL) was added iodomethane (4.4 mL, 72 mmol, 8 equiv.) followed by $K_2CO_3$ (3.74 g, 27 mmol, 3 equiv.) at room temperature under $N_2$ before stirring at reflux for 72 h. The reaction was allowed to cool to r.t. and quenched with water (100 mL). The product was extracted with EtOAc (3 × 50 mL), and the combined organic portions were dried ($Na_2SO_4$) and the solvent removed under reduced pressure. The product was purified by flash column chromatography [10 % EtOAc in hexane] to afford 3-methoxydibenzo[*b*,*d*]furan as a colourless solid (1.26 g, 71 %). (0.44 g, 63 %). m.p. 98 - 100 °C. $v_{max}$ 3044, 2845, 1633, 1595, 1455, 1423, 1275, 032, 815, 761, 748 $cm^{-1}$. $\delta_H$ (400 MHz, $CDCl_3$) 3.91 (3H, s, OC*H*$_3$), 6.95 (1H, dd, *J* = 2.2, 8.5 Hz, 2-*H*), 7.10 (1H, d, *J* = 2.2 Hz, 4-*H*), 7.29-7.33 (1H, m, 7-*H*), 7.36-7.40 (1H, m, 8-*H*), 7.53 (1H, d, *J* = 8.1 Hz, 6-*H*), 7.82 (1H, d, *J* = 8.5 Hz, 1-*H*), 7.86 (1H, m, 9-*H*). $\delta_C$ (100 MHz, $CDCl_3$) 55.8 ($CH_3$), 96.5 (CH), 111.0 (CH), 111.4 (CH), 117.3 (C), 119.8 (CH), 121.0 (CH), 122.7 (CH), 124.4 (C), 125.7 (CH), 156.3 (C), 157.5 (C), 159.9 (C). HRMS (APCI) found $[M]^+$ = 198.0676 $C_{13}H_{10}O_2$ requires $[M]^+$ = 198.0675.

To a stirred solution of TMEDA (2.4 mL, 15.8 mmol, 2 equiv.) in anhyd. THF (75 mL) at -20 °C under $N_2$ was added *n*-BuLi (6.3 mL, 15.8 mmol, 2 equiv.) slowly and the mixture stirred for 5 minutes before the dropwise addition of 3-methoxydibenzo[*b*,*d*]furan (1.57 g, 7.9 mmol, 1 equiv.). in THF (25 mL). The reaction was stirred for 30 min at -20 °C then 3.5 h at r.t. The reaction was cooled to -78 °C and $B(OMe)_3$ (1.8 mL, 15.8 mmol, 2 equiv.) was added, the reaction was allowed to warm to r.t. overnight. The reaction was cooled to -20 °C and quenched with dil. HCl (2M) and stirred for 30 min at r.t. The product was extracted with EtOAc (3 × 50 mL) and the combined organic extracts were washed with water (100 mL) and brine (100 mL), dried ($Na_2SO_4$) and the solvent removed under reduced pressure to afford (3-methoxydibenzo[b,d]furan-4-yl)boronic acid as a colourless solid (1.60 g, 84%). m.p. 150 - 151 °C. $v_{max}$ 3285, 2933, 2836, 1599, 1457, 1341, 1081, 778 $cm^{-1}$. $\delta_H$ (400 MHz, $CDCl_3$) 4.04 (3H, s, OC*H*$_3$), 6.80 [2H, s, B(O*H*)$_2$], 7.01 (1H, d, *J* = 8.5 Hz, 2-*H*), 7.34-7.38 (1H, m, 8-*H*), 7.41-7.45 (1H, m, 7-*H*), 7.57 (1H, d, *J* = 8.1 Hz, 6-*H*), 7.88 (1H, d, *J* = 7.4 Hz, 9-*H*), 7.99 (1H, d, *J* = 8.5 Hz, 1-*H*). $\delta_C$ (100 MHz, $CDCl_3$) 56.6 ($CH_3$), 102.5 [$CB(OH)_2$]*, 106.5 (CH), 111.5 (CH), 117.9 (C), 120.1 (CH), 123.4 (CH), 123.5 (C), 124.2 (CH), 126.6 (CH), 155.9 (C), 161.8 (C), 164.1 (C). HRMS (APCI) found $[M+H]^+$ = 243.0825 $C_{13}H_{12}BO_4$ requires $[M+H]^+$ = 243.0823. (*Only visible from HMBC.)

2,5-Dibromothiophene (0.28 mL, 2.5 mmol), (3-methoxydibenzo[*b*,*d*]furan-4-yl)boronic acid (1.20 g, 5 mmol, 2 equiv.), potassium carbonate (2.06 g, 14.9 mmol, 6 equiv.) and tetrakis(triphenylphosphine)palladium(0) (0.29 g, 0.2 mmol, 10 mol%) were added to degassed PhMe (30 mL) and EtOH (30 mL), the reaction mixture was stirred at reflux under $N_2$ for 4 h. The reaction was allowed to cool to r.t., poured onto water (80 mL) and extracted with DCM (3 × 30 mL). The combined organic portions were dried ($Na_2SO_4$) and the solvent removed. The product was purified by flash column chromatography [5 % EtOAc in hexane → 10 % EtOAc in hexane] to afford 2,5-bis(3-methoxydibenzo[*b*,*d*]furan-4-yl)thiophene as a yellow powder (0.23 g, 20 %). m.p. 235-237 °C (decomp. 230 °C). $v_{max}$ 1592, 1456, 1185, 1069, 803, 732 $cm^{-1}$. $\delta_H$ (400 MHz, $CDCl_3$) 4.09 (6H, s, 2 × OC*H*$_3$), 7.09-7.11 (2H, m, 2 × 8-*H*), 7.34-7.37 (2H, m, 2 × 7-*H*), 7.63-7.65 (2H, m, 2 × 6-*H*), 7.81-7.83 (2H, m, 2 × 1-*H*), 7.91-7.93 (2H, m, 2 × 9-*H*), 8.29 (2H, s, 3-th-H, 4-th-*H*). $\delta_C$ (100 MHz, $CDCl_3$) 56.8 (2 × $CH_3$), 107.7 (2 × C), 110.0 (2 × C), 111.7 (2 × C), 118.5 (2 × C), 118.8 (2 × C), 119.9 (2 × C), 122.9 (2 × C), 124.3 (2 × C), 126.1 (2 × C), 128.5 (2 × C), 133.9 (2 × C), 154.2 (2 × C), 155.7 (2 × C), 156.4 (2 × C). HRMS (JS) found $[M+H]^+$ = 477.1147 $C_{30}H_{21}O_4S$ requires $[M+H]^+$ = 477.1155.

**B.4. Synthesis of 7-Methoxy-2,2-dimethyl-8-(thiophen-2-yl)thiochromane - Compound 7 and of 2,5-bis(7-methoxy-2,2-dimethylthiochroman-8-yl)thiophene - Compound 8**

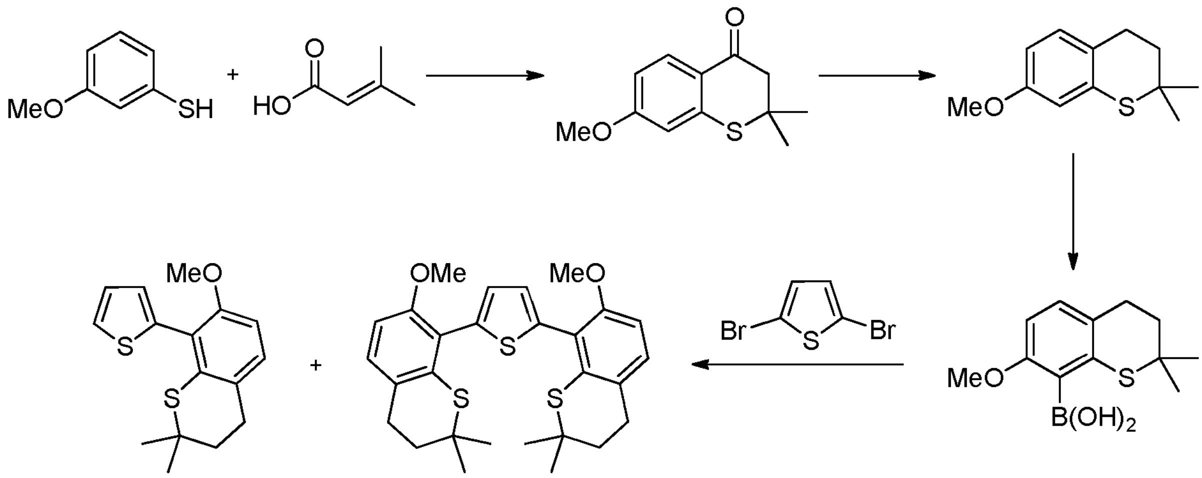

*Reaction scheme B.4*

3-Methoxythiophenol (4.4 mL, 36 mmol) and 3,3-dimethylacrylic acid (3.93 g, 39 mmol, 1.1 equiv.) were dissolved in $MeSO_3H$ (50 mL) and stirred at 70 °C for 4 hours. The reaction was allowed to cool, poured onto 200 mL ice-water and stirred for 30 min. The product was extracted with $Et_2O$ (3 × 100 mL) and the combined organic portions were washed with 5 % NaOH solution (2 × 50 mL), water (100 mL) and brine (100 mL). The organic portion was dried ($Na_2SO_4$) and the solvent removed *in vacuo* to afford a viscous yellow oil. The product was purified by FCC [5% EtOAc in hexane] to afford 7-methoxy-2,2-dimethylthiochroman-4-one as a pale yellow solid (4.59 g, 58 %). m.p. 67-69 °C (lit. m.p. 67-68 °C [F. Camps et al., Journal of Heterocyclic Chemistry, 1983, 20, 1115 - 1117]). $\nu_{max}$ 3011, 2977, 2965, 1668, 1589, 1484, 1305, 1237, 1041, 839 $cm^{-1}$. $\delta_H$ (400 MHz, $CDCl_3$) 1.46 [6H, s, C($CH_3$)$_2$], 2.83 (2H, s, $CH_2$), 3.84 (3H, s, O$CH_3$), 6.68 - 6.72 (2H, m, *6-H, 8-H),* 8.07 (1H, d, *J* = 8.5 Hz, 5-*H*). $\delta_C$ (100 MHz, $CDCl_3$) 28.7 (2 × $CH_3$), 44.9 (C), 53.7 ($CH_2$), 55.5 ($CH_3$), 110.7 (CH), 112.3 (CH), 123.4 (C), 130.9 (CH), 143.6 (C), 163.6 (C), 193.7 (C).

7-Methoxy-2,2-dimethylthiochroman-4-one (4.00 g, 18 mmol) was added to Zn dust (29.41 g, 450 mmol, 25 equiv.) in AcOH (75 mL) and stirred at reflux overnight. The reaction was allowed to cool to r.t. and filtered through Celite®. The reaction mixture was diluted with EtOAc (200 mL) and PhMe (200 mL). The solvent was removed under reduced pressure. The residue was dissolved in DCM and the solvent removed, the insoluble material was discarded. The product was purified by FCC [5 % EtOAc in hexane] to afford 7-methoxy-2,2-dimethylthiochromane as a colourless oil (2.60 g, 69 %). $\nu_{max}$ 2957, 2923, 2844, 1600, 1490, 1245, 1060, 1041, 804 $cm^{-1}$. $\delta_H$ (400 MHz, $CDCl_3$) 1.41 [6H, s, C($CH_3$)$_2$], 1.90 [2H, t, *J* = 6.5 Hz, $CH_2CH_2C(CH_3)_2$], 2.86 [2H, t, *J* = 6.5 Hz, $CH_2CH_2C(CH_3)_2$], 3.75 (3H, s, O$CH_3$), 6.56 - 6.60 (2H, m, *6-H,* 8-*H*), 7.00 (1H, d, *J* = 8.2 Hz, 5-*H*). $\delta_C$ (100 MHz, $CDCl_3$) 26.2 ($CH_2$), 30.2 (2 × $CH_3$), 37.9 ($CH_2$), 42.3 (C), 55.3 ($CH_3$), 110.7 (CH), 110.9 (CH), 124.3 (C), 130.5 (CH), 134.5 (C), 157.9 (C).

To TMEDA (2.9 mL, 19.2 mmol, 2 equiv.) in anhydrous THF (50 mL) at -20 °C under $N_2$ was added *n*-BuLi [2.5 M in hexanes, 7.7 mL, 19.2 mmol, 2 equiv.] dropwise over 5 min followed by the dropwise addition of a solution of 7-methoxy-2,2-dimethylthiochromane (2.00 g, 9.6 mmol) in THF (10 mL). The reaction mixture was stirred at -20 °C for 30 min and allowed to warm to r.t., the reaction was stirred for a further 3.5 h. The reaction mixture was cooled to -78 °C and $B(OMe)_3$ (2.1 mL, 19.2 mmol, 2 equiv.) was added. The mixture was allowed to warm to r.t. and stirred for 16 h. The reaction was quenched with 2 M HCl at 0 °C and stirred for 30 min. The product was extracted with EtOAc (3 × 50 mL) and washed with water and brine. The organic layer was dried ($Na_2SO_4$) and the solvent was removed under reduced pressure. The residue was triturated with hexane and the solid isolated by vacuum filtration to afford (7-methoxy-2,2-dimethylthiochroman-8-yl)boronic acid as an off-white powder (1.17 g, 48 %). m.p. 114-117 °C. $\nu_{max}$ 3210, 2980, 1579, 1566, 1463, 1386, 1193, 1033, 800, 779 $cm^{-1}$. $\delta_H$ (400 MHz, $CDCl_3$) 1.43 [6H, s, C($CH_3$)$_2$], 1.83 [2H, t, *J* = 6.4 Hz, $CH_2CH_2C(CH_3)_2$], 2.80 [2H, t, *J* = 6.4 Hz, $CH_2CH_2C(CH_3)_2$], 3.87 (3H, s, O$CH_3$), 6.71 (1H, d, *J* = 8.4 Hz, 6-*H*), 6.86 [2H, s, B(O*H*)$_2$], 7.17 (1H, d, *J* = 8.4 Hz, 5-*H*). $\delta_C$ (100 MHz, $CDCl_3$)* 28.9 ($CH_2$), 31.2 (2 × $CH_3$), 38.8 ($CH_2$), 45.6 (C), 56.0 ($CH_3$), 107.9 (CH), 130.5 (C), 131.6 (CH), 141.4 (C), 163.1 (C). (*$CB(OH)_2$ not visible in $^{13}C$.)

2,5-Dibromothiophene (0.21 mL, 1.9 mmol), (7-methoxy-2,2-dimethylthiochroman-8-yl)boronic acid (1.00 g, 4 mmol, 2.1 equiv.), potassium carbonate (1.57 g, 11.3 mmol, 6 equiv.) and tetrakis(triphenylphosphine)palladium(0) (0.22 g, 0.2 mmol, 10 mol%) were added to degassed PhMe (20 mL) and EtOH (20 mL), the reaction mixture was stirred at reflux under $N_2$ for 24 h. The reaction was allowed to cool to r.t., poured onto water (50 mL) and extracted with DCM (3 × 25 mL). The combined organic portions were dried ($Na_2SO_4$) and the solvent removed. The product was purified by flash column chromatography [5 % EtOAc in hexane] to afford: Fraction 1: 7-methoxy-2,2-dimethyl-8-(thiophen-2-yl)thiochromane as a colourless oil which solidified (0.17 g, 36 %). m.p. 99 - 101 °C. $\nu_{max}$ 2960, 2926, 1581, 1459, 1260, 1045, 809, 700 $cm^{-1}$.

(400 MHz, $CDCl_3$) 1.34 [6H, s, $C(CH_3)_2$], 1.86 [2H, t, $J$ = 6.4 Hz, $CH_2CH_2C(CH_3)_2$], 2.94 [2H, t, $J$ = 6.4 Hz, $CH_2CH_2C(CH_3)_2$], 3.73 (3H, s, $OCH_3$), 6.66 (1H, d, $J$ = 8.4 Hz, 6-Ch-$H$), 6.99 (1H, dd, $J$ = 1.1, 3.4 Hz, 3-Th-$H$), 7.10-7.14 (2H, m, 5-Ch-H, 4-Th-$H$), 7.44 (1H, dd, $J$ = 1.1, 5.1 Hz, 5-Th-$H$). $\delta_C$ (100 MHz, $CDCl_3$) 27.0 ($CH_2$), 30.2 (2 $\times$ $CH_3$), 37.3 ($CH_2$), 42.4 (C), 56.0 ($CH_3$), 106.8 (CH), 120.1 (C), 125.0 (C), 126.3 (CH), 126.7 (CH), 128.5 (CH), 130.4 (CH), 136.5 (C), 137.3 (C), 156.5 (C). HRMS (Jetstream) found $[M+H]^+$ = 291.0871 $C_{16}H_{19}OS_2$ requires $[M+H]^+$ = 291.0872. Fraction 2: 2,5-bis(7-methoxy-2,2-dimethylthiochroman-8-yl)thiophene as an off-white solid (0.56 g, 60 %). m.p. 162 - 164 °C. $\nu_{max}$ 2960, 2924, 1735, 1582, 1435, 1260, 1234, 1042, 797 $cm^{-1}$. $\delta_H$ (400 MHz, $CDCl_3$) 1.37 [12H, s, 2 $\times$ $C(CH_3)_2$], 1.87 [4H, t, $J$ = 6.5 Hz, 2 $\times$ $CH_2C(CH_3)_2$], 2.93 [4H, t, $J$ = 6.5 Hz, 2 $\times$ $CH_2CH_2C(CH_3)_2$], 3.75 (6H, s, 2 $\times$ $OCH_3$), 6.66 (2H, d, $J$ = 8.4 Hz, 2 $\times$ 6-$H$), 7.01 (2H, s, 3-$H$, 4-$H$), 7.08 (2H, d, $J$ = 8.4 Hz, 2 $\times$ 5-$H$). $\delta_C$ (100 MHz, $CDCl_3$) 27.2 (2 $\times$ $CH_2$), 30.3 (4 $\times$ $CH_3$), 37.3 (2 $\times$ $CH_2$), 42.3 (2 $\times$ C), 56.1 (2 $\times$ $CH_3$), 106.9 (2 $\times$ CH), 120.6 (2 $\times$ C), 125.0 (2 $\times$ C), 128.0 (2 $\times$ CH), 130.2 (2 $\times$ CH), 137.3 (2 $\times$ C), 137.4 (2 $\times$ C), 156.4 (2 $\times$ C). HRMS (Jetstream) found $[M+H]^+$ = 497.1638 $C_{28}H_{33}O_2S_3$ requires $[M+H]^+$ = 497.1637.

**B.5. Synthesis of 2-(2-isopropoxy-6-methoxyphenyl)-7-methoxy-4,4-dimethyl-4H-thieno[2,3-c]chromene** - **Compound 17**

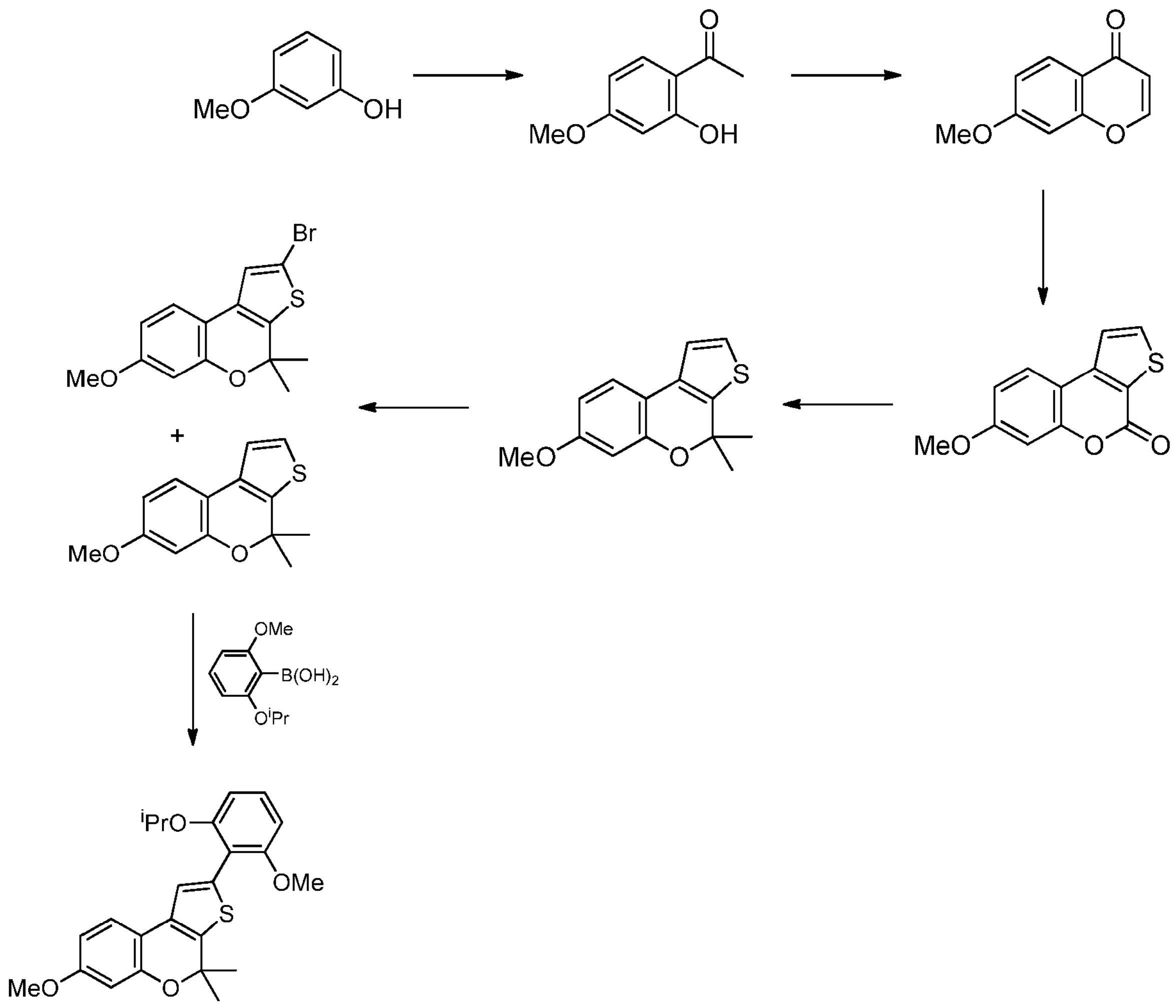

*Reaction scheme B.5*

3-Methoxyphenol (30 g, 0.24 mol), $ZnCl_2$ (22.9 g, 0.24 mol) and acetic acid (60 mL) were stirred and heated at 120 °C for 6 hours. The reaction mixture was cooled to ambient temperature and poured into water (300 mL) and extracted with EtOAc (3 $\times$ 300 mL). The organic layers were combined, dried ($Na_2SO_4$) and the solvent was removed under reduced pressure. The resulting orange residue was chromatographed on silica gel [eluent = 7:93 EtOAc:Hexane] to give 2'-hydroxy-4'-methoxyacetophenone as a colourless solid, (10.3 g, 26 %). $\delta_H$ (400 MHz, $CDCl_3$) 12.76 (s, 1 H), 7.63 (d, $J$ = 8.8

Hz, 1 H), 6.44 (dd, *J* = 8.8, 1.2 Hz, 1 H), 6.39 (d, *J* = 1.4 Hz, 1H), 3.83 (s, 3H), 2.56 (s, 3H). $\delta_C$ (101 MHz, $CDCl_3$) 202.8, 166.3, 165.4, 132.4, 114.0, 107.7, 100.9, 55.7, 26.3.

2'-Hydroxy-4'-methoxyacetophenone (10.0 g, 56.8 mmol) and DMFDMA (22.7 mL, 170.4 mmol) dissolved toluene (300 mL). The reaction mixture was then in stirred and heated at 90 °C for 16 hours. The reaction mixture was then cooled to 0 °C and $HCl_{(aq)}$ (37 %) (34 mL) was added. The reaction mixture was then stirred and heated at 60 °C for 1 hour. The reaction mixture was cooled to ambient temperature and diluted with EtOAc (500 mL). The reaction mixture was washed with water (3 × 250 mL), brine (2 × 200 mL), dried ($Na_2SO_4$) and the solvent was removed under reduced pressure. The resulting residue was chromatographed on silica gel [eluent = 3:7 EtOAc:Hexane graduated to 1:1 EtOAc:Hex] to give the 7-methoxy-4H-chromen-4-one as a yellow solid, (7.8 g, 74 %). $\delta_H$ (300 MHz, $CDCl_3$) 8.09 (d, *J* = 8.8 Hz, 1 H), 7.77 (d, *J* = 6.1 Hz, 1 H), 6.96 (dd, *J* = 8.8, 2.4 Hz, 1 H), 6.82 (d, *J* = 2.4 Hz, 1 H), 6.26 (d, *J* = 6.1 Hz, 1 H), 3.90 (s, 3 H). $\delta_C$ (75 MHz, $CDCl_3$) 177.2, 164.3, 158.6, 155.1, 127.5, 119.1, 114.9, 113.2, 100.8, 56.1.

7-Methoxy-4*H*-chromen-4-one (7.0 g, 40 mmol), ethyl mercaptoacetate (4.82 mL, 44 mmol), and DBU (11.94 mL, 80.0 mmol) were dissolved in 1,4-dioxane (200 mL). The reaction mixture was then heated and stirred at 60 °C for 12 hours under $N_2$. The reaction mixture was then cooled to ambient temperature and the reaction mixture was filtered to acquire a colourless precipitate. The precipitate was washed with 1,4-dioxane (30 mL) and dried under reduced pressure to give the 7-methoxy-4H-thieno[2,3-c]chromen-4-one as a colourless solid, (6.58g, 71 %). $\delta_H$ (300 MHz, $CDCl_3$) 7.90 (d, *J* = 5.2 Hz, 1 H), 7.73 (d, *J* = 5.2 Hz, 1 H), 7.56 (d, *J* = 5.2 Hz, 1 H), 6.98 - 6.87 (m, 2 H), 3.89 (s, 3 H). $\delta_C$ (75 MHz, $CDCl_3$) 158.1, 157.0, 147.8, 145.6, 137.4, 125.6, 123.0, 119.3, 118.5, 117.9, 107.7, 56.7.

7-Methoxy-4*H*-thieno[2,3-*c*]chromen-4-one (2.0 g, 8.6 mmol) was stirred in THF (30 mL) at 0 °C under $N_2$. A solution of MeMgBr (3.0 M in diethyl ether, 7.2 mL, 21.6 mmol) was added dropwise and the reaction mixture was stirred at 0 °C for 2 hours. The reaction mixture was then warmed to ambient temperature and stirred for 1 hour. Brine (50 mL) was added and the reaction mixture as extracted with EtOAc (3 × 50 mL). The organic layers were combined, dried ($Na_2SO_4$) and the solvent was removed under reduced pressure. The resulting residue was dissolved in toluene (10 mL) and molecular sieves 3 Å (2 g) added and the reaction mixture was heated at 100 °C for 6 hours. The reaction mixture was filtered and the solids were washed with toluene (10 mL). The solvent was removed under reduced pressure and the resulting residue was chromatographed on silica gel [eluent = 1.5:98.5 EtOAc:Hexane] to give 7-methoxy-4,4-dimethyl-4H-thieno[2,3-*c*]chromene as a colourless oil, (1.76 g, 83 %). $\delta_H$ (400 MHz, $CDCl_3$) 7.35 (dd, *J* = 6.4, 2.4 Hz, 1 H), 7.73 (app. s, 2 H), 6.60 - 6.51 (m, 2 H), 3.81 (s, 3 H), 1.68 (s, 6 H). $\delta_C$ (101 MHz, $CDCl_3$) 160.1, 152.8, 137.2, 131.1, 123.8, 123.7, 123.2, 122.2, 114.2, 107.5, 103.0, 77.7, 55.4, 28.9.

7-Methoxy-4,4-dimethyl-4*H*-thieno[2,3-*c*]chromene (0.50 g, 2.0 mmol) was dissolved in anhydrous THF (10 mL) and the reaction mixture was cooled to -78 °C under $N_2$. A solution of n-BuLi (2.5 M in hexanes, 0.84 mL, 2.1 mmol) was added dropwise and the reaction mixture was stirred at -78 °C under $N_2$ for 1 hour. Dibromoethane (DBE) (0.35 mL, 4.0 mmol) was added dropwise and the reaction mixture was warmed to ambient temperature. Brine (10 mL) was added and the reaction mixture was extracted with EtOAc (3 × 20 mL). The dried (anhyd. $Na_2SO_4$) solvent was removed under reduced pressure and the resulting residue was chromatographed on silica gel [eluent = 1:99 EtOAc:Hexane] to give an inseparable mixture of 7-methoxy-4,4-dimethyl-4*H*-thieno[2,3-*c*]chromene (45 %) and 2-bromo-7-methoxy-4,4-dimethyl-4*H*-thieno[2,3-*c*]chromene (55 %) (relative percentages determined by $^1$H NMR spectroscopy). The foregoing mixture was used directly in the next step without further purification. The aforementioned mixture was dissolved in a mixture of toluene and 1,4-dioxane (1:1) (8 mL) and (2-isopropoxy-6-methoxyphenyl)boronic acid (3.0 mmol) and $K_3PO_4$ (0.86, 4.0 mmol) was added. The reaction mixture was then degassed with argon and $Pd(OAc)_2$ (9 mg, 0.04 mmol) and X-Phos (38 mg, 0.08 mmol) was added. The reaction mixture was then stirred and heated at 105 °C for 16 hours under $N_2$. The reaction mixture was then cooled to ambient temperature whereupon brine (30 mL) was added followed by extraction with EtOAc (3 × 30 mL). The organic layers where combined, dried (anhyd. $Na_2SO_4$) and the solvent was removed under reduced pressure. The resulting residue was chromatographed on silica gel [eluent = 1:19 EtOAc:Hexane] to give 2-(2-isopropoxy-6-methoxyphenyl)-7-methoxy-4,4-dimethyl-4*H*-thieno[2,3-*c*]chromene as a colourless oil, (0.28 g, 31 %, yield over two steps). $\delta_H$ (300 MHz, $CDCl_3$) 7.66 (s, 1 H), 7.34 (dd, *J*= 5.4, 3.6 Hz, 1 H), 7.19 (t, *J* = 8.3 Hz 1 H), 6.64 (t, 8.3 Hz, 2 H), 6.58 - 6.49 (m, 2H), 4.56 (sept, *J* = 5.9 Hz, 1 H), 3.86 (s, 3H), 3.80 (s, 3 H) 1.70 (s, 6 H), 1.34 (d, *J* = 6.1 Hz, 6 H). $\delta_C$ (75 MHz, $CDCl_3$) 159.6, 157.7, 155.9, 152.9, 137.2, 132.9, 129.6, 128.3, 123.8, 123.5, 114.6, 113.6, 107.7, 107.4, 104.2, 102.9, 77.8, 71.6, 55.9, 55.4, 29.0, 22.1.

**B.6. Synthesis of 7,7'-Dimethoxy-4,4,4',4'-tetramethyl-4*H*,4'*H*-2,2'-bithieno[2,3-*c*]chromene - Compound 16**

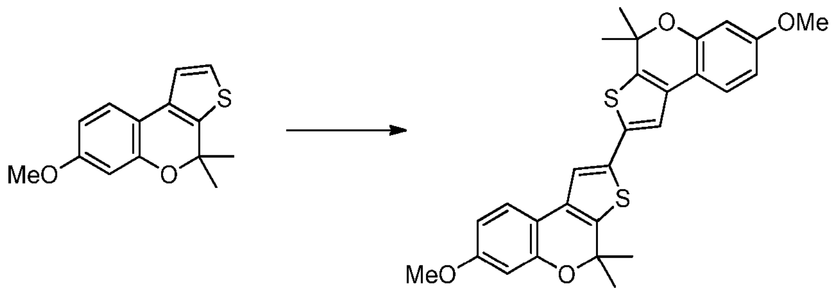

*Reaction scheme B.6*

7-Methoxy-4,4-dimethyl-4*H*-thieno[2,3-*c*]chromene (0.50 g, 2.0 mmol) was dissolved in anhydrous THF (10 mL) and the reaction mixture was cooled to -78 °C under $N_2$. A solution of n-BuLi (2.5 M in hexanes, 1.0 mL, 2.5 mmol) was added dropwise and the reaction mixture was stirred at -78 °C under $N_2$ for 20 minutes then slowly warmed to -15 °C. The reaction mixture was then re-cooled to -78 °C and $CuCl_2$ (0.54 g, 4 mmol) was added. The reaction mixture was then slowly warmed to room temperature and then diluted with toluene (30 mL) followed by brine (30 mL). The reaction mixture was then extracted with EtOAc (3 × 30 mL). The organic layers where combined, dried (anhyd. $Na_2SO_4$) and the solvent was removed under reduced pressure. The resulting residue was chromatographed on silica gel [eluent = 1:19 EtOAc:Hexane graduated to 1:9 EtOAc:Hexane] to give 7,7'-dimethoxy-4,4,4',4'-tetramethyl-4*H*,4'*H*-2,2'-bithieno[2,3-*c*]chromene as a colourless solid, (0.35 g, 70 %). $\delta_H$ (300 MHz, $CDCl_3$) = 7.33 (dd, *J* = 7.9, 0.6 Hz, 2 H), 7.25 (app. s, 2 H), 6.60 - 6.51 (m, 4 H), 3.81 (s, 6 H), 1.68 (s, 12 H). $\delta_C$ (75 MHz, $CDCl_3$) 160.2, 153.0, 136.3, 135.7, 131.8, 123.6, 118.5, 113.7, 107.6, 103.0, 77.5, 55.4, 28.8.

**B.7. Synthesis of 2,5-bis(7-Methoxy-2,2-dimethylchroman-8-yl)furan - Compound 6**

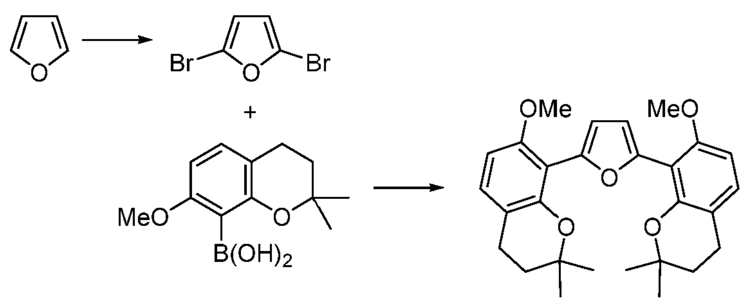

*Reaction scheme B.7*

A mixture of furan (20 g, 294 mmol) and NBS (104.71 g, 588.4 mmol) in DCM (600 ml) was sonicated for 5 min with occasional stirring, left to stand at rt for 12 h. The resulting mixture was washed with water (400 ml), dried ($Na_2SO_4$) and the solvent removed under reduced pressure. The residue was filtered through silica using pet ether (40-60) as eluent and the solvent removed under reduced pressure to give 2,5-dibromofuran as a pale-yellow oil, (15.65 g, 23 %) which was used without further purification in the next step.

2,5-Dibromofuran (1.31 g, 5.8 mmol), (7-methoxy-2,2-dimethylchroman-8-yl)boronic acid (3.48 g, 14.5 mmol, 2.5 equiv.), potassium carbonate (4.82 g, 34.9 mmol, 6 equiv.) and tetrakis(triphenylphosphine)palladium(0) (669 mg g, 0.3 mmol, 10 mol%) were added to degassed PhMe (30 mL) and EtOH (30 mL), the reaction mixture was stirred at reflux under $N_2$ for 16 h. The reaction was allowed to cool to r.t., poured onto water (100 mL) and extracted with DCM (3 × 50 mL). The combined organic portions were dried ($Na_2SO_4$) and the solvent removed under reduced pressure. The product was purified by flash column chromatography [10 - 15 % gradient EtOAc in pet ether 40-60] to afford 2,5-bis(7-methoxy-2,2-dimethylchroman-8-yl)furan as a viscous pale-yellow oil which was crystallised from pentane (2.24 g, 79 %). $\delta_H$ (600 MHz, $CDCl_3$) 1.32 [12H, s, 2 × C(C*H*$_3$)$_2$], 1.78 [4H, t, *J* = 6.8 Hz,2 × C*H*$_2$C(CH$_3$)$_2$], 2.75 [4H, dt, *J* = 0.9, 6.8 Hz, 2 × C*H*$_2$CH$_2$C(CH$_3$)$_2$], 3.79 (6H, s, 2 × OC*H*$_3$), 6.49 (2H, d, *J* = 8.4 Hz, 2 × 6-*H*), 6.72 (2H, s, 3-*H*, 4-*H*), 6.94 (2H, dt, *J* = 0.9, 8.4 Hz, 2 × 5-*H*). $\delta_C$ (150 MHz, $CDCl_3$) 22.3 (2 × $CH_2$), 27.0 (4 × $CH_3$), 32.7 (2 × $CH_2$), 56.7 (2 × $CH_3$), 74.5 (2 × C), 104.3 (2 × CH), 1110.3 (2 × C), 112.3 (2 × C), 114.0 (2 × CH), 128.7 (2 × CH), 146.3 (2 × C), 152.4 (2 × C), 157.0 (2 × C).

**B.8. Synthesis of 2,5-bis(7-Methoxy-2,2-dimethylchroman-8-yl)thieno[3,2-*b*]thiophene - Compound 14**

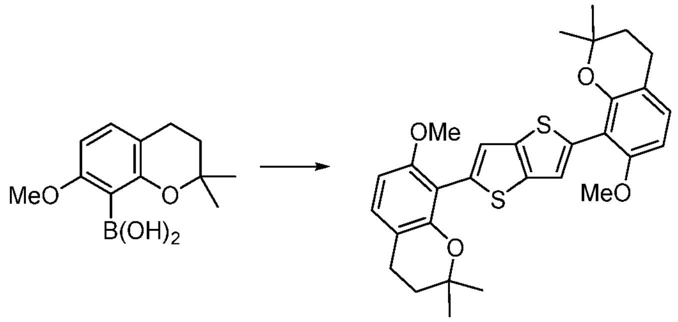

*Reaction scheme B.8*

2,5-Dibromothieno[3,2-b]thiophene (1.50 g, 5.0 mmol), (7-methoxy-2,2-dimethylchroman-8-yl)boronic acid (3.78 g, 11 mmol, 2.2 equiv.), potassium carbonate (3.05 g, 22.1 mmol, 4.4 equiv.) and tetrakis(triphenylphosphine) palladium(0) (500 mg, 0.43 mmol, 9 mol%) were added to degassed PhMe (30 mL) and EtOH (30 mL), the reaction mixture was stirred at reflux under $N_2$ for 16 h. The reaction was allowed to cool to r.t., poured onto water (100 mL) and extracted with DCM (3 × 50 mL). The combined organic portions were dried ($Na_2SO_4$) and the solvent removed under reduced pressure. The product was purified by flash column chromatography [10 % EtOAc in pet ether 40-60] to DCM [40 % in pet ether 40-60] gradient. The solvent was removed under reduced pressure and the residue crystallised twice from hot EtOAc/hexanes to afford 2,5-bis(7-methoxy-2,2-dimethylchroman-8-yl)thieno[3,2-*b*]thiophene as yellow needles (1.75 g, 47 %). $\delta_H$ (600 MHz, $CDCl_3$) 1.38 [12H, s, 2 × $C(CH_3)_2$], 1.81 [4H, t, *J* = 6.8 Hz, 2 × $CH_2C(CH_3)_2$], 2.78 [4H, dt, *J* = 1, 6.8 Hz, 2 × $CH_2CH_2C(CH_3)_2$], 3.83 (6H, s, 2 × $OCH_3$), 6.53 (2H, d, *J* = 8.4 Hz, 2 × 6-*H*), 6.97 (2H, dt, *J* = 1, 8.4 Hz, 2 × *5-H),* 7.66 (2H, s, 3-*H,* 6-*H*). $\delta_C$ (150 MHz, $CDCl_3$) 22.4, 27.0, 32.7, 56.0, 75.2, 103.4, 112.8, 114.0, 121.0, 128.5, 135.6, 139.0, 151.9, 156.1.

**B.9. Synthesis of 2,5-bis(2-Ethyl-7-methoxy-2-methylchroman-8-yl)thieno[3,2-*b*]thiophene - Compound 15**

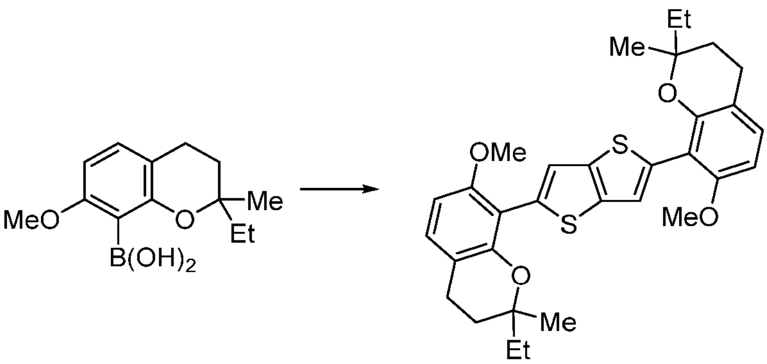

*Reaction scheme B.9*

2,5-Dibromothieno[3,2-b]thiophene (1.50 g, 5.0 mmol), (2-ethyl-7-methoxy-2-methylchroman-8-yl)boronic acid (2.77 g, 11 mmol, 2.2 equiv.), potassium carbonate (2.29 g, 16.6 mmol, 3.32 equiv.) and tetrakis(triphenylphosphine) palladium(0) (290 mg, 0.25 mmol, 5 mol%) were added to degassed PhMe (30 mL) and EtOH (30 mL), the reaction mixture was stirred at reflux under $N_2$ for 16 h. The reaction was allowed to cool to r.t., poured onto water (100 mL) and extracted with DCM (3 × 50 mL). The combined organic portions were dried ($Na_2SO_4$) and the solvent removed under reduced pressure. The product was purified by flash column chromatography on silica using (DCM/EtOAc/pet ether [40-60]) (10/7/83) eluent. The fluorescent band was collected and the solvent was removed under reduced pressure. The residue crystallised hot PhMe/hexanes to give the *title compound* (2.16 g, 49 %) as yellow needles. $\delta_H$ (600 MHz, $CDCl_3$) 0.97 [6H, t, *J* = 7.5 Hz, $CH_2CH_3$], 1.31 [6H, s, 2 × $C(CH_3)_2$], 1.60 - 1.90 [8H, m], 2.73 - 2.85 [4H, m], 3.84 (6H, s), 6.54 (2H, d, *J* = 8.4 Hz,

2 × *6-H),* 6.98 (2H, dt, *J* = 1, 8.4 Hz, 2 × 5-*H*), 7.66 (2H, s, 3-*H*, 6-*H*). $\delta_C$ (150 MHz, $CDCl_3$) 8.3, 22.0, 23.5, 30.3, 32.6, 56.0, 77.6, 103.3, 112.8, 114.3, 121.0, 128.5, 135.5, 139.0, 151.9, 156.2.

**B.10. Synthesis of 2,5-bis(7-Ethoxy-2,2-dimethylchroman-8-yl)thiophene - Compound 5**

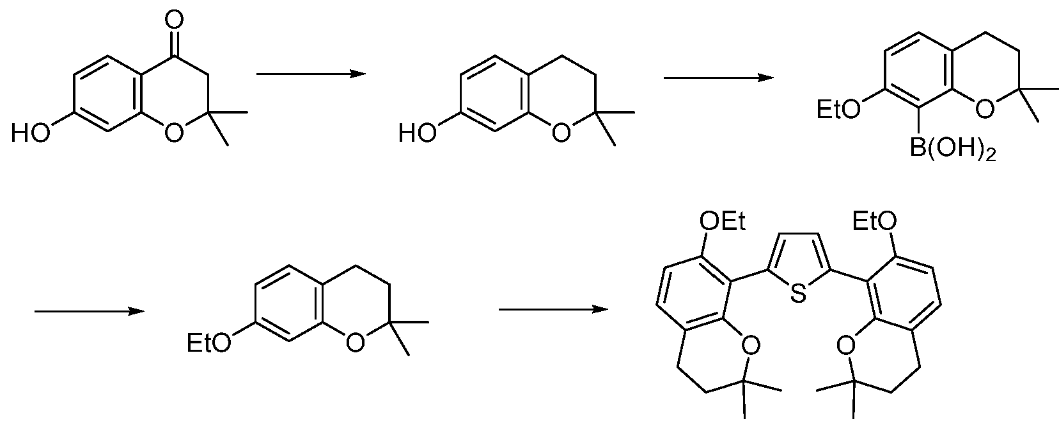

*Reaction scheme B.10*

To a vigorously stirred mixture of 7-hydroxy-2,2-dimethylchroman-4-one (10 g, 40.9 mmol) and Zn (33.34 g, 513 mmol) in MeOH (100 mL) was added c. HCl (100 mL) dropwise. After the addition stirring was continued for 2 h and the mixture poured into water (400 mL) and extracted with DCM (3 × 120 mL). The combined organic phase was washed with water (200 ml), dried ($Na_2SO_4$) and the solvent removed under reduced pressure to give 2,2-dimethylchroman-7-ol (9.03 g, 97 %) as a colourless oil which was used without further purification in the next step.

A mixture of 2,2-dimethylchroman-7-ol (5 g, 28.1 mmol), EtI (8.76 g, 56.2 mmol) and $K_2CO_3$ (9.69 g, 70.2 mmol) in AcMe (60 mL) was heated at reflux for 6 h, cooled, poured into water and extracted with DCM (3 × 50 mL). The combined organic phase was dried ($Na_2SO_4$) and the solvent removed under reduced pressure. The residue was filtered through silica using DCM (50 % in pet ether 40-60) as eluent. The solvent was removed under reduced pressure to give 7-ethoxy-2,2-dimethylchromane as a colourless oil, (4.46 g, 77 %). $\delta_H$ (600 MHz, $CDCl_3$) 6.83 (app. d, *J* = 8.3 Hz, 1 H), 6.32 (dd, *J* = 8.3, 2.5 Hz, 1 H), 6.24 (d, *J* = 2.5 Hz, 1 H), 3.87 (q, *J* = 7 Hz, 2 H), 2.60 (app. t, *J* = 6.7 Hz, 2 H), 1.68 (t, *J* = 6.8 Hz, 2 H), 1.28 (t, *J* = 6.7 Hz, 2 H), 1.23 (s, 6 H). $\delta_C$ (150 MHz, $CDCl_3$) 158.4, 154.6, 129.8, 112.9, 107.5, 102.3, 74.2, 63.3, 33.0, 26.8, 21.7, 14.9.

A solution of BuLi (10.5 mL, 2.5 M, 26.2 mmol) in hexanes was added dropwise to a solution of 7-ethoxy-2,2-dimethylchromane (4.50 g, 21.8 mmol) and TMEDA (4 mL, 26.8 mmol) in THF (50 mL) with stirring at -78 °C. Stirring was continued for 20 min $B(OMe)_3$ (4.7 mL, 42 mmol) was added in one portion with stirring. The resulting solution was warmed to rt, acidified with HCl (2 M, 50 mL) and the phases separated. The aqueous phase was extracted with $Et_2O$ (2 × 50 ml) and the combined organic phase washed with water (100 mL), dried ($Na_2SO_4$). The solvent was removed under reduced pressure and the residue triturated with hexanes to give (7-ethoxy-2,2-dimethylchroman-8-yl)boronic acid as a colourless powder, (3.44 g, 63 %) which was used without further purification in the next step.

2,5-Dibromothiophene (1.41 g, 5.8 mmol), (7-ethoxy-2,2-dimethylchroman-8-yl)boronic acid (3.00 g, 12.1 mmol, 2.5 equiv.), potassium carbonate (4.82 g, 34.9 mmol, 6 equiv.) and tetrakis(triphenylphosphine)palladium(0) (336 mg g, 0.15 mmol, 5 mol%) were added to degassed PhMe (30 mL) and EtOH (30 mL), the reaction mixture was stirred at reflux under $N_2$ for 16 h. The reaction was allowed to cool to r.t., poured onto water (100 mL) and extracted with DCM (3 × 50 mL). The combined organic portions were dried ($Na_2SO_4$) and the solvent removed under reduced pressure. The product was purified by flash column chromatography [10 % EtOAc in pet ether 40-60]. The solvent was removed under reduced pressure and the residue crystallised from DCM/pet ether (40-60) to afford 2,5-bis(7-ethoxy-2,2-dimethylchroman-8-yl) thiophene (2.24 g, 79 %). $\delta_H$ (400 MHz, $CDCl_3$) 1.40 [12H, s, 2 × $C(CH_3)_2$], 1.43 [6H, t, *J* = 6.9 Hz, $OCH_2CH_3$] 1.81 [4H, t, *J* = 6.8 Hz, 2 × $CH_2C(CH_3)_2$], 2.89 [4H, dt, *J* = 1.0, 6.8 Hz, 2 × $CH_2CH_2C(CH_3)_2$], 4.06 (4H, q, *J* = 6.9 Hz, 2 × $OCH_2$), 6.53 (2H, d, *J* = 8.4 Hz, 2 × 6-*H*), 6.91 (2H, dt, *J* = 1, 8.4 Hz, 2 × *5-H),* 7.68 (2H, s, 3-*H*, 4-*H*). $\delta_C$ (100 MHz, $CDCl_3$) 15.0 (2 × $CH_2CH_2C(CH_3)_2$), 22.5 (2 × $CH_2CH_2C(CH_3)_2$, 27.0 (4 × $CH_3$), 32.8 (2 × $CH_2CH_2C(CH_3)_2$), 64.8 (2 × $OCH_2CH_3$), 74.8 (2 × C), 105.0 (2 × CH), 113.4 (2 × C), 113.9 (2 × C), 127.6 (2 × CH), 127.9 (2 × CH), 133.9 (2 × C), 151.6 (2 × C), 155.2 (2 × C).

**B.11. Synthesis of 2,5-bis(2,2,8,8-tetramethyl-3,4,7,8-tetrahydro-2*H*,6*H*-pyrano[3,2-g]chromen-10-yl)thiophene - Compound 4**

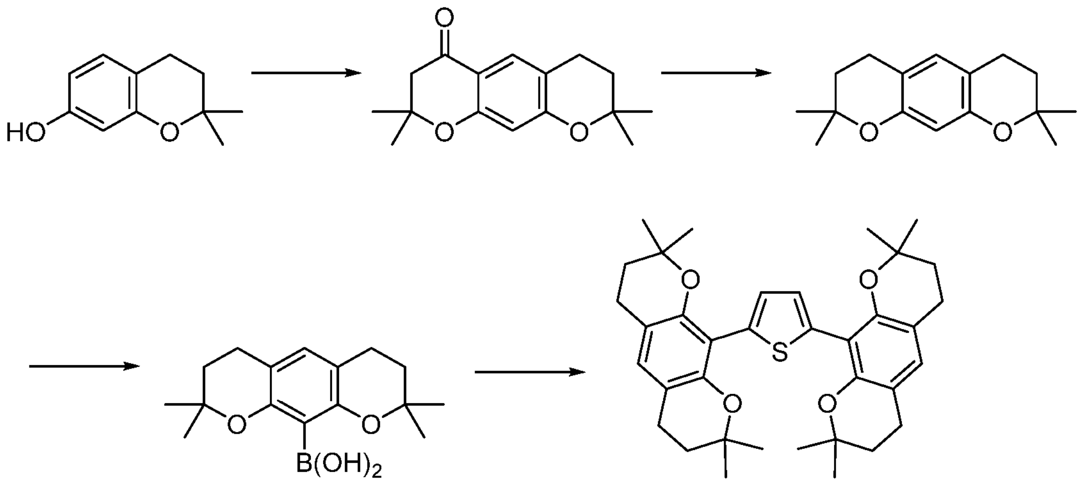

*Reaction scheme B.11*

A mixture of 2,2-dimethylchroman-7-ol (10.71 g, 60.2 mmol) and 3,3-dimethylacrylic acid (6.62 g, 62 mmol) in methansulfonic acid (50 ml) was heated at 50 °C for 2 h and poured into water (300 ml). The resulting mixture was basified with $NaHCO_3$ and extracted with EtOAc (3 x 100 ml). The combined organic phase was washed with aq. $NaHCO_3$ (100 ml), dried ($Na_2SO_4$) and the solvent removed under reduced pressure. The residue was filtered through silica using DCM as eluent and the solvent removed under reduced pressure to give 2,2,8,8-tetramethyl-3,4,7,8-tetrahydro-2*H*,6*H*-pyrano [3,2-*g*]chromen-6-one as a colourless powder, (12.34 g, 74 %). $\delta_H$ (400 MHz, $CDCl_3$) 1.34 (6H, s, 2 $\times$ $CH_3$), 1.42 (6 H, s, 2 $\times$ $CH_3$), 1.80 (2 H, t, *J* = 6.7 Hz, $CH_2CH_2C(CH_3)$), 2.63 (2 H, s, $CH_2CO$), 2.73 (2 H, dt, *J* = 1, 6.7 Hz, $CH_2CH_2C(CH_3)$), 6.26 (1H, s, CH), 6.60 (1H, t, *J* = 1 Hz, CH). $\delta_C$ (100 MHz, $CDCl_3$) 21.6, 26.8, 27.0, 32.7, 48.8, 75.8, 79.0, 104.8, 113.8, 114.9, 127.7, 159.9, 161.4, 191.5.

To a vigorously stirred mixture of 2,2,8,8-tetramethyl-3,4,7,8-tetrahydro-2*H*,6*H*-pyrano[3,2-*g*]chromen-6-one (12.31 g, 47.3 mmol) and Zn (38.47 g, 592 mmol) in MeOH (200 mL) was added c. HCl (120 mL) dropwise. After the addition stirring was continued for 2 h and the mixture poured into water (500 mL) and extracted with DCM (3 $\times$ 150 mL). The combined organic phase was washed with water (200 mL), dried ($Na_2SO_4$) and the solvent removed under reduced pressure to give 2,2,8,8-tetramethyl-3,4,7,8-tetrahydro-2*H*,6*H*-pyrano[3,2-*g*]chromene (11.33 g, 97 %) as a colourless powder. $\delta_H$ (400 MHz, $CDCl_3$) 1.35 (12H, s, 4 $\times$ $CH_3$), 1.80 (4 H, t, *J* = 6.7 Hz, 2 $\times$ $CH_2CH_2C(CH_3)$), 2.71 (2 H, app. t, *J* = 6.7 Hz, 2 $\times$ $CH_2CH_2C(CH_3)$), 6.27 (1H, s, CH), 6.77 (1H, app. s, CH). $\delta_C$ (100 MHz, $CDCl_3$) 21.8, 26.9, 33.2, 73.8, 104.8, 112.6, 129.4, 153.1.

A solution of BuLi (21.5 ml, 2.5 M, 53.8 mmol) in hexanes was added dropwise to a solution of 2,2,8,8-tetramethyl-3,4,7,8-tetrahydro-2*H*,6*H*-pyrano[3,2-*g*]chromene (11.01 g, 44.8 mmol) and TMEDA (8.1 mL, 54.3 mmol) in THF (100 mL) with stirring at -78 °C. Stirring was continued for 20 min $B(OMe)_3$ (9.7 mL, 86.7 mmol) was added in one portion with stirring. The resulting solution was warmed to rt, acidified with HCl (2 M, 100 mL) and the phases separated. The aqueous phase was extracted with $Et_2O$ (2 $\times$ 50 mL) and the combined organic phase washed with water (100 mL), dried ($Na_2SO_4$). The solvent was reduced under reduced pressure (~50 mL) and diluted with hexanes. The resultant precipitate was filtered, washed with hexanes and air dried to give (2,2,8,8-tetramethyl-3,4,7,8-tetrahydro-2*H*,6*H*-pyrano [3,2-*g*]chromen-10-yl)boronic acid as a colourless powder, (5.54 g, 43 %) which was used without further purification in the next step. $\delta_H$ (600 MHz, $CDCl_3$) 1.40 (12H, s, 4 $\times$ $CH_3$), 1.83 (4 H, t, *J* = 6.8 Hz, 2 $\times$ $CH_2CH_2C(CH_3)$), 2.73 (4 H, dt, *J* = 1.0, 6.8 Hz, 2 $\times$ $CH_2CH_2C(CH_3)$), 6.89 (1H, p, *J* = 1.0 Hz, CH), 7.56 (2H, s, $B(OH)_2$). $\delta_C$ (150 MHz, $CDCl_3$) 21.9, 26.9, 32.5, 76.0, 105.5, 113.2, 133.1, 157.9.

2,5-Dibromothiophene (1.41 g, 5.8 mmol), (2,2,8,8-tetramethyl-3,4,7,8-tetrahydro-2*H*,6*H*-pyrano[3,2-*g*]chromen-10-yl)boronic acid (3.58 g, 12.5 mmol, 2.15 equiv.), potassium carbonate (4.82 g, 34.9 mmol, 6 equiv.) and tetrakis(triphenylphosphine)palladium(0) (336 mg g, 0.15 mmol, 5 mol%) were added to degassed PhMe (30 mL) and EtOH (30 mL), the reaction mixture was stirred at reflux under $N_2$ for 16 h. The reaction was allowed to cool to r.t., poured onto water (100 mL) and extracted with DCM (3 $\times$ 50 mL). The combined organic portions were dried ($Na_2SO_4$) and the solvent removed under reduced pressure. The residue was filtered through silica using DCM/EtOAc/hex (30/5/65) and the solvent removed under reduced pressure. The residue was crystallised twice from hot DCM (40 ml) at - 20 °C. The

colourless plates were dried under vacuum (70 °C, 2 × $10^{-2}$ mmHg) afford 2,5-bis(2,2,8,8-tetramethyl-3,4,7,8-tetrahydro-2*H*,6*H*-pyrano[3,2-*g*]chromen-10-yl)thiophene as a colourless powder (2.49 g, 76 %). $\delta_H$ (400 MHz, $CDCl_3$) 1.39 (24H, s, 8 × $CH_3$), 1.79 (8 H, t, *J* = 6.8 Hz, 4 × $CH_2CH_2C(CH_3)$), 2.75 (8 H, t, *J* = 6.8 Hz, 4 × $CH_2CH_2C(CH_3)$), 6.67 (2H, bs, 2 ×CH), 7.74 (2H, bs, 2 ×CH). $\delta_C$ (150 MHz, $CDCl_3$) 22.5, 27.1, 33.0, 53.4, 74.5, 112.2, 127.5, 126.6, 134.0, 149.8.

**B.12. Synthesis of 2,5-bis(2-Ethyl-7-methoxy-2-methylchroman-8-yl)thiophene - Compound 9**

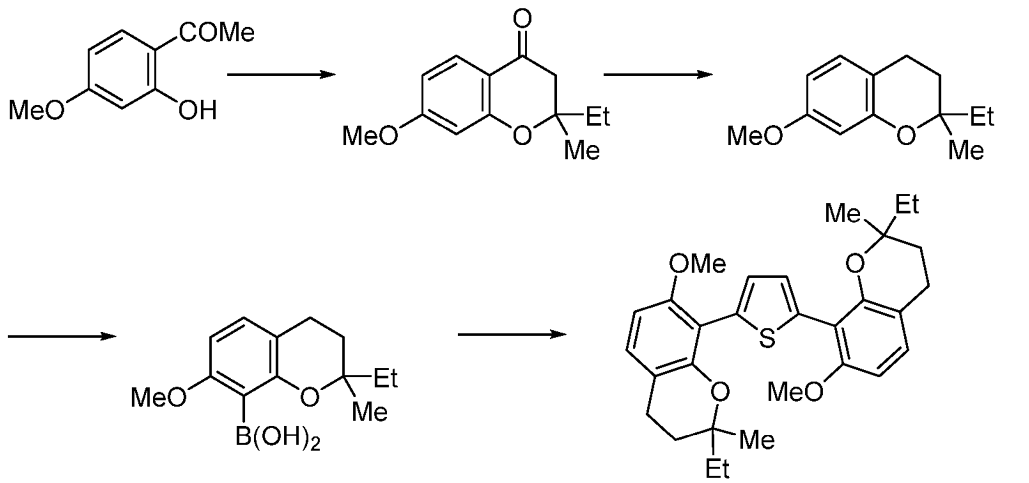

*Reaction scheme B.12*

A solution of 1-(2-hydroxy-4-methoxyphenyl)ethan-1-one (8 g, 48.2 mmol), butanone (5.20 g, 72.2 mmol) and pyrrolidine (5.13 g, 72.2 mmol) in MeOH (200 mL) was stood at rt overnight. The solvent was removed under reduced pressure and the residue chromatographed on silica using DCM 80 - 100 % gradient in pet ether 40-60) as eluent. The second band was collected and the solvent was removed under reduced pressure to give 2-ethyl-7-methoxy-2-methylchroman-4-one as pale brown viscous oil, (3.37 g, 34 %) which was used in the next step without further purification.

A mixture of 2-ethyl-7-methoxy-2-methylchroman-4-one (9.83 g, 44.7 mmol) and Zn (36.30 g, 558 mmol) in MeOH (100 mL) was added c. HCl (100 mL) dropwise. After the addition stirring was continued for 2 h and the mixture poured into water (300 mL) and extracted with DCM (3 × 80 mL). The combined organic phase was washed with water (100 mL), dried ($Na_2SO_4$) and the solvent removed under reduced pressure to give 2-ethyl-7-methoxy-2-methylchromane (8.64 g, 94 %) as a colourless oil which was used without further purification in the next step. $\delta_H$ (600 MHz, $CDCl_3$) = 6.84 (dt, *J* = 0.9, 8.3 Hz, 1 H), 6.33 (dd, *J* = 8.3, 2.6 Hz, 1 H), 6.27 (d, *J* = 2.6 Hz, 1 H), 3.66 (s, 3 H), 2.60 (dt, *J* = 0.9, 6.5 Hz, 2 H), 1.76 - 1.47 (m, 4 H), 1.17 (app. s, 3 H), 0.87 (app. t, *J* = 7.5 Hz, 3 H). δc (150 MHz, $CDCl_3$) 159.1, 154.8, 129.8, 113.3, 106.8, 101.8, 76.6, 55.2, 32.2, 30.6, 23.6, 21.4, 8.0.

A solution of BuLi (20.1 mL, 2.5 M, 52.5 mmol) in hexanes was added dropwise to a solution of 2-ethyl-7-methoxy-2-methylchromane (8.64 g, 41.9 mmol) and TMEDA (8.1 mL, 50.9 mmol) in THF (100 mL) with stirring at -78 °C. Stirring was continued for 20 min and $B(OMe)_3$ (9.1 mL, 81.3 mmol) was added in one portion with stirring. The resulting solution was warmed to rt, acidified with HCl (2 M, 100 mL) and the phases separated. The aqueous phase was extracted with $Et_2O$ (2 × 50 mL) and the combined organic phase washed with water (100 mL), dried ($Na_2SO_4$) and the solvent was reduced under reduced pressure. The residue was chromatographed on silica using EtOAc (70 % in pet ether 40-60) as eluent. The second band was collected and the solvent removed under reduced pressure to give (2-ethyl-7-methoxy-2-methylchroman-8-yl)boronic acid as a pale yellow oil, (7.30 g, 70 %). $\delta_H$ (400 MHz, $CDCl_3$) 0.98 (3H, t, *J* = 7.5 Hz, $CH_2CH_3$), 1.33 (3 H, s, $CH_2C(CH_3)Et$), 1.60 - 1.93 (4 H, m), 2.74 (2H, app. t, *J* = 6.7 Hz, $CH_2CH_2C(Me)Et$), 3.87 (3H, s, OMe), 6.51 (1H, d, *J* = 8.5 Hz, CH), 7.11 (1H, app. d, *J* = 8.5 Hz, CH), 7.39 (2H, s, $B(OH)_2$). $\delta_C$ (150 MHz, $CDCl_3$). 8.0, 21.6, 23.8, 30.0, 32.2, 55.9, 78.9, 103.3, 114.7, 133.0, 159.5, 163.9, (C8 not observed under applied NMR acquisition parameters)

2,5-Dibromothiophene (1.41 g, 5.8 mmol), (2-ethyl-7-methoxy-2-methylchroman-8-yl)boronic acid (3.00 g, 12.1 mmol, 2.08 equiv.), potassium carbonate (4.82 g, 34.9 mmol, 6 equiv.) and tetrakis(triphenylphosphine)palladium(0) (336 mg g, 0.15 mmol, 5 mol%) were added to degassed PhMe (30 mL) and EtOH (30 mL), the reaction mixture was stirred at reflux under $N_2$ for 16 h. The reaction was allowed to cool to r.t., poured onto water (100 mL) and extracted with DCM (3 × 50 mL). The combined organic portions were dried ($Na_2SO_4$) and the solvent removed under reduced pressure. The residue was chromatographed on silica using EtOAc (12 % in pet ether 40-60) as eluent. The solvent was removed under reduced pressure and the residue chromatographed on silica using EtOAc (8 - 15 % in pet ether 40-60) as eluent to give

2,5-bis(2-ethyl-7-methoxy-2-methylchroman-8-yl)thiophene as a viscous yellow oil (1.82 g, 64 %). $\delta_H$ (400 MHz, $CDCl_3$) 0.92 - 0.99 (6H, m, 2 × $CH_2CH_3$), 1.29 (6H, s, $CH_3$), 1.59 - 1.90 (8 H, m), 2.70 - 2.85 (4 H, m), 6.52 (2H, *J* = 8.4 Hz, 2 × CH), 6.93 (2H, bd, *J* = 8.4 Hz, 2 ×CH),7.55 (2H, bs). $\delta_C$[1] (100 MHz, $CDCl_3$) 8.2, 22.1, 23.4, 23.5, 30.4, 32.5, 32.6, 56.077.3, 103.4, 113.0, 114.2, 127.7, 127.8, 133.9, 151.7, 156.0. ([1]Mixture of diastereomers, all carbon resonances are listed)

**Example 2: characterization and activity of exemplary compounds of formula (I)**

The oxidation-reduction potentials of exemplary compounds of formula (I) were measured and compared to a compound of the prior art.

Procedure for the measurement of the oxidation-reduction potential: cyclic voltammetry method with 3 electrodes:

The oxido-reduction potentials of the compounds are measured by cyclic voltammetry with 3 electrodes:

- 1 platinum working electrode
- 1 platinum counter electrode
- 1 platinum reference electrode immersed in a solution composed of 0.01M AgNO3 + 0.1 TBAP (tetrabutylammonium perchlorate) in acetonitrile.

All redox potentials are given for scan rate of the potential fixed to 100mV/s.
$E_1^{ox}$ refers to the first oxidation peak of the analyzed molecule.
$E_2^{ox}$ refers to the second oxidation peak of the analyzed molecule.
$E_1^{1/2}$ corresponds to the oxido-reduction potential of an oxidant/reductor system as calculated below:

$E_1^{1/2} = (E1^{red} + E_1^{ox})/2$; wherein $E_1^{red}$ corresponds to the first reduction peak of the analyzed compound. $\Delta E^{ox}$ corresponds to the difference between $E_1^{ox}$ and $E_2^{ox}$ as calculated below:

$$\Delta E^{ox} = |E_2^{ox}| - |E_1^{ox}|.$$

The values of the indicated potentials correspond to the first oxidation/reduction potentials of the molecules compared to a standard reference electrode of hydrogen SHE.
The solution used for the analysis was prepared with 0.005 M of the electrochromic dye to be analyzed, 0.250 M of $TBABF_4$ salt in propylene carbonate as solvent.

Measurement of the absorption spectrum of the dyes by spectroelectrochemistry:

*Principle of the measurement:*

In a quartz cell, the dye of formula (I) is activated on a platinum grid using a scan of the potential close to the $E^{1/2}$ potential. The absorption spectrum is recorded in a continuous manner.
The solution of the dye to be analyzed is composed of :

0.005 M of the dye of formula (I) to be analyzed

0.250 M of TBA BF4- in propylene carbonate as solvent

*Method of measurement:*

The solution with the dye of formula (I) is introduced into a quartz cell where at least one working electrode in the form of a platinum grid is placed to color the analyzed compound of formula (I) on this electrode. The absorption spectrum of the analyzed compound as a function of time is measured by uv-visible spectroscopy.
The counter electrode is a platinum wire and the reference electrode is a platinum wire immersed in a solution containing 0.01M AgNo3 and 0.1 M TBAP in acetonitrile.
The activation potential falls within the scan of the potential as follows E1/2 +0.2V to E1/2 - 0.1V.
The results for each of the synthesized compounds are indicated in **Table 1** below. $E_1^{ox}$ corresponds to the first

oxidation potential. The color indicated in Table 1 is the visual colour perceived by emmetropic eyes under day light conditions.

For comparison purposes, **Table 1** also includes the results obtained for a known compound, COMP.1 (a red methyl phenothiazine).

By comparing the known compound (comparative reducing electrochromic compound 1) COMP. 1 (red) to the exemplary compound of formula (I), Compound 11 (orange), we see that Compound 11 features a narrower absorption band than COMP. 1 and, in addition, the FWMH (full width at mid height) of compound 11 is about twice smaller than that of COMP. 1 as seen in **Table 2.** As previously indicated, narrow absorption bands are particularly interesting since it allows manufacturing optical devices which provide better eye protection, visual aid and contrast enhancement.

In addition, the molar absorptivity of the compounds of formula (I) is as seen in **Table 2** below, in many cases significantly higher when compared to that of other compounds of the prior art, such as for example COMP. 1. For example, the molar absorptivity of compound 11 is of $\varepsilon_{503}$ of 32500 L/mol/cm, whereas COMP. 1 features a molar absorptivity $\varepsilon_{520}$ of only 6130 L/mol/cm. Accordingly, the use of compounds of formula (I) herein disclosed having a high absorptivity is particularly interesting since it allows reducing the concentration of those dyes in the formulations and devices comprising those, while providing the desired tint with the desired intensity when activated.

**Table 1**

| Compound | Structure | $E_1^{1/2}$ (V) | $E_1^{1/2}$ (V) vs SHE | Color | $E_1^{ox}$ peak potential (V) | E1 peak potential (V) vs SHE | $E_2^{ox}$ peak potential (V) | E2 peak potential (V) vs SHE | $\Delta E^{ox}$ |
|---|---|---|---|---|---|---|---|---|---|
| 1 | 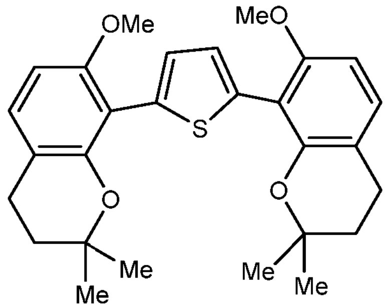 | 0.52 | 1.062 | Brown | 0.57 | 1.112 | 0.93 | 1.472 | 0.36 |
| 2 | 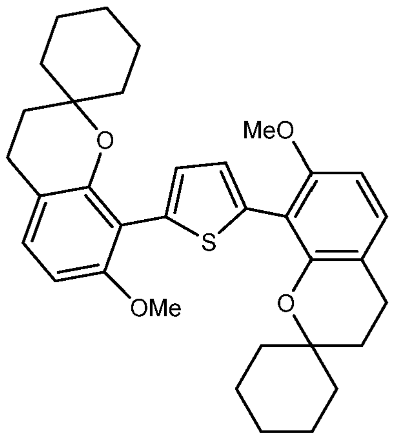 | 0.53 | 1.072 | Brown | 0.55 | 1.092 | 0.98 | 1.522 | 0.43 |

(continued)

| Compound | Structure | $E_1^{1/2}$ (V) | $E_1^{1/2}$ (V) vs SHE | Color | $E_1^{ox}$ peak potential (V) | E1 peak potential (V) vs SHE | $E_2^{ox}$ peak potential (V) | E2 peak potential (V) vs SHE | $\Delta E^{ox}$ |
|---|---|---|---|---|---|---|---|---|---|
| 3 | 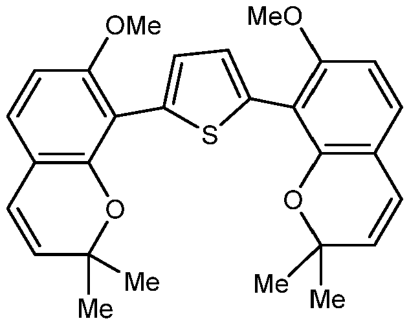 | n.a. | n.a. | Grey | 0.69 | 1.232 | 0.99 | 1.532 | 0.3 |
| 4 | 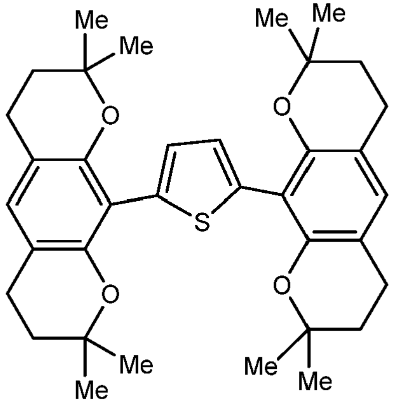 | 0.42 | 0.96 | Orange | 0.47 | 1.012 | 0.84 | 1.382 | 0.37 |

(continued)

| Compound | Structure | $E_1^{1/2}$ (V) | $E_1^{1/2}$ (V) vs SHE | Color | $E_1^{ox}$ peak potential (V) | E1 peak potential (V) vs SHE | $E_2^{ox}$ peak potential (V) | E2 peak potential (V) vs SHE | $\Delta E^{ox}$ |
|---|---|---|---|---|---|---|---|---|---|
| 5 | 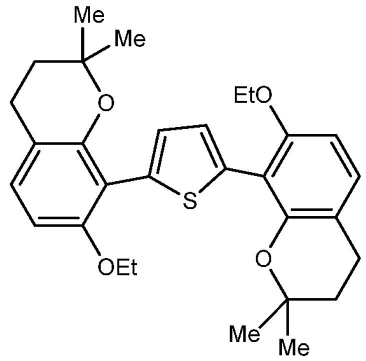 | 0.51 | 1.05 | Red | 0.53 | 1.072 | 0.85 | 1.392 | 0.32 |
| 6 | 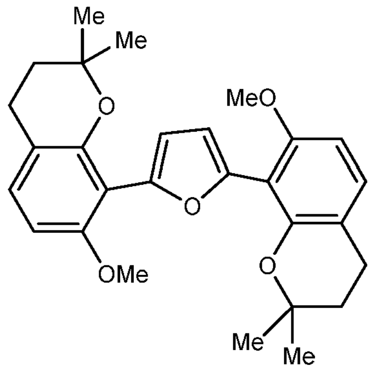 | 0.46 | 1 | Orange | 0.5 | 1.042 | 0.8 | 1.342 | 0.3 |
| 7 | 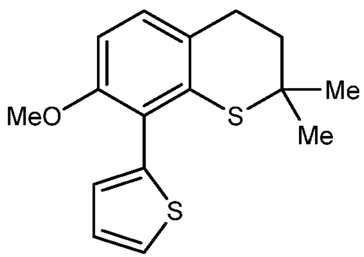 | n.a | n.a | Pale pink | 0.96 | 1.502 | n.a. | n.a | n.a |

(continued)

| Compound | Structure | $E_1^{1/2}$ (V) | $E_1^{1/2}$ (V) vs SHE | Color | $E_1^{ox}$ peak potential (V) | E1 peak potential (V) vs SHE | $E_2^{ox}$ peak potential (V) | E2 peak potential (V) vs SHE | $\Delta E^{ox}$ |
|---|---|---|---|---|---|---|---|---|---|
| 8 | 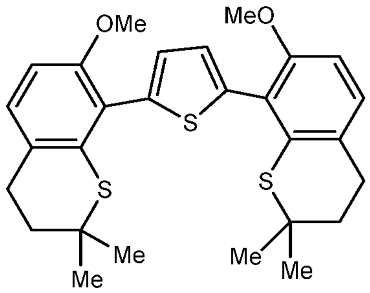 | n.a. | n.a | Green | 0.83 | 1.372 | 1.02 | 1.562 | 0.19 |
| 9 | 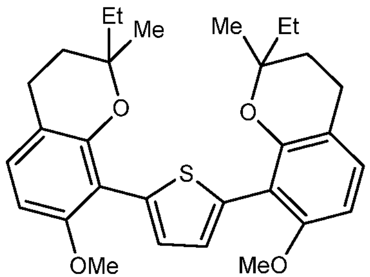 | n.a | n.a | Red | 0.57 | 1.112 | 0.89 | 1.432 | 0.32 |
| 10 | 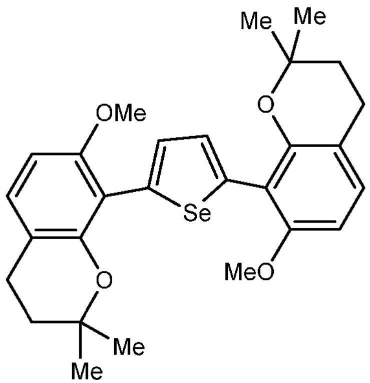 | 0.46 | 1 | Purple | 0.5 | 1.042 | 0.85 | 1.392 | 0.35 |

(continued)

| Compound | Structure | $E_1^{1/2}$ (V) | $E_1^{1/2}$ (V) vs SHE | Color | $E_1^{ox}$ peak potential (V) | E1 peak potential (V) vs SHE | $E_2^{ox}$ peak potential (V) | E2 peak potential (V) vs SHE | $\Delta E^{ox}$ |
|---|---|---|---|---|---|---|---|---|---|
| 11 | 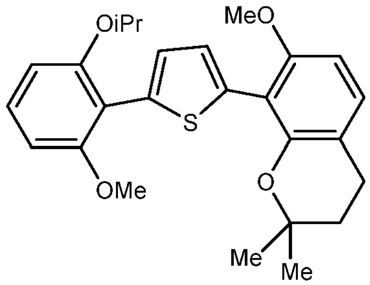 | 0.55 | 1.092 | Orange | 0.57 | 1.112 | 0.92 | 1.462 | 0.35 |
| 12 | 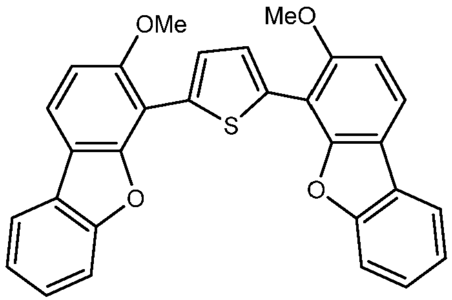 | n.a. | n.a. | Brown | 0.63 | 1.172 | | n.a. | n.a. |
| 13 | 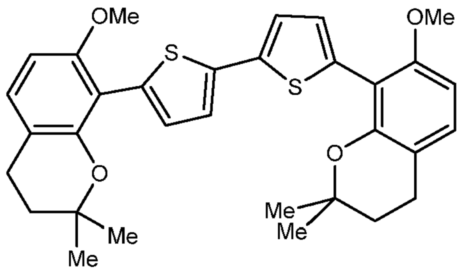 | 0.38 | 0.92 | Purple | 0.4 | 0.942 | 0.75 | 1.292 | 0.35 |

(continued)

| Compound | Structure | $E_1^{1/2}$ (V) | $E_1^{1/2}$ (V) vs SHE | Color | $E_1^{ox}$ peak potential (V) | E1 peak potential (V) vs SHE | $E_2^{ox}$ peak potential (V) | E2 peak potential (V) vs SHE | $\Delta E^{ox}$ |
|---|---|---|---|---|---|---|---|---|---|
| 14 | 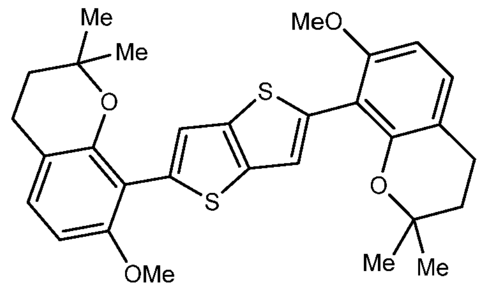 | 0.45 | 0.99 | Red | 0.48 | 1.022 | 0.76 | 1.302 | 0.28 |
| 15 | 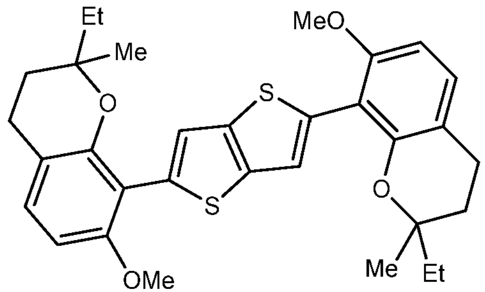 | 0.47 | 1.01 | Red | 0.5 | 1.042 | 0.78 | 1.322 | 0.28 |
| 16 | 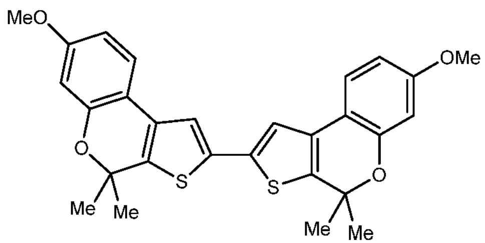 | n.a | n.a | Blue | 0.7 | 1.242 | n.a | n.a | |

(continued)

| Compound | Structure | $E_1^{1/2}$ (V) | $E_1^{1/2}$ (V) vs SHE | Color | $E_1^{ox}$ peak potential (V) | E1 peak potential (V) vs SHE | $E_2^{ox}$ peak potential (V) | E2 peak potential (V) vs SHE | $\Delta E^{ox}$ |
|---|---|---|---|---|---|---|---|---|---|
| 17 | 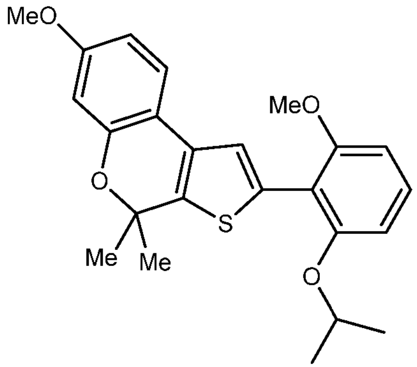 | n.a. | n.a. | Purple | 0.65 | 1.192 | 0.8 | 1.342 | 0.15 |
| COMP. 1 | 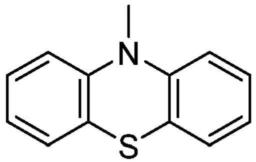 | 0.4 | 0.942 | Red | 0.45 | 0.992 | 1.01 | 1.552 | 0.56 |

**Table 2**

| Compound | Structure | εmax (L/mol/cm) | FWMH (nm) |
|---|---|---|---|
| 1 | 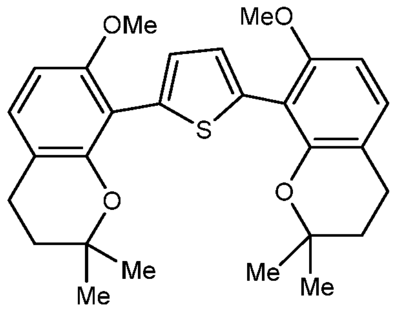 | 32000 | 20-30 |
| 4 | 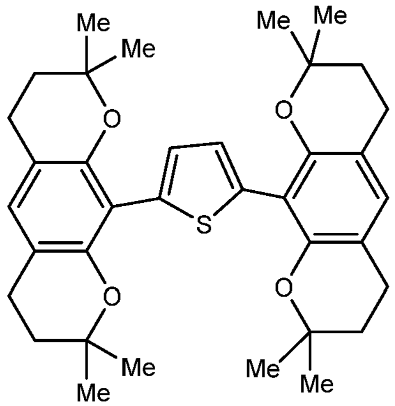 | 38000 | 20-50 |
| 5 | 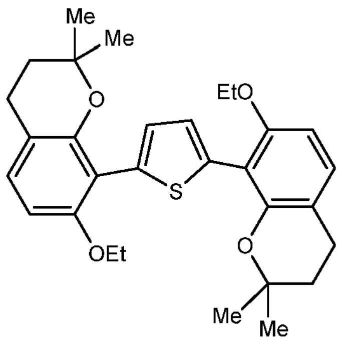 | 44000 | 30-60 |
| 11 | 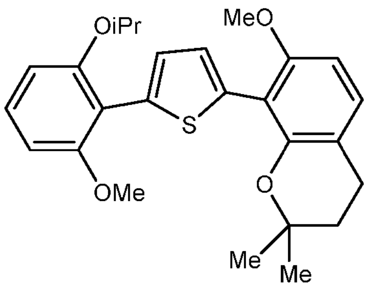 | 32500 | 20-50 |
| 14 | 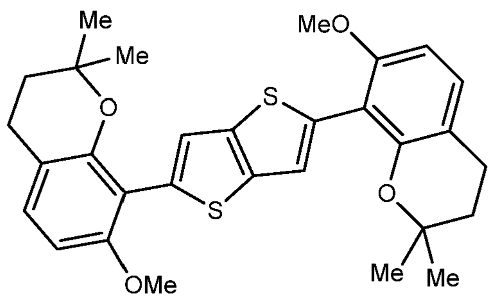 | 52000 | 20-50 |

(continued)

| Compound | Structure | εmax (L/mol/cm) | FWMH (nm) |
|---|---|---|---|
| COMP. 1 | 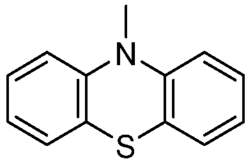 | 6130 | 80-100 |

## Claims

**1.** An electrochromic compound represented by formula (I):

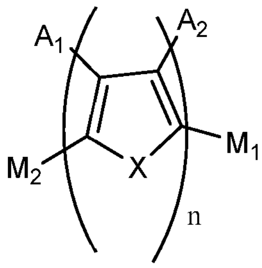

(I)

wherein

(i) $\mathbf{A_1}$ and $\mathbf{A_2}$ are both H; n is 1, 2 or 3; and $\mathbf{M_1}$ and $\mathbf{M_2}$ are each independently selected from the group consisting of H, 2,6-di-$C_{1-12}$ alkoxyphenyl, Ma, Mb and Mc:

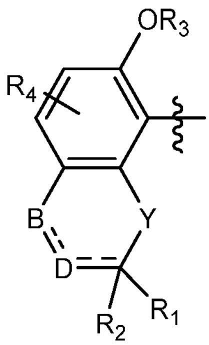

(Ma),

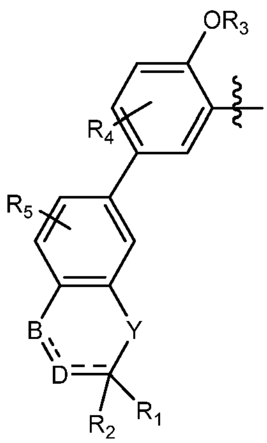

(Mb) and

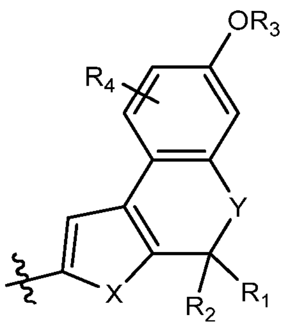

(Mc),

and wherein **at least one** of $\mathbf{M_1}$ and $\mathbf{M_2}$ is selected from the group consisting of Ma, Mb and Mc;
or
(ii) n is 1; $\mathbf{A_2}$ is H; $\mathbf{A_1}$ and $\mathbf{M_2}$ form together a heterocyclic group fused to the central 5-membered heterocyclic ring they are attached to; wherein said heterocyclic group formed is a group Ia:

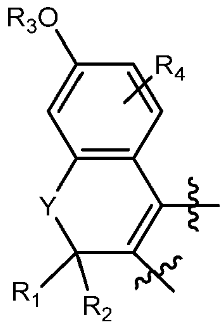
(Ia);

and $\mathbf{M_1}$ is selected from the group consisting of H, 2,6-di-$C_{1-12}$ alkoxyphenyl, Ma, Mb and Mc; or

(iii) n is 1; $\mathbf{A_1}$ is H; $\mathbf{M_2}$ is selected from the group consisting of H, 2,6-di-$C_{1-12}$ alkoxyphenyl, Ma, Mb and Mc; and $\mathbf{A_2}$ and $\mathbf{M_1}$ form together a heterocyclic group fused to the central 5-membered heteroaromatic ring they are attached to; wherein said heterocyclic group is selected from the group consisting of Ib, Ic and Id:

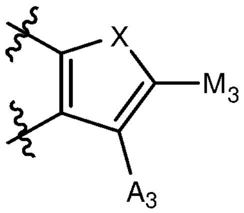
(Ib),
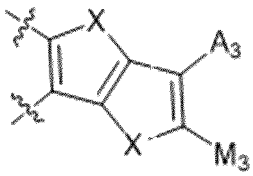
(Ic) and
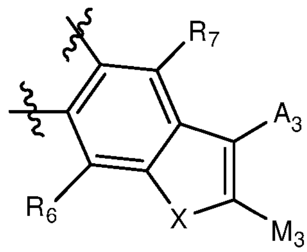
(Id);

and wherein $\mathbf{A_3}$ is H and $\mathbf{M_3}$ is selected from the group consisting of H, 2,6-di-$C_{1-12}$ alkoxyphenyl, Ma, Mb and Mc, wherein **at least one** of $\mathbf{M_2}$ and $\mathbf{M_3}$ is selected from the group consisting of Ma, Mb and Mc;

and wherein

------ is a single bond or a double bond;
each **X** is an atom independently selected from the group consisting of S, Se and O;
each **Y** is an atom independently selected from the group consisting of O, S, $-NR_8$, $N-C(O)R_8$ and $N-S(O)_2R_8$ ;
**B** is present or not, and, if present is CH or $CH_2$;
**D** is $CR_{10}$, CH or $(CH_2)_m$, wherein m is 1, 2 or 3;
$\mathbf{R_1}$ is present or not; when $\mathbf{R_1}$ is present, each $\mathbf{R_1}$ is independently selected from the group consisting of H, $C_{3-12}$ cycloalkyl, linear $C_{1-12}$ alkyl and branched $C_{3-12}$ alkyl; and each $\mathbf{R_2}$ is independently selected from the group consisting of H, $C_{3-12}$ cycloalkyl, linear $C_{1-12}$ alkyl and branched $C_{3-12}$ alkyl; or $\mathbf{R_1}$ and $\mathbf{R_2}$ form together with the carbon atom to which they are attached, a $C_{4-12}$ spiro-cycloalkyl group; and when $\mathbf{R_1}$ is not present, **D** is $CR_{10}$, wherein $\mathbf{R_2}$ and $\mathbf{R_{10}}$ form together with the carbon atom to which they are attached a $C_{6-10}$ aryl group fused to the ring to which $\mathbf{R_{10}}$ and $\mathbf{R_2}$ are attached to;
each $\mathbf{R_3}$ is independently selected from the group consisting of linear $C_{1-12}$ alkyl, branched $C_{3-12}$ alkyl, $C_{1-12}$ haloalkyl, $C_{3-12}$ cycloalkyl, $-C(O)NR_8R_9$, and a 6-membered heteroaromatic ring including at least 1 oxygen atom; and each $\mathbf{R_4}$ is independently selected from the group consisting of H, $C_{1-12}$ alkoxy, $C_{1-12}$ alkyl and $-NR_8R_9$; or when $\mathbf{R_4}$ and $\mathbf{OR_3}$ are substituents of two adjacent carbon atoms of the benzene ring they are attached to, said $\mathbf{R_4}$ and $\mathbf{OR_3}$ form together with the carbon atom to which they are attached, a 5 to 7-membered oxygen-containing ring fused to the benzene ring to which $\mathbf{R_4}$ and $\mathbf{OR_3}$ are attached; wherein said 5 to 7-membered oxygen-containing ring is optionally substituted with one or more $C_{1-6}$ alkyl groups;
each $\mathbf{R_5}$ is selected from the group consisting of H, $C_{1-12}$ alkoxy, $C_{1-12}$ alkyl and $-NR_8R_9$;
$\mathbf{R_6}$ and $\mathbf{R_7}$ are each independently selected from the group consisting of H, linear $C_{1-12}$ alkyl, branched $C_{3-12}$ alkyl, $C_{6-10}$ aryl, $C_{1-12}$ alkoxy; and
$\mathbf{R_8}$ and $\mathbf{R_9}$ are each independently selected from the group consisting of H, linear $C_{1-12}$ alkyl, branched $C_{3-12}$ alkyl and $C_{6-10}$ aryl.

2. The electrochromic compound according to claim 1, wherein $\mathbf{A_1}$ and $A_2$ are both H; n is 1, 2 or 3; and $\mathbf{M_1}$ and $\mathbf{M_2}$ are each independently selected from the group consisting of H, 2,6-di-$C_{1-12}$ alkoxyphenyl, Ma, Mb and Mc:

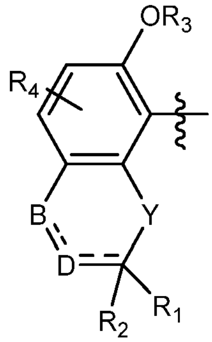 (Ma), 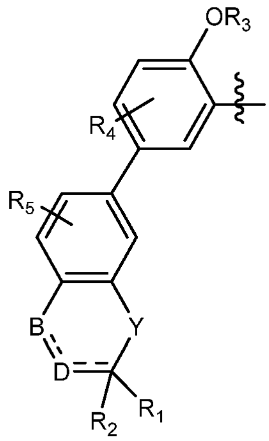 (Mb) and 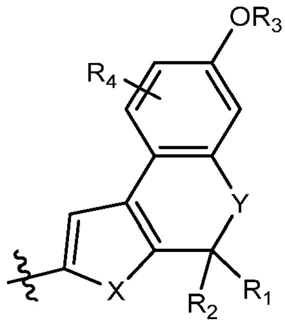 (Mc),

wherein **at least one** of $\mathbf{M_1}$ and $\mathbf{M_2}$ is selected from the group consisting of Ma, Mb and Mc; and wherein $\mathbf{R_1}$, $\mathbf{R_2}$, $\mathbf{R_3}$, $\mathbf{R_4}$, **- - - - -, Y, B** and **D** are each independently defined according to claim 1.

**3.** The electrochromic compound according to claim 2, wherein $\mathbf{A_1}$ and $\mathbf{A_2}$ are both H and n is 1.

**4.** The electrochromic compound according to any of claims 1 to 3, wherein said compound is represented by Formula (II):

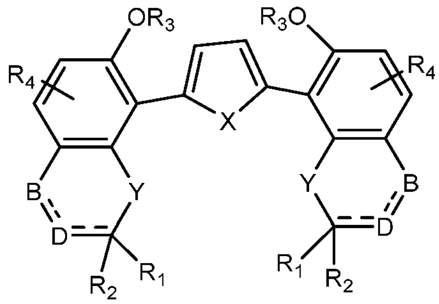 (II)

and wherein $\mathbf{R_1}$, $\mathbf{R_2}$, $\mathbf{R_3}$, $\mathbf{R_4}$, **- - - - - -, Y, B and D** are each independently defined according to claim 1.

**5.** The electrochromic compound according to claim 1, wherein one of $\mathbf{M_1}$ or $\mathbf{M_2}$ is a group 2,6-di-$C_{1-12}$ alkoxyphenyl.

**6.** The electrochromic compound according to claim 1, wherein n is 1; $\mathbf{A_2}$ is H; and $\mathbf{A_1}$ and $\mathbf{M_2}$ form together a heterocyclic group fused to the central 5-membered heterocyclic ring they are attached to; wherein said heterocyclic group formed is a group Ia:

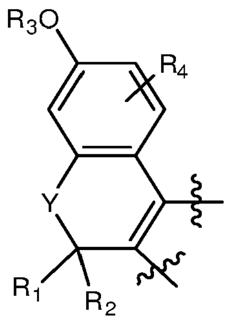 (Ia);

and $\mathbf{M_1}$ is selected from the group consisting of H, 2,6-di-$C_{1-12}$ alkoxyphenyl, Ma, Mb and Mc; and wherein $\mathbf{R_1}$, $\mathbf{R_2}$, $\mathbf{R_3}$, $\mathbf{R_4}$, **Y,** Ma, Mb and Mc are each independently defined according to claim 1.

**7.** The electrochromic compound according to claim 1, wherein n is 1; $\mathbf{A_1}$ is H; $\mathbf{M_2}$ is selected from the group consisting of H, 2,6-di-$C_{1-12}$ alkoxyphenyl, Ma, Mb and Mc; and $\mathbf{A_2}$ and $\mathbf{M_1}$ form together a heterocyclic group fused to the central 5-membered heteroaromatic ring they are attached to; wherein said heterocyclic group is selected from the group consisting of Ib, Ic and Id:

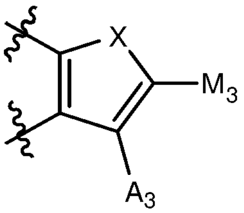 (Ib), 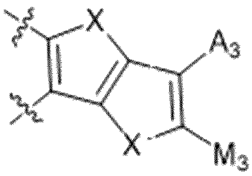 (Ic) and 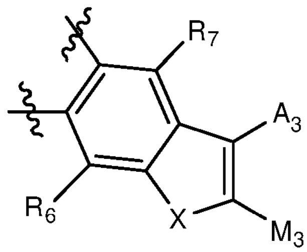 (Id);

and wherein $\mathbf{A_3}$ is H and $\mathbf{M_3}$ is selected from the group consisting of H, 2,6-di-$C_{1-12}$ alkoxyphenyl, Ma, Mb and Mc, wherein **at least one of $\mathbf{M_2}$** and $\mathbf{M_3}$ is selected from the group consisting of Ma, Mb and Mc; and wherein $\mathbf{R_6}$, $\mathbf{R_7}$, **X,** Ma, Mb and Mc are each independently defined according to claim 1.

**8.** The electrochromic compound according to any of claims 1 to 7, wherein one or more of X is S.

**9.** The electrochromic compound according to any of claims 1 to 8, wherein one or more of Y is O.

**10.** The electrochromic compound according to any of claims 1 to 9, wherein ------ is a single bond and B and D are both $CH_2$.

**11.** The electrochromic compound according to any of claims 1 to 11, wherein $\mathbf{R_1}$ and $\mathbf{R_2}$ are each independently a linear $C_{1-6}$ alkyl group or a branched $C_{3-6}$ alkyl group; $\mathbf{R_3}$ is independently a linear $C_{1-6}$ alkyl group, a branched $C_{3-6}$ alkyl group or $C_{1-6}$ (per)fluoroalkyl group; and $\mathbf{R_4}$, $\mathbf{R_5}$, $\mathbf{R_6}$ and $\mathbf{R_7}$ **are** all H.

**12.** The electrochromic compound according to claim 1, wherein said compound is selected from the group consisting of:

| | |
|---|---|
| Compound 1 | 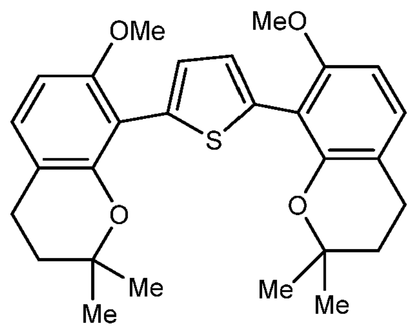 |
| Compound 2 | 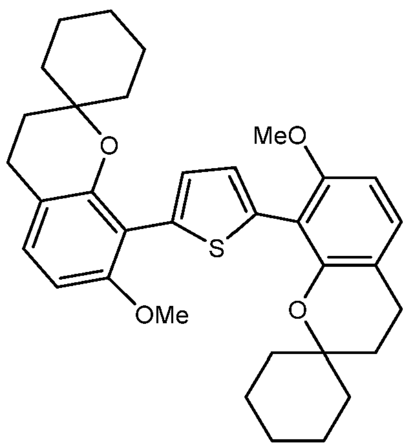 |

(continued)

| | |
|---|---|
| Compound 3 | 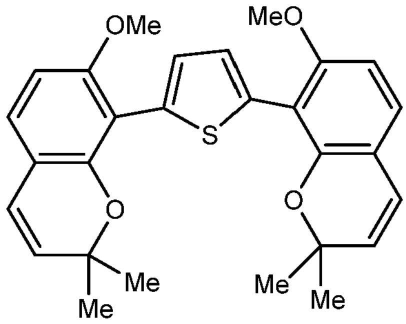 |
| Compound 4 | 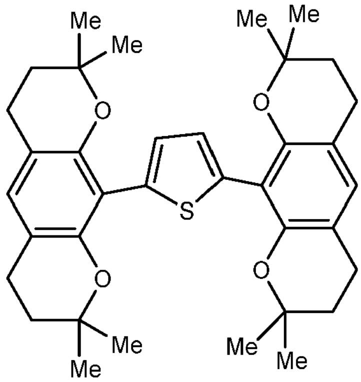 |
| Compound 5 | 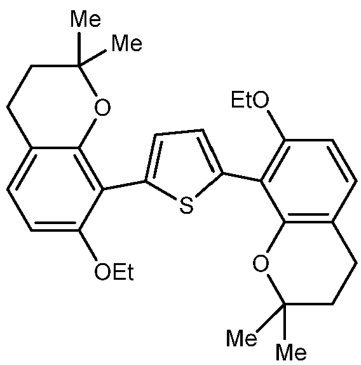 |
| Compound 6 | 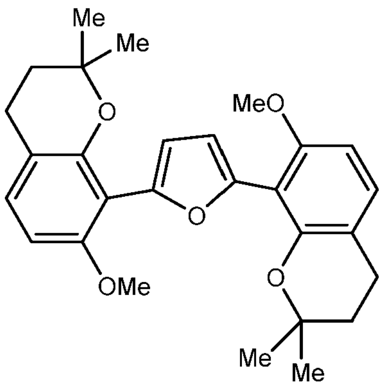 |

(continued)

| | |
|---|---|
| Compound 7 | 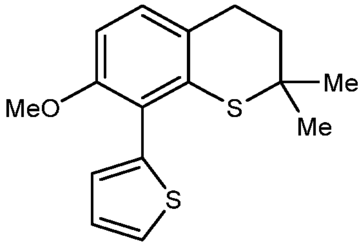 |
| Compound 8 | 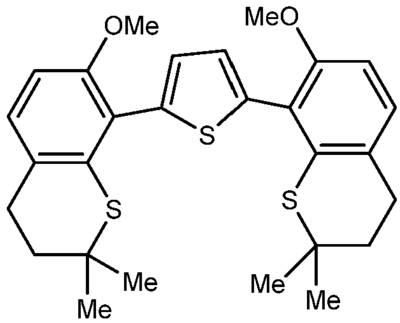 |
| Compound 9 | 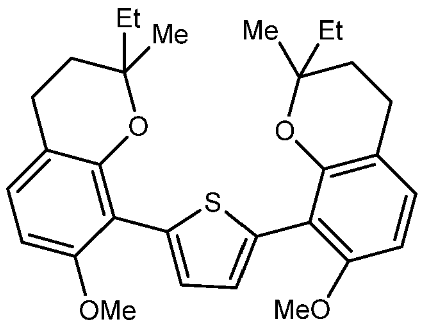 |
| Compound 10 | 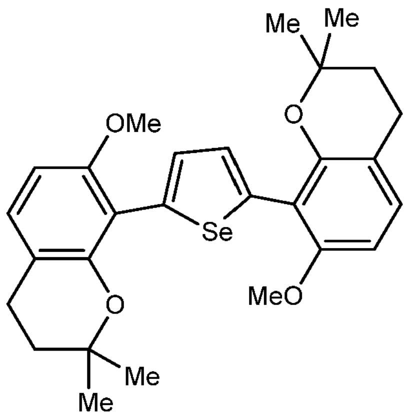 |
| Compound 11 | 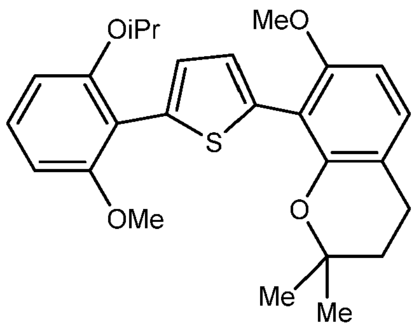 |

(continued)

| | |
|---|---|
| Compound 12 | 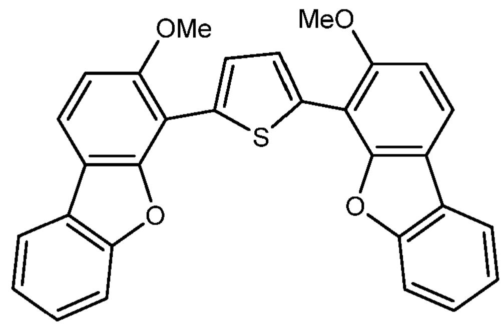 |
| Compound 13 | 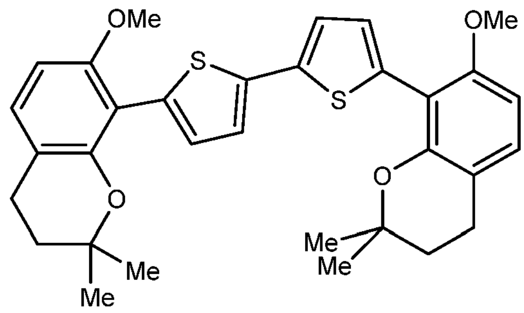 |
| Compound 14 | 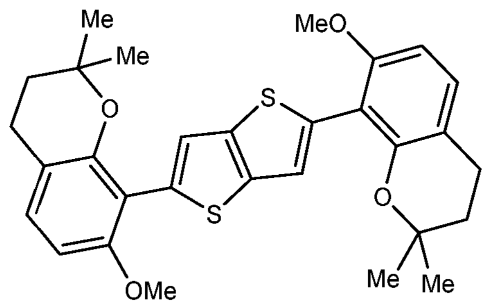 |
| Compound 15 | 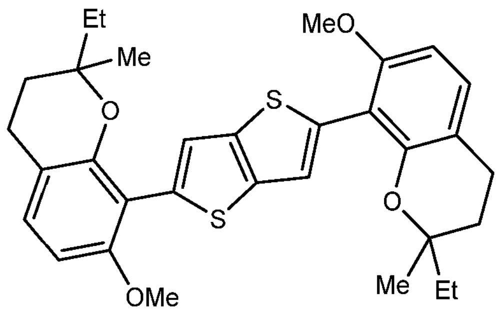 |
| Compound 16 | 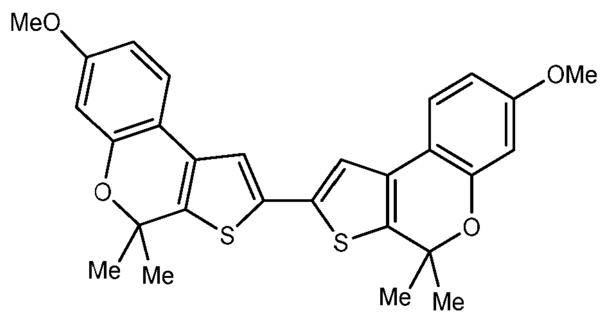 |

(continued)

| | |
|---|---|
| Compound 17 | 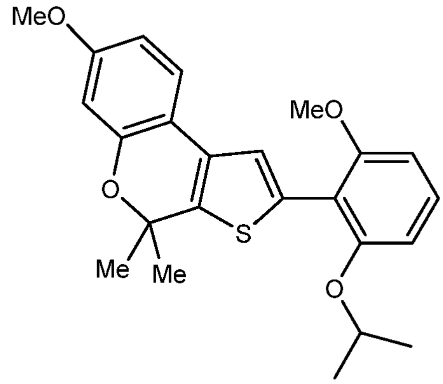 |

**13.** An electrochromic composition comprising at least one electrochromic compound as defined in any one of claims 1 to 12, and a host medium.

**14.** An electrochromic device comprising at least one electrochromic compound as defined in any of claims 1 to 12, or an electrochromic composition according to claim 13, wherein said electrochromic device is selected from an optical article, a window, a visor, a mirror, a head mounted device, a variable transmission device and a display.

**15.** The electrochromic device according to claim 14, wherein said optical article is an optical lens, preferably an ophthalmic lens, or an optical filter.

**Patentansprüche**

**1.** Elektrochrome Verbindung, die durch Formel (I) wiedergegeben wird:

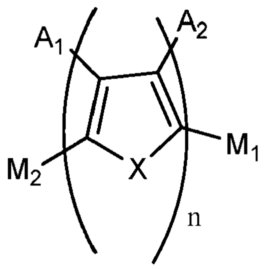

(I)

wobei

(i) $\mathbf{A_1}$ und $A_2$ beide für H stehen; n für 1, 2 oder 3 steht und $M_1$ und $M_2$ jeweils unabhängig aus der Gruppe bestehend aus H, 2,6-Di-$C_{1-12}$-alkoxyphenyl, Ma, Mb und Mc ausgewählt sind:

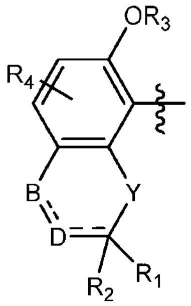 (Ma), 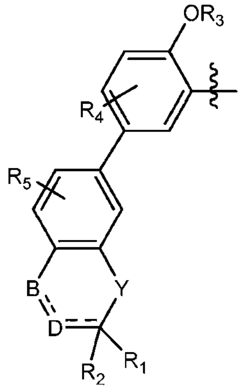 (Mb) und 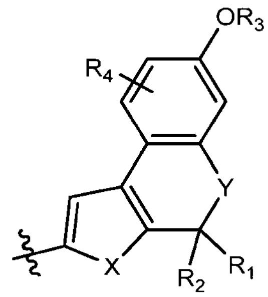 (Mc),

und wobei mindestens eines von $M_1$ und $M_2$ aus der Gruppe bestehend aus Ma, Mb und Mc ausgewählt ist; oder

(ii) n für 1 steht; $A_2$ für H steht; $A_1$ und $M_2$ zusammen eine heterocyclische Gruppe bilden, die an den zentralen 5-gliedrigen heterocyclischen Ring, an den sie gebunden sind, ankondensiert ist; wobei es sich bei der gebildeten heterocyclischen Gruppe um eine Gruppe Ia handelt:

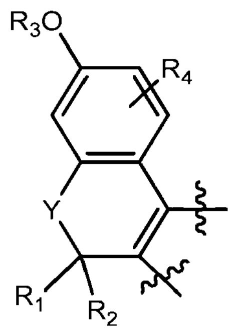 (Ia);

und $M_1$ aus der Gruppe bestehend aus H, 2,6-Di-$C_{1-12}$-alkoxyphenyl, Ma, Mb und Mc ausgewählt ist; oder

(iii) n für 1 steht; $A_1$ für H steht; $M_2$ aus der Gruppe bestehend aus H, 2,6-Di-$C_{1-12}$-alkoxyphenyl, Ma, Mb und Mc ausgewählt ist und $A_2$ und $M_1$ zusammen eine heterocyclische Gruppe bilden, die an den zentralen 5-gliedrigen heterocyclischen Ring, an den sie gebunden sind, ankondensiert ist; wobei die heterocyclische Gruppe aus der Gruppe bestehend aus Ib, Ic und Id ausgewählt ist:

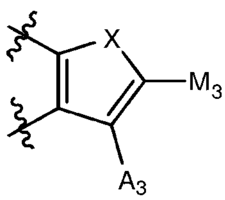 (Ib), 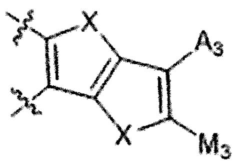 (Ic) und 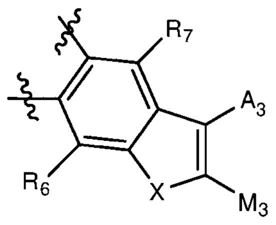 (Id);

und wobei $A_3$ für H steht und $M_3$ aus der Gruppe bestehend aus H, 2,6-Di-$C_{1-12}$-alkoxyphenyl, Ma, Mb und Mc ausgewählt ist, wobei mindestens eines von $M_2$ und $M_3$ aus der Gruppe bestehend aus Ma, Mb und Mc ausgewählt ist;

und wobei

- - - - - - für eine Einfachbindung oder eine Doppelbindung steht;
X jeweils für ein Atom steht, das unabhängig aus der Gruppe bestehend aus S, Se und O ausgewählt ist;
Y jeweils für ein Atom steht, das unabhängig aus der Gruppe bestehend aus O, S, $-NR_8$, $N-C(O)R_8$ und $N-S(O)_2R_8$ ausgewählt ist;

B vorliegt oder nicht und dann, wenn es vorliegt, für CH oder $CH_2$ steht;
D für $CR_{10}$, CH oder $(CH_2)_m$ steht, wobei m für 1, 2 oder 3 steht;
$R_1$ vorliegt oder nicht; dann, wenn $R_1$ vorliegt, $R_1$ jeweils unabhängig aus der Gruppe bestehend aus H, $C_{3-12}$-Cycloalkyl, linearem $C_{1-12}$-Alkyl und verzweigtem $C_{3-12}$-Alkyl ausgewählt ist; und $R_2$ jeweils unabhängig aus der Gruppe bestehend aus H, $C_{3-12}$-Cycloalkyl, linearem $C_{1-12}$-Alkyl und verzweigtem $C_{3-12}$-Alkyl ausgewählt ist; oder $R_1$ und $R_2$ zusammen mit dem Kohlenstoffatom, an das sie gebunden sind, eine $C_{4-12}$-Spirocycloalkylgruppe bilden; und dann, wenn $R_1$ nicht vorliegt, D für $CR_{10}$ steht, wobei $R_2$ und $R_{10}$ zusammen mit dem Kohlenstoffatom, an das sie gebunden sind, eine $C_{6-10}$-Arylgruppe bilden, die an den Ring, an den $R_{10}$ und $R_2$ gebunden sind, ankondensiert ist;
$R_3$ jeweils unabhängig aus der Gruppe bestehend aus linearem $C_{1-12}$-Alkyl, verzweigtem $C_{3-12}$-Alkyl, $C_{1-12}$-Halogenalkyl, $C_{3-12}$-Cycloalkyl, -C(O)$NR_8R_9$ und einem 6-gliedrigen heteroaromatischen Ring, der mindestens 1 Sauerstoffatom enthält, ausgewählt ist und $R_4$ jeweils unabhängig aus der Gruppe bestehend aus H, $C_{1-12}$-Alkoxy, $C_{1-12}$-Alkyl und -$NR_8R_9$ ausgewählt ist; oder dann, wenn $R_4$ und $OR_3$ Substituenten von zwei benachbarten Kohlenstoffatomen des Benzolrings, an den sie gebunden sind, sind, das $R_4$ und $OR_3$ zusammen mit dem Kohlenstoffatom, an den sie gebunden sind, einen 5- bis 7-gliedrigen sauerstoffhaltigen Ring bilden, der an den Benzolring, an den $R_4$ und $OR_3$ gebunden sind, ankondensiert ist; wobei der 5- bis 7-gliedrige sauerstoffhaltige Ring gegebenenfalls durch eine oder mehrere $C_{1-6}$-Alkylgruppen substituiert ist;
$R_5$ jeweils aus der Gruppe bestehend aus H, $C_{1-12}$-Alkoxy, $C_{1-12}$-Alkyl und -$NR_8R_9$ ausgewählt ist;
$R_6$ und $R_7$ jeweils unabhängig aus der Gruppe bestehend aus H, linearem $C_{1-12}$-Alkyl, verzweigtem $C_{3-12}$-Alkyl, $C_{6-10}$-Aryl, $C_{1-12}$-Alkoxy ausgewählt sind; und
$R_8$ und $R_9$ jeweils unabhängig aus der Gruppe bestehend aus H, linearem $C_{1-12}$-Alkyl, verzweigtem $C_{3-12}$-Alkyl und $C_{6-10}$-Aryl ausgewählt sind.

**2.** Elektrochrome Verbindung nach Anspruch 1, wobei $A_1$ und $A_2$ beide für H stehen; n für 1, 2 oder 3 steht und $M_1$ und $M_2$ jeweils unabhängig aus der Gruppe bestehend aus H, 2,6-Di-$C_{1-12}$-alkoxyphenyl, Ma, Mb und Mc ausgewählt sind:

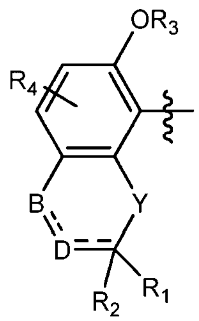 (Ma), 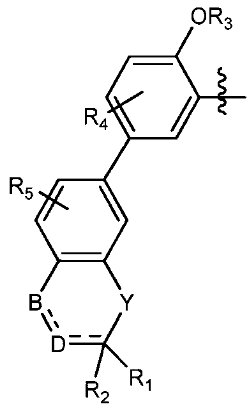 (Mb) und 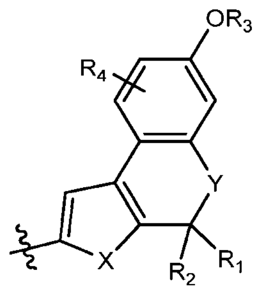 (Mc),

wobei mindestens eines von $M_1$ und $M_2$ aus der Gruppe bestehend aus Ma, Mb und Mc ausgewählt ist; wobei $R_1$, $R_2$, $R_3$, $R_4$, ------, Y, B und D jeweils unabhängig gemäß Anspruch 1 definiert sind.

**3.** Elektrochrome Verbindung nach Anspruch 2, wobei $A_1$ und $A_2$ beide für H stehen und n für 1 steht.

**4.** Elektrochrome Verbindung nach einem der Ansprüche 1 bis 3, wobei die Verbindung durch Formel (II) wiedergegeben wird:

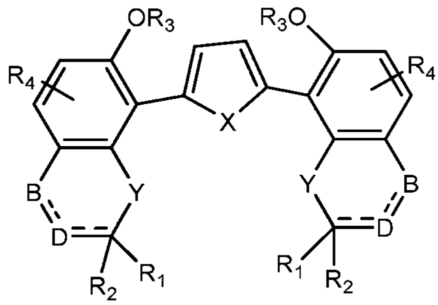

(II)

und wobei $R_1$, $R_2$, $R_3$, $R_4$, ------, Y, B und D jeweils unabhängig gemäß Anspruch 1 definiert sind.

**5.** Elektrochrome Verbindung nach Anspruch 1, wobei eines von $M_1$ oder $M_2$ für eine Gruppe 2,6-Di-$C_{1-12}$-alkoxyphenyl steht.

**6.** Elektrochrome Verbindung nach Anspruch 1, wobei n für 1 steht; $A_2$ für H steht und $A_1$ und $M_2$ zusammen eine heterocyclische Gruppe bilden, die an den zentralen 5-gliedrigen heterocyclischen Ring, an den sie gebunden sind, ankondensiert ist; wobei es sich bei der gebildeten heterocyclischen Gruppe um eine Gruppe Ia handelt:

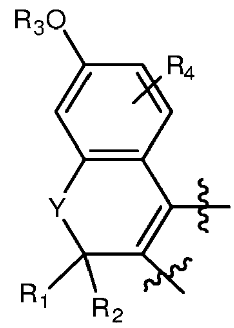

(Ia);

und $M_1$ aus der Gruppe bestehend aus H, 2, 6-Di-$C_{1-12}$-alkoxyphenyl, Ma, Mb und Mc ausgewählt ist; und wobei $R_1$, $R_2$, $R_3$, $R_4$, Y, Ma, Mb und Mc jeweils unabhängig gemäß Anspruch 1 definiert sind.

**7.** Elektrochrome Verbindung nach Anspruch 1, wobei n für 1 steht; $A_1$ für H steht; $M_2$ aus der Gruppe bestehend aus H, 2,6-Di-$C_{1-12}$-alkoxyphenyl, Ma, Mb und Mc ausgewählt ist und $A_2$ und $M_1$ zusammen eine heterocyclische Gruppe bilden, die an den zentralen 5-gliedrigen heterocyclischen Ring, an den sie gebunden sind, ankondensiert ist; wobei die heterocyclische Gruppe aus der Gruppe bestehend aus Ib, Ic und Id ausgewählt ist:

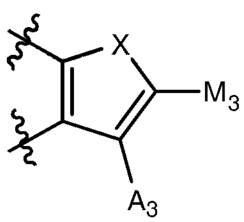

(Ib),

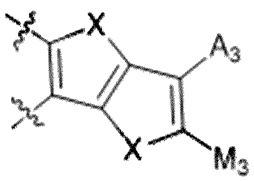

(Ic) und

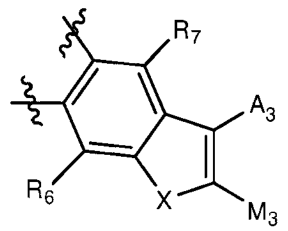

(Id);

und wobei $A_3$ für H steht und $M_3$ aus der Gruppe bestehend aus H, 2,6-Di-$C_{1-12}$-alkoxyphenyl, Ma, Mb und Mc ausgewählt ist, wobei mindestens eines von $M_2$ und $M_3$ aus der Gruppe bestehend aus Ma, Mb und Mc ausgewählt ist; und wobei $R_6$, $R_7$, X, Ma, Mb und Mc jeweils unabhängig gemäß Anspruch 1 definiert sind.

**8.** Elektrochrome Verbindung nach einem der Ansprüche 1 bis 7, wobei eines oder mehrere von X für S steht bzw. stehen.

**9.** Elektrochrome Verbindung nach einem der Ansprüche 1 bis 8, wobei eines oder mehrere von Y für O steht bzw.

stehen.

**10.** Elektrochrome Verbindung nach einem der Ansprüche 1 bis 9, wobei - - - - - für eine Einfachbindung steht und B und D beide für $CH_2$ stehen.

**11.** Elektrochrome Verbindung nach einem der Ansprüche 1 bis 11, wobei $R_1$ und $R_2$ jeweils unabhängig für eine lineare $C_{1-6}$-Alkylgruppe oder eine verzweigte $C_{3-6}$-Alkylgruppe stehen; $R_3$ unabhängig für eine lineare $C_{1-6}$-Alkylgruppe, eine verzweigte $C_{3-6}$-Alkylgruppe oder eine $C_{1-6}$- (Per) fluoralkylgruppe steht und $R_4$, $R_5$, $R_6$ und $R_7$ alle für H stehen.

**12.** Elektrochrome Verbindung nach Anspruch 1, wobei die Verbindung ausgewählt ist aus der Gruppe bestehend aus:

| | |
|---|---|
| Verbindung 1 | 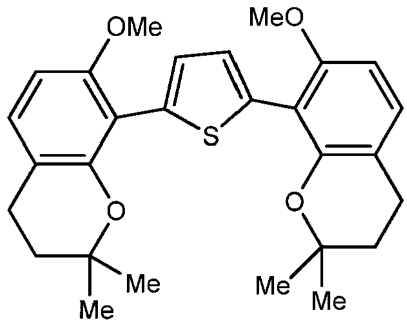 |
| Verbindung 2 | 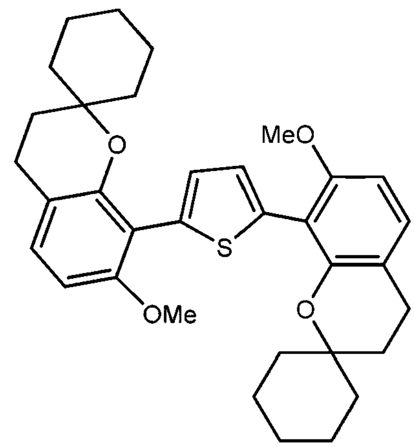 |
| Verbindung 3 | 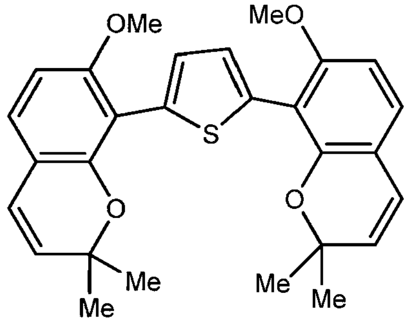 |

(continued)

| | |
|---|---|
| Verbindung 4 | 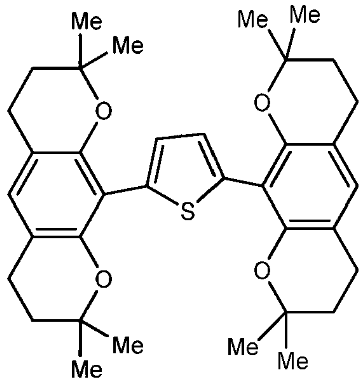 |
| Verbindung 5 | 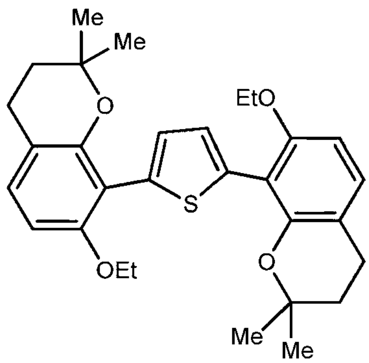 |
| Verbindung 6 | 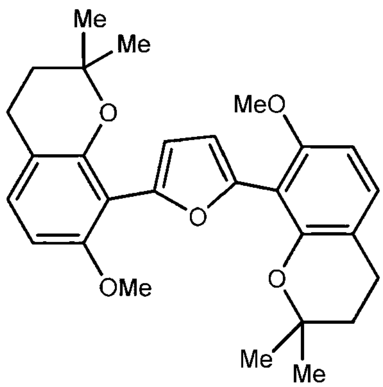 |
| Verbindung 7 | 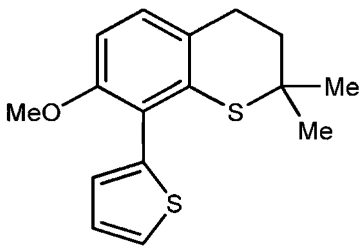 |

(continued)

| | |
|---|---|
| Verbindung 8 | 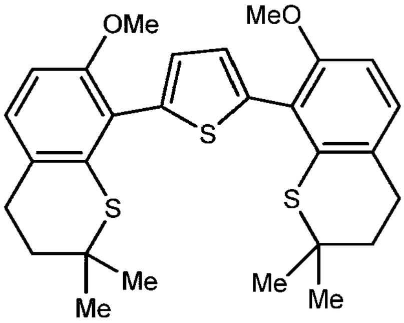 |
| Verbindung 9 | 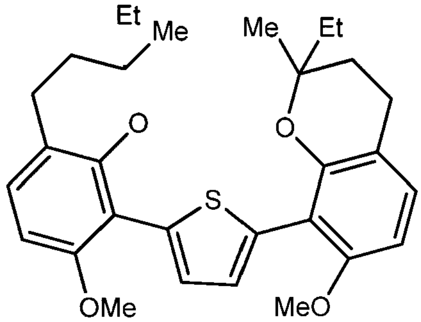 |
| Verbindung 10 | 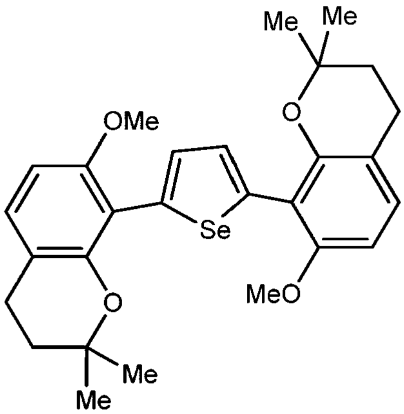 |
| Verbindung 11 | 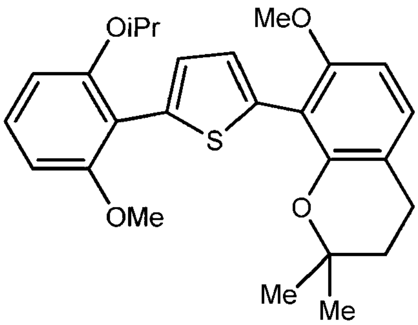 |

(continued)

| | |
|---|---|
| Verbindung 12 | 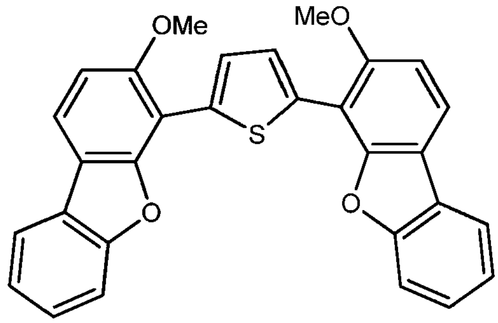 |
| Verbindung 13 | 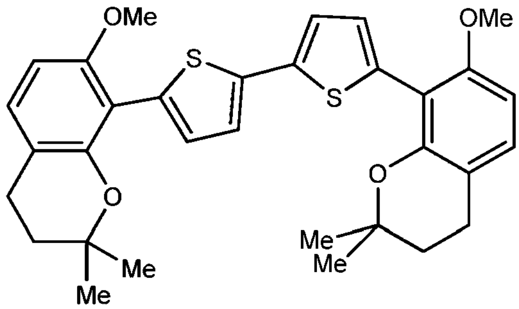 |
| Verbindung 14 | 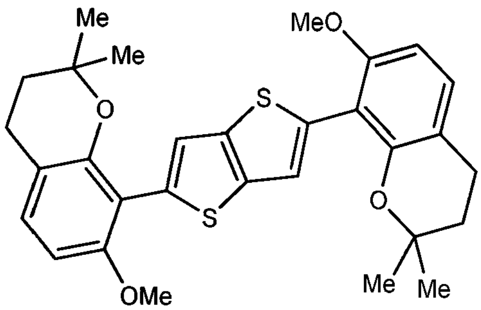 |
| Verbindung 15 | 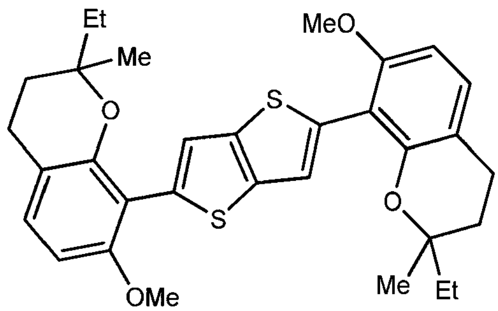 |
| Verbindung 16 | 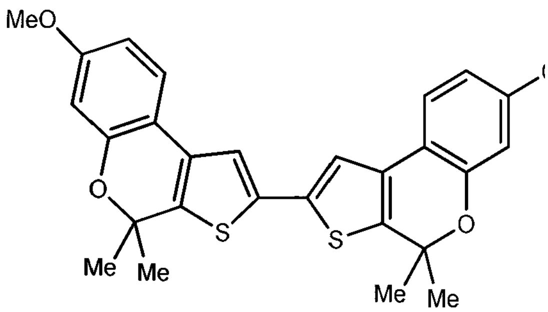 |

(continued)

| | |
|---|---|
| Verbindung 17 | 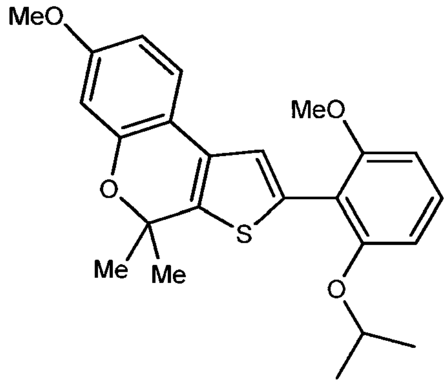 |

**13.** Elektrochrome Zusammensetzung, umfassend mindestens eine elektrochrome Verbindung gemäß einem der Ansprüche 1 bis 12 und ein Wirtsmedium.

**14.** Elektrochrome Vorrichtung, umfassend mindestens eine elektrochrome Verbindung gemäß einem der Ansprüche 1 bis 12 oder eine elektrochrome Zusammensetzung nach Anspruch 13, wobei die elektrochrome Vorrichtung aus einem optischen Artikel, einem Fenster, einem Spiegel, eine am Kopf befestigten Vorrichtung, einer Vorrichtung mit variabler Transmission und einer Anzeige ausgewählt ist.

**15.** Elektrochrome Vorrichtung nach Anspruch 14, wobei es sich bei dem optischen Artikel um eine optische Linse, vorzugsweise eine ophthalmische Linse, oder ein optisches Filter handelt.

**Revendications**

**1.** Composé électrochromique représenté par la formule (I) :

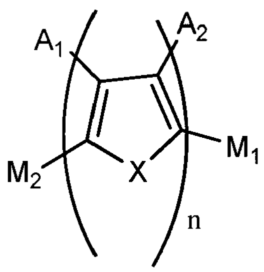

(I)

dans laquelle

(i) $\mathbf{A_1}$ et $\mathbf{A_2}$ sont tous deux H ; n est 1, 2 ou 3 ; et $\mathbf{M_1}$ et $\mathbf{M_2}$ sont chacun indépendamment choisis dans le groupe constitué par H, 2,6-di-$C_{1-12}$ alcoxyphényle, Ma, Mb et Mc :

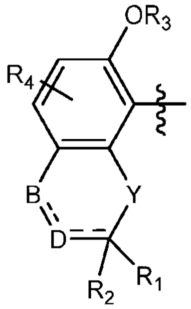 (Ma), 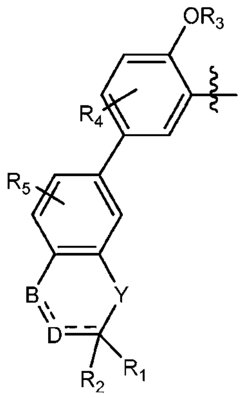 (Mb) et 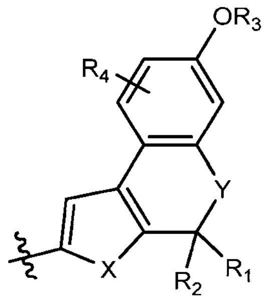 (Mc),

et dans laquelle **au moins l'un** parmi $\mathbf{M_1}$ et $\mathbf{M_2}$ est choisi dans le groupe constitué par Ma, Mb et Mc ;
ou
(ii) **n** est 1 ; $\mathbf{A_2}$ est H ; $\mathbf{A_1}$ et $\mathbf{M_2}$ forment ensemble un groupe hétérocyclique condensé au cycle hétérocyclique à 5 chaînons central auquel ils sont fixés ; dans laquelle ledit groupe hétérocyclique formé est un groupe Ia :

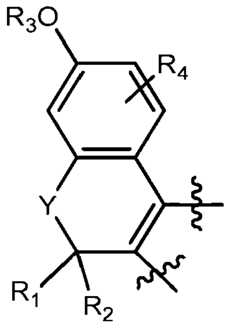 (Ia);

et $\mathbf{M_1}$ est choisi dans le groupe constitué par H, 2,6-di-$C_{1-12}$ alcoxyphényle, Ma, Mb et Mc ;
ou
(iii) **n** est 1 ; $\mathbf{A_1}$ est H ; $\mathbf{M_2}$ est choisi dans le groupe constitué par H, 2,6-di-$C_{1-12}$ alcoxyphényle, Ma, Mb et Mc ; et $\mathbf{A_2}$ et $\mathbf{M_1}$ forment ensemble un groupe hétérocyclique condensé au cycle hétéroaromatique à 5 chaînons central auquel ils sont fixés ; dans laquelle ledit groupe hétérocyclique est choisi dans le groupe constitué par Ib, Ic et Id :

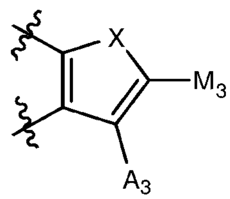 (Ib), 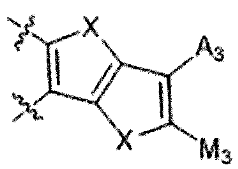 (Ic) et 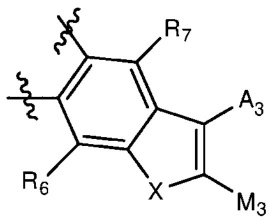 (Id);

et dans laquelle $\mathbf{A_3}$ est H et $\mathbf{M_3}$ est choisi dans le groupe constitué par H, 2,6-di-$C_{1-12}$ alcoxyphényle, Ma, Mb et Mc, dans laquelle **au moins l'un** parmi $\mathbf{M_2}$ et $\mathbf{M_3}$ est choisi dans le groupe constitué par Ma, Mb et Mc ;

et dans laquelle

- - - - - - est une simple liaison ou une double liaison ;
chaque **X** est un atome indépendamment choisi dans le groupe constitué par S, Se et O ;
chaque **Y** est un atome indépendamment choisi dans le groupe constitué par O, S, $-NR_8$, $N-C(O)R_8$ et $N-S(O)_2R_8$ ;
**B** est présent ou non, et, s'il est présent, est CH ou $CH_2$ ;
**D** est $CR_{10}$, CH ou $(CH_2)_m$, dans laquelle m est 1, 2 ou 3 ;
$\mathbf{R_1}$ est présent ou non ; lorsque $\mathbf{R_1}$ est présent, chaque $\mathbf{R_1}$ est indépendamment choisi dans le groupe constitué

par H, $C_{3-12}$ cycloalkyle, $C_{1-12}$ alkyle linéaire et $C_{3-12}$ alkyle ramifié ; et chaque $\mathbf{R_2}$ est indépendamment choisi dans le groupe constitué par H, $C_{3-12}$ cycloalkyle, $C_{1-12}$ alkyle linéaire et $C_{3-12}$ alkyle ramifié ; ou $\mathbf{R_1}$ et $\mathbf{R_2}$ forment conjointement avec l'atome de carbone auquel ils sont fixés, un groupe $C_{4-12}$ spiro-cycloalkyle ; et lorsque $\mathbf{R_1}$ n'est pas présent, **D** est $CR_{10}$, dans laquelle $\mathbf{R_2}$ et $\mathbf{R_{10}}$ forment conjointement avec l'atome de carbone auquel ils sont fixés un groupe $C_{6-10}$ aryle condensé au cycle auquel $\mathbf{R_{10}}$ et $\mathbf{R_2}$ sont fixés ;

chaque $\mathbf{R_3}$ est indépendamment choisi dans le groupe constitué par $C_{1-12}$ alkyle linéaire, $C_{3-12}$ alkyle ramifié, $C_{1-12}$ halogénoalkyle, $C_{3-12}$ cycloalkyle, $-C(O)NR_8R_9$, et un cycle hétéroaromatique à 6 chaînons comprenant au moins un 1 atome d'oxygène ; et chaque $\mathbf{R_4}$ est indépendamment choisi dans le groupe constitué par H, $C_{1-12}$ alcoxy, $C_{1-12}$ alkyle et $-NR_8R_9$ ; ou lorsque $\mathbf{R_4}$ et $\mathbf{OR_3}$ sont des substituants de deux atomes de carbone adjacents du cycle benzénique auquel ils sont fixés, lesdits $\mathbf{R_4}$ et $\mathbf{OR_3}$ forment conjointement avec l'atome de carbone auquel ils sont fixés, un cycle contenant de l'oxygène à 5 à 7 chaînons condensé au cycle benzénique auquel $R_4$ et $OR_3$ sont fixés ; dans laquelle ledit cycle contenant de l'oxygène à 5 à 7 chaînons est éventuellement substitué par un ou plusieurs groupes $C_{1-6}$ alkyle ;

chaque $\mathbf{R_5}$ est choisi dans le groupe constitué par H, $C_{1-12}$ alcoxy, $C_{1-12}$ alkyle et $-NR_8R_9$ ;

$\mathbf{R_6}$ et $\mathbf{R_7}$ sont chacun indépendamment choisis dans le groupe constitué par H, $C_{1-12}$ alkyle linéaire, $C_{3-12}$ alkyle ramifié, $C_{6-10}$ aryle, $C_{1-12}$ alcoxy ; et

$\mathbf{R_8}$ et $\mathbf{R_9}$ sont chacun indépendamment choisis dans le groupe constitué par H, $C_{1-12}$ alkyle linéaire, $C_{3-12}$ alkyle ramifié et $C_{6-10}$ aryle.

**2.** Composé électrochromique selon la revendication 1, dans lequel $\mathbf{A_1}$ et $\mathbf{A_2}$ sont tous deux H ; **n** est 1, 2 ou 3 ; et $\mathbf{M_1}$ et $\mathbf{M_2}$ sont chacun indépendamment choisis dans le groupe constitué par H, 2,6-di-$C_{1-12}$ alcoxyphényle, Ma, Mb et Mc :

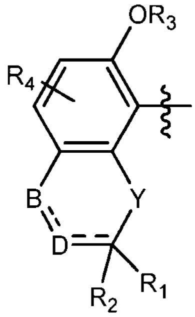
(Ma),
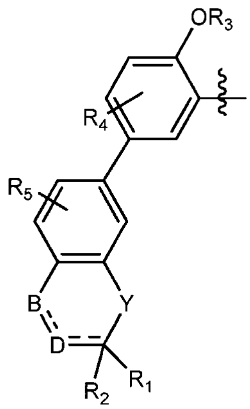
(Mb) et
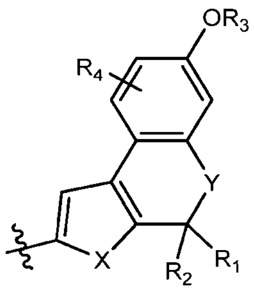
(Mc),

dans lequel **au moins l'un** parmi $\mathbf{M_1}$ et $\mathbf{M_2}$ est choisi dans le groupe constitué par Ma, Mb et Mc ; et dans lequel $\mathbf{R_1}$, $\mathbf{R_2}$, $\mathbf{R_3}$, $\mathbf{R_4}$, ------, **Y, B** et **D** sont chacun indépendamment définis selon la revendication 1.

**3.** Composé électrochromique selon la revendication 2, dans lequel $\mathbf{A_1}$ et $\mathbf{A_2}$ sont tous deux H et **n** est 1.

**4.** Composé électrochromique selon l'une quelconque des revendications 1 à 3, dans lequel ledit composé est représenté par la Formule (II) :

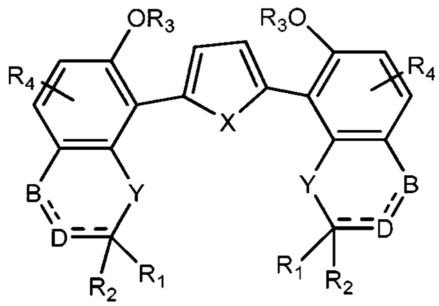
(II)

et dans laquelle $\mathbf{R_1}$, $\mathbf{R_2}$, $\mathbf{R_3}$, $\mathbf{R_4}$, ------ , **Y, B** et **D** sont chacun indépendamment définis selon la revendication 1.

**5.** Composé électrochromique selon la revendication 1, dans lequel l'un parmi $\mathbf{M_1}$ ou $\mathbf{M_2}$ est un groupe 2, 6-di-$C_{1-12}$ alcoxyphényle.

**6.** Composé électrochromique selon la revendication 1, dans lequel **n** est 1 ; $\mathbf{A_2}$ est H ; et $\mathbf{A_1}$ et $\mathbf{M_2}$ forment ensemble un groupe hétérocyclique condensé au cycle hétérocyclique à 5 chaînons central auquel ils sont fixés ; dans lequel ledit groupe hétérocyclique formé est un groupe Ia :

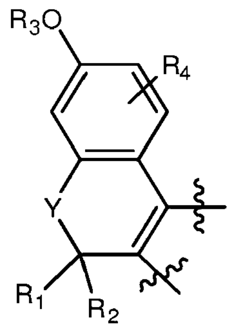

(Ia);

et $M_1$ est choisi dans le groupe constitué par H, 2, 6-di-$C_{1-12}$ alcoxyphényle, Ma, Mb et Mc ; et dans lequel $R_1$, $R_2$, $R_3$, $R_4$, Y, Ma, Mb et Mc sont chacun indépendamment définis selon la revendication 1.

**7.** Composé électrochromique selon la revendication 1, dans lequel n est 1 ; $A_1$ est H ; $M_2$ est choisi dans le groupe constitué par H, 2,6-di-$C_{1-12}$ alcoxyphényle, Ma, Mb et Mc ; et $A_2$ et $M_1$ forment ensemble un groupe hétérocyclique condensé au cycle hétéroaromatique à 5 chaînons central auquel ils sont fixés ; dans lequel ledit groupe hétérocyclique est choisi dans le groupe constitué par Ib, Ic et Id :

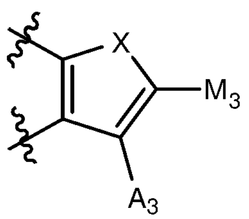

(Ib),

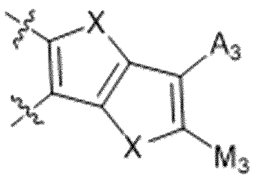

(Ic) et

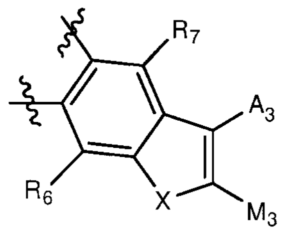

(Id);

et dans laquelle $A_3$ est H et $M_3$ est choisi dans le groupe constitué par H, 2,6-di-$C_{1-12}$ alcoxyphényle, Ma, Mb et Mc, dans laquelle au moins l'un parmi $M_2$ et $M_3$ est choisi dans le groupe constitué par Ma, Mb et Mc ; et dans laquelle $R_6$, $R_7$, X, Ma, Mb et Mc sont chacun indépendamment définis selon la revendication 1.

**8.** Composé électrochromique selon l'une quelconque des revendications 1 à 7, dans lequel un ou plusieurs parmi X est S.

**9.** Composé électrochromique selon l'une quelconque des revendications 1 à 8, dans lequel un ou plusieurs parmi Y est O.

**10.** Composé électrochromique selon l'une quelconque des revendications 1 à 9, dans lequel - - - - - - est une simple liaison et B et D sont tous deux $CH_2$.

**11.** Composé électrochromique selon l'une quelconque des revendications 1 à 11, dans lequel $\mathbf{R_1}$ et $\mathbf{R_2}$ sont chacun indépendamment un groupe $C_{1-6}$ alkyle linéaire ou un groupe $C_{3-6}$ alkyle ramifié ; $\mathbf{R_3}$ est indépendamment un groupe $C_{1-6}$ alkyle linéaire, un groupe $C_{3-6}$ alkyle ramifié ou un groupe $C_{1-6}$ (per) fluoroalkyle ; et $\mathbf{R_4}$, $\mathbf{R_5}$, $\mathbf{R_6}$ et $\mathbf{R_7}$ sont tous H.

**12.** Composé électrochromique selon la revendication 1, dans lequel ledit composé est choisi dans le groupe constitué par :

| | |
|---|---|
| Composé 1 | 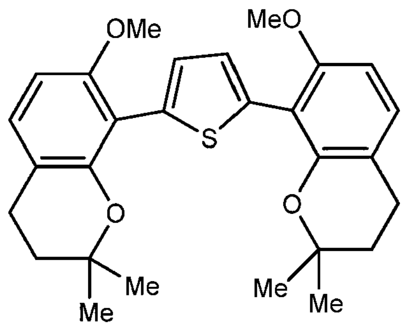 |
| Composé 2 | 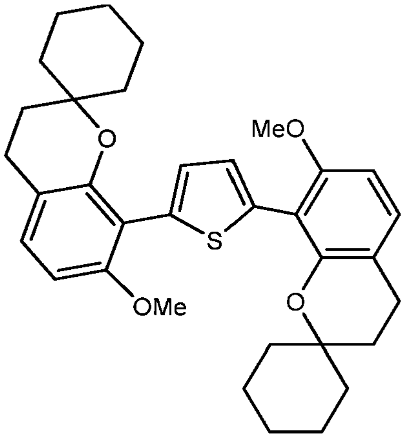 |
| Composé 3 | 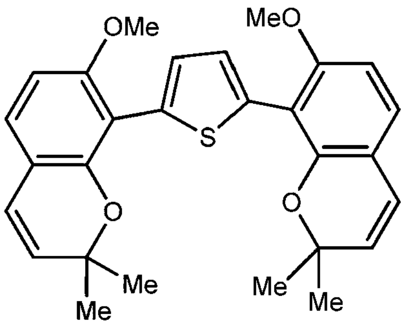 |
| Composé 4 | 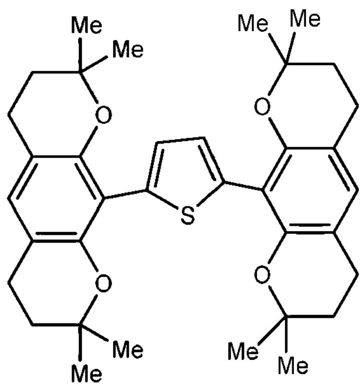 |

(continued)

| | |
|---|---|
| Composé 5 | 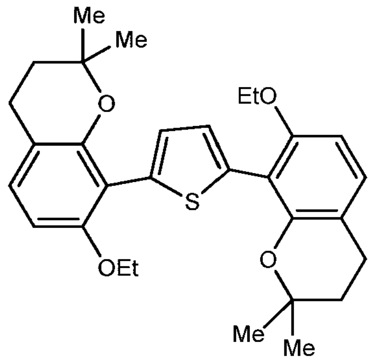 |
| Composé 6 | 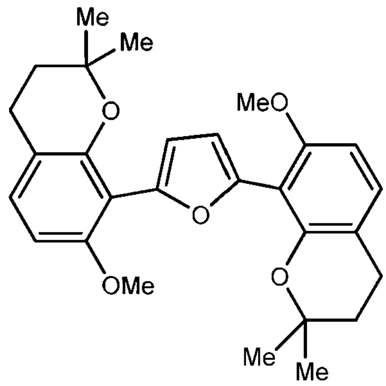 |
| Composé 7 | 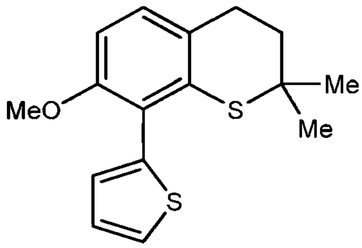 |
| Composé 8 | 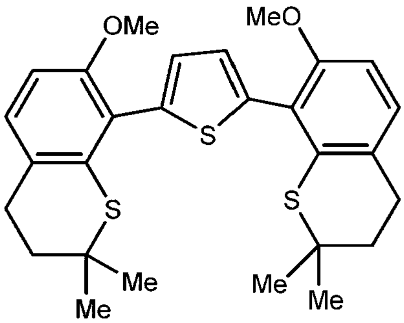 |
| Composé 9 | 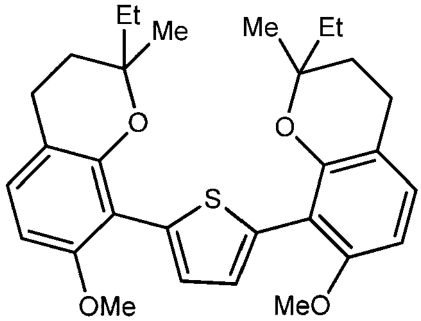 |

(continued)

| | |
|---|---|
| Composé 10 | 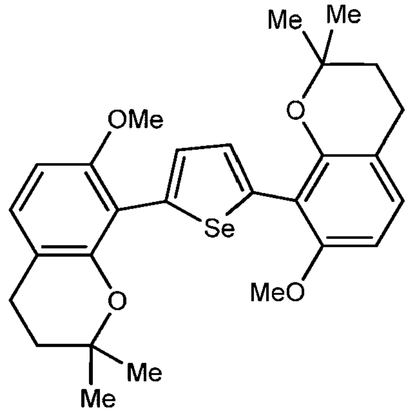 |
| Composé 11 | 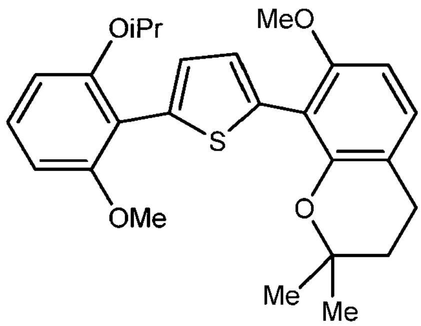 |
| Composé 12 | 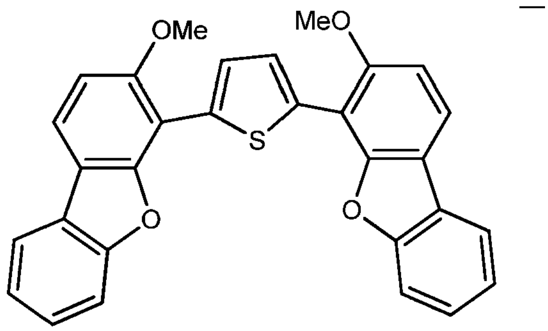 |
| Composé 13 | 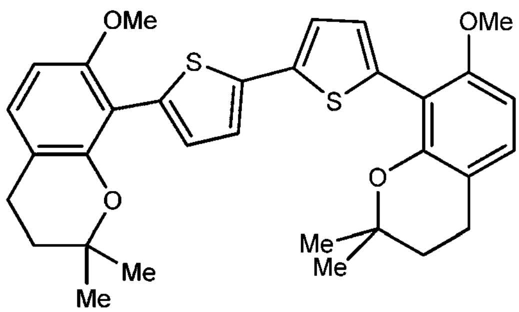 |
| Composé 14 | 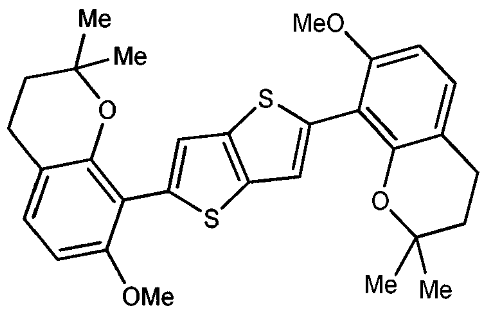 |

(continued)

| | |
|---|---|
| Composé 15 | 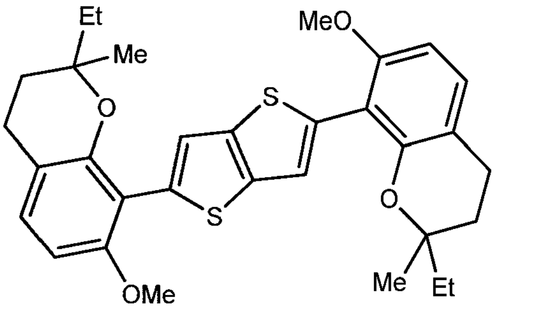 |
| Composé 16 | 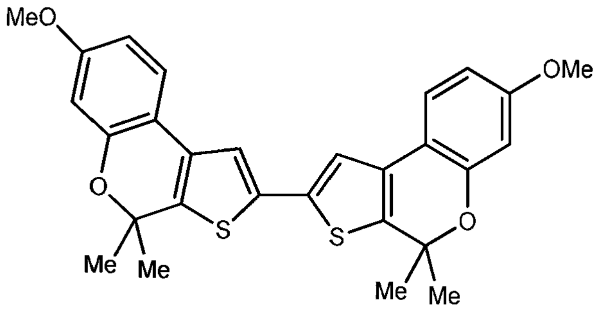 |
| Composé 17 | 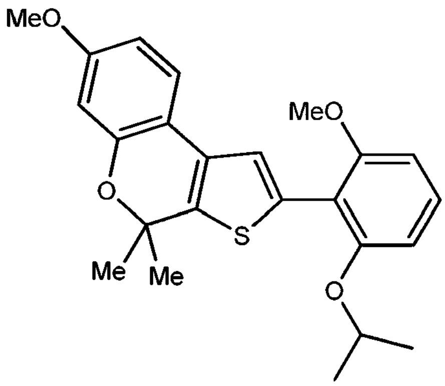 |

13. Composition électrochromique comprenant au moins un composé électrochromique tel que défini dans l'une quelconque des revendications 1 à 12 et un milieu hôte.

14. Dispositif électrochromique comprenant au moins un composé électrochromique tel que défini dans l'une quelconque des revendications 1 à 12, ou une composition électrochromique selon la revendication 13, dans lequel ledit dispositif électrochromique est choisi parmi un article optique, une fenêtre, une visière, un miroir, un dispositif monté sur la tête, un dispositif à transmission variable et un affichage.

15. Dispositif électrochromique selon la revendication 14, dans lequel ledit article optique est une lentille optique, de préférence une lentille ophtalmique, ou un filtre optique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2021284902 A
- WO 2006013250 A
- FR 2937154
- FR 2950710


### Non-patent literature cited in the description


- **H. J. KABBE**. *Synthesis*, 1978, 886-887
- **K. MERAZ et al.** *Tetrahedron Letters*, 2016, vol. 57, 5057-5061
- **R. CHATURVEDI et al.** *Indian Journal of Chemistry, Section B*, 1992, vol. 31B, 338-339
- **S. E. CLAYTON et al.** *Tetrahedron*, 1993, vol. 49
- **P. J. BROGDEN et al.** *Journal of the Chemical Society, Perkin Transactions*, 1983, vol. 1, 827-830
- **J. TERCIO et al.** *Synthesis*, 1987, vol. 2, 149-153
- **B. M. TROST et al.** *Journal of the American Chemical Society*, 1998, vol. 120, 9074-9075
- **A. BERMEJO et al.** *Bioorganic & Medicinal Chemistry*, 2019, vol. 27, 115162
- **B. ROBILLARD et al.** *Journal of Organic Chemistry*, 1986, vol. 51, 1700-1704
- **S. R. FLETCHER et al.** *Journal of Medicinal Chemistry*, 2002, vol. 45, 492-503
- **V. SNIECKUS**. *Chemical Reviews*, 1990, vol. 90, 879-933
- **V. SNIECKUS et al.** Modern Arene Chemistry. 2002, 330-367
- **V. SNIECKUS**. *Pure and Applied Chemistry*, 1990, vol. 62, 2047-2056
- **V. SNIECKUS**. *Bulletin de la Societe Chimique de France*, 1988, 67-78
- **V. SNIECKUS et al.** Handbook of C-H Transformations. 2005, vol. 1, 262-264
- **M. HALLET et al.** *Bulletin des Societes Chimiques Belges*, 1952, vol. 61, 33-43
- **L. A. PAQUETTE et al.** *Tetrahedron Letters*, 1993, vol. 34, 3235-3238
- **R. J. BETHUNE et al.** *Journal of the Chemical Society, Perkin Transactions*, 1994, vol. 1, 1925-1933
- **N. MIYAURA et al.** *Chemical Reviews*, 1995, vol. 95, 2457-2483
- **K. MERAZ et al.** *Tetrahedron Letters*, 2016, vol. 57, 5057-5061
- **M. V. B. REDDY et al.** *Bioorganic and Medicinal Chemistry*, 2008, vol. 16, 7358-7370
- **Y. JACQUOT et al.** *Medicinal Chemistry Research*, 2013, vol. 22, 681-691
- **Y WEI et al.** *Organic Letters*, 2011, vol. 13, 5504-5507
- **H. ERDTMAN et al.** *Acta Chemica Scandinavica*, 1961, vol. 15, 1761-1764
- **F. CAMPS et al.** *Journal of Heterocyclic Chemistry*, 1983, vol. 20, 1115-1117